# EUROPEAN PATENT APPLICATION

(11) **EP 4 643 776 A1**
(43) Date of publication of application: **05.11.2025**
(21) Application number: 24749595.5
(22) Date of filing: 25.01.2024
(51) Int. Cl.: A61B 5/145

(54) **PHYSIOLOGICAL SIGN DETECTION APPARATUS**

(30) Priority: 31.01.2023 CN 202310113276
(71) Applicant: Huawei Technologies Co., Ltd., Shenzhen, Guangdong 518129 (CN)
(72) Inventor: DONG, Wenxiao, Shenzhen, Guangdong 518129 (CN); SUN, Yuning, Shenzhen, Guangdong 518129 (CN); ZOU, Lin, Shenzhen, Guangdong 518129 (CN); LIU, Xiangyu, Shenzhen, Guangdong 518129 (CN); XIE, Songlin, Shenzhen, Guangdong 518129 (CN); HU, Yifan, Shenzhen, Guangdong 518129 (CN)
(74) Representative: Epping - Hermann - Fischer
(86) International application number: PCT/CN2024/073978
(87) International publication number: WO 2024/160114

(57) **Abstract**

A physiological sign detection apparatus (100) is provided, including a housing (1), a base (3), an implantation base (5), a first elastic member (2), a sensor assembly (20), and a housing cap (8). The housing (1) includes a top wall (12) and a side wall (11), and the side wall (11) of the housing (1) is annularly connected to a circumferential edge of the top wall (12) of the housing (1). The base (3) is mounted on an inner side of the housing (1) and slidably connected to the side wall (11) of the housing (1). The implantation base (5) is mounted on an inner side of the base (3) and slidably connected to the base (3). The first elastic member (2) is connected between the top wall (12) of the housing (1) and the implantation base (5). The sensor assembly (20) is fastened to the implantation base (5), and the sensor assembly (20) includes a sensor (244). The housing cap (8) is detachably fastened to an end that is of the side wall (11) of the housing (1) and that faces away from the top wall (12) of the housing (1). When the housing cap (8) is detached from the housing (1), an abutting end (301) of the base (3) abuts against an organism (30), and the housing (1) moves in a direction close to the organism (30) relative to the base (3), the first elastic member (2) pushes the implantation base (5) toward the organism (30), the sensor assembly (20) abuts against the organism (30), and the sensor (244) is punctured into the organism (30). The physiological sign detection apparatus (100) has an anti-accidental touch function, and user experience is better.

## Description

This application claims priority to Chinese Patent Application No. 202310113276.X, filed with the China National Intellectual Property Administration on January 31, 2023 and entitled "PHYSIOLOGICAL SIGN DETECTION APPARATUS", which is incorporated herein by reference in its entirety.

### TECHNICAL FIELD

This application relates to the field of medical device technologies, and in particular, to a physiological sign detection apparatus.

### BACKGROUND

Diabetes is a chronic disease that may cause many complications. A conventional diabetes monitoring manner is to use a blood glucose meter to prick a finger to take blood and perform single-point measurement on a glucose concentration in venous blood. Some current minimally invasive blood glucose detection products may implement continuous monitoring for more than 7 days by implanting a biosensor under skin to be in contact with tissue fluids. The minimally invasive blood glucose detection product applies a small sensor apparatus (usually referred to as a patch) to human skin by using an implantation apparatus. A sensor of the patch is guided by a puncture needle to subcutaneous tissue, to be in contact with body fluid. The patch transmits analytic data obtained through the sensor to a terminal such as a mobile phone or a display instrument. Most existing physiological sign detection apparatuses are of a button type, and a patch is ejected by pressing a button of the physiological sign detection apparatus. However, a button-type physiological sign detection apparatus is prone to an accidental touch. When the physiological sign detection apparatus is not aligned with skin, a patch is ejected when a button is accidentally touched. Consequently, the physiological sign detection apparatus becomes invalid, and a sensor cannot be implanted into subcutaneous tissue. Consequently, blood glucose detection cannot be implemented, and use experience of a user is poor.

### SUMMARY

This application provides a physiological sign detection apparatus. The physiological sign detection apparatus includes the physiological sign detection apparatus and a patch. The patch is built in the physiological sign detection apparatus. The physiological sign detection apparatus has an anti-accidental touch function. The physiological sign detection apparatus can effectively implant a sensor of the patch into subcutaneous tissue of a user, to implement physiological sign detection, so that user experience is better.

According to a first aspect, this application provides a physiological sign detection apparatus. The physiological sign detection apparatus includes a housing, a base, an implantation base, a first elastic member, a sensor assembly, and a housing cap. The housing includes a top wall and a side wall, the side wall of the housing is annularly connected to a circumferential edge of the top wall of the housing, and an opening is formed on a side that is of the side wall of the housing and that is away from the top wall of the housing. The base is mounted on an inner side of the housing and slidably connected to the side wall of the housing, the base includes an abutting end, and the abutting end protrudes from the housing through the opening. The implantation base is mounted on an inner side of the base and slidably connected to the base. The first elastic member is connected between the top wall of the housing and the implantation base. The sensor assembly is fastened to the implantation base and located on a side that is of the implantation base and that faces away from the top wall of the housing, and the sensor assembly includes a sensor and a puncture needle. The housing cap is detachably fastened to an end that is of the side wall of the housing and that faces away from the top wall of the housing, and the housing cap wraps the abutting end and closes the opening. When the housing cap is detached from the housing, the abutting end abuts against an organism, and the housing moves in a direction close to the organism relative to the base, the first elastic member pushes the implantation base toward the organism, the sensor assembly abuts against the organism, and the sensor is punctured into the organism under guidance of the puncture needle.

In this application, the housing is provided with no button. After the housing and the housing cap (as shown in the figure) are assembled, the housing and the housing cap form a sealed cavity, to form good isolation and protection for an implantation component and the sensor assembly on an inner side. When the housing cap is not detached from the housing, an implantation apparatus does not trigger an implantation action, and the sensor assembly is not ejected. In addition, the sensor assembly is ejected only when a user holds the base against the organism and pushes the housing toward the organism. Therefore, a risk of false triggering of the physiological sign detection apparatus is low, and reliability is higher. In addition, in an implantation process, a movement direction of the housing is consistent with a movement direction of the puncture needle, an implantation direction of the sensor assembly is easy to control, an implantation force of the sensor assembly is controlled by the first elastic member, and a magnitude of the implantation force is stable and is not affected by another external force. The physiological sign detection apparatus has a moderate implantation force and a fast implantation speed, to help reduce a pain feeling of the user.

In some possible implementations, the base further includes a second elastic arm, the second elastic arm is located on a side that is of the base and that faces away from the abutting end, a tail end of the second elastic arm is provided with a snap-fitting hook, the implantation base is provided with snap-fitting space, and at least a part of the snap-fitting hook of the second elastic arm is located in the snap-fitting space, so that the second elastic arm is snap-fitted into the implantation base. The housing further includes a toggle, and the toggle is fastened to a side that is of the top wall of the housing and that faces the opening. When the housing moves in the direction close to the organism relative to the base, the toggle pushes the second elastic arm, and the snap-fitting hook of the second elastic arm is detached from the snap-fitting space.

In this application, a limit of the base on the implantation base is removed through cooperation between the toggle and the second elastic arm, so that the implantation base is detached from the base under an elastic force of the first elastic member. When the housing moves in the implantation direction, the toggle gradually pushes a second cantilever to move toward an outer side of a side plate of the implantation base, and compresses the first elastic member. When the snap-fitting hook of the second elastic arm is detached from the snap-fitting space, the implantation base moves in the implantation direction under the elastic force of the first elastic member. A limiting mechanism and a limit release mechanism of the implantation base of the physiological sign detection apparatus are simple and are arranged compactly, and a transmission speed between the housing and the base is fast.

In some possible implementations, the second elastic arm is provided with a guiding groove, at least a part of the toggle is located in the guiding groove of the second elastic arm, and the toggle moves along the guiding groove in a process in which the toggle pushes the second elastic arm. In this application, the second elastic arm is provided with the guiding groove, and the toggle can move along the guiding groove, so that relative movement between the toggle and the second elastic arm is stable, to prevent the toggle from being detached from the second elastic arm, thereby helping improve reliability of the physiological sign detection apparatus.

In some possible implementations, the implantation base has a snap-fitting hole and a notch, and the notch is located on a side that is of the snap-fitting hole and that is close to the top wall of the housing, and communicates with the snap-fitting hole. The housing further includes a second cantilever, the second cantilever is fastened to a side that is of the top wall of the housing and that faces the opening, a tail end of the second cantilever is provided with a snap-fitting hook, and at least a part of the snap-fitting hook of the second cantilever is located in the snap-fitting hole, so that the second cantilever is snap-fitted into the implantation base. The base further includes a push block, the push block is convexly disposed on an inner side surface of the base, and at least a part of the push block is snap-fitted into the snap-fitting hole. When the housing moves in the direction close to the organism relative to the base, the second cantilever is squeezed by the push block, the snap-fitting hook of the second cantilever is detached from the snap-fitting hole, the implantation base moves in the direction close to the organism relative to the base, and the push block is detached from the snap-fitting hole through the notch.

In this application, a limit of the base on the implantation base is removed through cooperation between the push block and the second cantilever, so that the implantation base is detached from the base under an elastic force of the first elastic member. When the housing moves in the implantation direction, the push block gradually pushes the second cantilever to move toward an inner side of a side plate of the implantation base, and compresses the first elastic member. When the snap-fitting hook of the second cantilever is detached from the snap-fitting hole, the implantation base immediately moves in the implantation direction under the elastic force of the first elastic member. A limiting mechanism and a limit release mechanism of the implantation base of the physiological sign detection apparatus are simple and are arranged compactly, and a transmission speed between the housing and the base is fast.

In some possible implementations, the base further includes a first elastic arm, an end that is of the first elastic arm and that faces away from the abutting end is provided with a hook, the hook of the first elastic arm protrudes from an outer side surface of the base, and the hook of the first elastic arm is snap-fitted into the side wall of the housing, to limit movement of the housing in a direction away from the abutting end relative to the base. In this application, the hook of the first elastic arm limits a first sliding block in an axial direction, to prevent the housing from moving in the direction away from the abutting end relative to the base, and prevent the housing from being detached from the base.

In some possible implementations, the housing further includes a first guiding structure, the first guiding structure is fastened to an inner side surface of the side wall of the housing, the base further includes a second guiding structure, the second guiding structure is fastened to the outer side surface of the base, and the first guiding structure is slidably connected to the second guiding structure.

In this application, the first guiding structure is slidably connected to the second guiding structure for mutual cooperation, to guide the housing and the base to slide relative to each other in an axial direction of the housing, so that the housing and the base limit each other in a circumferential direction of the housing, and the base is prevented from rotating relative to the housing in the circumferential direction. When there are a plurality of first guiding structures and a plurality of second guiding structures, this helps balance a force between the housing and the base, to prevent the base from being skewed relative to the housing. In addition, the first sliding block can slide between a step surface and the hook of the first elastic arm, so that the housing can move in the axial direction relative to the base, and movement of the housing in the axial direction relative to the base is limited, to limit a distance at which the housing can move in the implantation direction relative to the base.

In some possible implementations, the base further includes a third guiding structure, the third guiding structure is fastened to the inner side surface of the base, the implantation base further includes a fourth guiding structure, the fourth guiding structure is fastened to an outer side surface of the implantation base, and the third guiding structure is slidably connected to the fourth guiding structure.

In this application, the third guiding structure of the base is slidably connected to the fourth guiding structure of the implantation base for mutual cooperation, so that the base and the implantation base limit each other in the circumferential direction, and the implantation base is prevented from rotating relative to the base in the circumferential direction. A third sliding block can slide in a sliding groove of a third elastic arm and a third guiding arm, so that the implantation base can move in the axial direction relative to the base, and the third elastic arm may further limit movement of the implantation base in the axial direction.

In some possible implementations, the sensor assembly further includes a needle base, the puncture needle is fastened to the needle base, a puncture end of the puncture needle protrudes from the needle base, the sensor includes an implantation portion, the puncture needle wraps the implantation portion of the sensor, the physiological sign detection apparatus further includes a needle extraction base, the needle extraction base is snap-fitted into the implantation base, and the needle extraction base is snap-fitted into the needle base. When the implantation base moves in the direction close to the organism, the implantation base pushes the needle base toward the organism through the needle extraction base, and the puncture end of the puncture needle and the implantation portion of the sensor are punctured into the organism.

In this application, the needle extraction base is snap-fitted into the needle base, so that the needle base can move synchronously with the needle extraction base. When the implantation base moves in the implantation direction, the implantation base pushes the sensor assembly to also move in the implantation direction, so that a bonding member of the sensor assembly can be stuck on the organism. In addition, the implantation base pushes the needle base to the organism through the needle extraction base, so that the puncture needle can be punctured into the organism. When the needle extraction base moves in a reverse direction of the implantation direction, the needle extraction base drives, through the needle base, the puncture needle to move in the reverse direction of the implantation direction, so that the puncture needle is detached from the organism. In some possible implementations, the physiological sign detection apparatus further includes a second elastic member, and the second elastic member is located between the implantation base and the needle extraction base. When the sensor is punctured into the organism, the second elastic member pushes the needle extraction base to a side away from the organism, the needle extraction base drives the needle base and the puncture needle to move to the side away from the organism, and the puncture needle is detached from the organism.

In some possible implementations, the implantation base includes a side plate, a bottom plate, and a fourth elastic arm, a circumferential edge of the side plate of the implantation base is fastened to the bottom plate of the implantation base, the fourth elastic arm is spaced apart from an upper side plate of the implantation base in a circumferential direction, an end of the fourth elastic arm is fastened to the bottom plate or the side plate of the implantation base, the other end of the fourth elastic arm is provided with a snap-fitting hook, the needle extraction base is mounted between the bottom plate of the implantation base and the snap-fitting hook of the fourth elastic arm, and the base has avoidance space. When the sensor is punctured into the organism, the needle extraction base pushes away the fourth elastic arm, the snap-fitting hook of the fourth elastic arm retreats to the avoidance space, and the needle extraction base is detached from the snap-fitting hook of the fourth elastic arm.

In this application, when the sensor is implanted into the organism, the avoidance space of the base provides retraction space for the snap-fitting hook of the fourth elastic arm. In addition, an elastic force of the second elastic member enables the needle extraction base to push away and be detached from the fourth elastic arm, so that the needle extraction base moves toward the reverse direction of the implantation direction, and drives the puncture needle to be detached from the organism. A needle extraction force of the needle extraction base is provided only by the second elastic member, so that the physiological sign detection apparatus can automatically and quickly retract a needle, to reduce user operations, and help improve use experience of the user.

In some possible implementations, the physiological sign detection apparatus further includes a stopper member, the stopper member is fastened to an end that is of the implantation base and that faces away from the needle extraction base, and the first elastic member is mounted between the stopper member and the top wall of the housing.

In some possible implementations, the sensor assembly further includes a sensor base and a sealing bottle, the sensor is fastened to the sensor base, the sensor base is located between the needle base and the sealing bottle, the needle base is located between the needle base and the sealing bottle, an end of the needle base passes through the sensor base, the sealing bottle is detachably fastened to the needle base and is hermetically connected to the sensor base, the needle base is hermetically connected to the sensor base, the sealing bottle has a hollow cavity, and the implantation portion of the sensor and the puncture end of the puncture needle are located in the hollow cavity of the sealing bottle.

In this application, the sensor base is located between the needle base and the sealing bottle, an end of the needle base passes through the sensor base, the sealing bottle is detachably fastened to the needle base, the implantation portion of the sensor is located in the hollow cavity of the sealing bottle, and the sealing bottle may isolate and protect the implantation portion of the sensor, to avoid damage to a sensing layer of the implantation portion in a subsequent assembly process of the sensor and another component, thereby helping improve reliability and detection accuracy of the physiological sign detection apparatus, and ensuring batch consistency of the sensor in a production process.

In some possible implementations, the sensor assembly further includes an upper cover and a sealing bottle, the sensor is snap-fitted into the upper cover, the upper cover is located between the needle base and the sealing bottle, an end of the needle base passes through the upper cover, the sealing bottle is detachably fastened to the needle base and is hermetically connected to the upper cover, the needle base is hermetically connected to the upper cover, the sealing bottle has a hollow cavity, and the implantation portion of the sensor and the puncture end of the puncture needle are located in the hollow cavity of the sealing bottle.

In this application, a sensor sealing assembly includes the upper cover, and the upper cover only needs to be provided with a second through hole that allows a guiding portion of the needle base to pass through. A hole diameter of the second through hole is smaller. In addition, the needle base is fastened to the upper cover by being securely connected to the sealing bottle. Assembly between the upper cover and the needle base is simpler, and a lower cover does not need to be provided with a structure for being snap-fitted into the sensor sealing assembly, to help simplify a structure of the lower cover.

In some possible implementations, the sealing bottle is threadedly connected to the needle base, the sealing bottle includes a ratchet, the ratchet is convexly disposed on an outer side surface of the sealing bottle, the housing cap is threadedly connected to the housing, the housing cap includes a pawl, and the pawl and the ratchet are disposed in cooperation. In a process in which the housing cap is screwed on the housing, the pawl is slidably connected to the ratchet, and in a process in which the housing cap is detached from the housing, the pawl is snap-fitted into the ratchet and pushes the sealing bottle to be detached from the needle base.

In this application, the housing cap is threadedly connected to the housing, the sealing bottle is threadedly connected to the needle base, and the pawl of the housing cap cooperates with the ratchet of the sealing bottle. When the housing cap is detached from the housing, the sealing bottle may be pushed to be detached from the needle base, so that the puncture needle and the sensor are exposed, to help simplify operation steps of the physiological sign detection apparatus, and improve use experience of the user.

In some possible implementations, the sealing bottle is snap-fitted into or interference-connected to the needle base, the sealing bottle includes a boss, the boss is located at an end that is of the sealing bottle and that faces away from the needle base, the housing cap is snap-fitted into or interference-connected to the housing, the housing cap includes a snap-fitting jaw, and the snap-fitting jaw is snap-fitted with the boss. In a process in which the housing cap is detached from the housing, the housing cap drives the sealing bottle to be detached from the needle base. In this application, the sealing bottle is snap-fitted into or interference-connected to the needle base, to securely connect the needle base, a first sealing member, the sensor base, a second sealing member, and the sealing bottle, so that the first sealing member is hermetically connected to the needle base and the sensor base, the second sealing member is hermetically connected to the sensor base and the sealing bottle, and the implantation portion of the sensor is located in sealed space formed by using the sensor sealing assembly. In addition, the implantation portion of the sensor is located in the hollow cavity of the sealing bottle, and the sealing bottle may isolate and protect the implantation portion of the sensor, to avoid damage to a sensing layer of the implantation portion in a subsequent assembly process of the sensor and another component, thereby helping improve reliability and detection accuracy of the physiological sign detection apparatus.

In some possible implementations, at least a partial region of the sealing bottle allows ionizing radiation to pass through.

In this application, at least the partial region of the sealing bottle allows ionizing radiation to pass through, so that the sensor sealing assembly can sterilize the puncture end of the puncture needle and the implantation portion of the sensor through irradiation. In addition, after sterilization, the puncture end of the puncture needle and the implantation portion of the sensor are still located in the sealed space formed by using the sensor sealing assembly. As a sterile barrier, the sealing bottle may form good isolation protection for the puncture end of the puncture needle and the implantation portion of the sensor. The puncture end of the puncture needle and the implantation portion of the sensor are not contaminated for a second time. In subsequent packaging, a sterilized package for ensuring that the sensor is located in a sterile environment does not need to be additionally added to the physiological sign detection apparatus, to reduce costs. In addition, because the sensor sealing assembly may be independent of an internal circuit of the sensor assembly, it is possible to perform only radiation sterilization on the sensor sealing assembly, and no additional shielding protection needs to be set. Sterilization operations are simple, and an internal circuit of the sensor is not damaged. In addition, the sensor sealing assembly has a small volume, and a plurality of sensor sealing assemblies can be sterilized simultaneously through one radiation sterilization operation, to greatly improve sterilization efficiency and reducing sterilization costs.

In some possible implementations, the sensor and the sensor base are integrally formed by using an in-mold injection molding process, and a region that is of the sensor and that is in contact with the sensor base is covered with a heat insulation layer. In this application, the sensor and the sensor base are integrally formed by using the in-mold injection molding process, and the region that is of the sensor and that is in contact with the sensor base is covered with the heat insulation layer, to reduce heat conduction in a process of performing in-mold injection molding on the sensor and the sensor base, and prevent heat from being transferred from a fastening portion of the sensor to the implantation portion, so as to prevent an enzyme at the sensing layer of the implantation portion from being deactivated due to a high temperature, thereby helping improve detection accuracy of the physiological sign detection apparatus.

In some possible implementations, the sensor assembly further includes the upper cover, a battery, a connector, a circuit board, and a lower cover, the upper cover is fastened to the lower cover, accommodation space is formed between the upper cover and the lower cover, the battery, the connector, the circuit board, and a connecting portion of the sensor are located in the accommodation space, the circuit board is fastened to the lower cover, the battery and the connector are fastened to the circuit board and are electrically connected to the circuit board, and the sensor is electrically connected to the connector.

In this application, the circuit board and the lower cover form an integral mechanical part by using an adhesive process, so that the circuit board and a bottom plate of the lower cover are stably fastened, to avoid a collision caused by movement of the circuit board relative to the lower cover in a transportation process of the physiological sign detection apparatus, and help improve reliability of the physiological sign detection apparatus.

In some possible implementations, the connector includes a connecting groove, a plurality of first spring plates, and a plurality of second spring plates, the connecting groove includes a first groove wall and a second groove wall that are disposed opposite to each other, the plurality of first spring plates are convexly disposed on the first groove wall, the plurality of second spring plates are convexly disposed on the second groove wall, and the connecting portion of the sensor is snap-fitted into the connecting groove of the connector and is electrically connected to the plurality of first spring plates and/or the plurality of second spring plates.

In this application, at least a part of the plurality of first spring plates of the connector is convexly disposed on the first groove wall of the connecting groove, and at least a part of the plurality of second spring plates is convexly disposed on the second groove wall of the connecting groove. A conductive layer on a surface of the sensor can be electrically connected to the plurality of first spring plates and the plurality of second spring plates only by snap-fitting the sensor into the connecting groove of the connector. Assembly between the sensor and the connector is simpler, and an electrical connection between the sensor and the connector is more stable. In addition, the sensor can maintain a plate-like structure, and does not need to be folded additionally due to an electrical connection requirement, so that the sensor has high reliability. In addition, because spring plates are convexly disposed on groove walls on two sides of the connecting groove, the connector has more contacts configured to be electrically connected to the sensor, to reduce an arrangement area of the first spring plate and the second spring plate, thereby reducing a volume of the connector, helping miniaturize the sensor assembly. In addition, miniaturization of the sensor assembly also helps miniaturize the physiological sign detection apparatus.

In some possible implementations, the first spring plate and the second spring plate each are formed by bending a metal plate, a plate surface of the first spring plate is in contact with the sensor, and a plate surface of the second spring plate is in contact with the sensor.

In this application, the plate surface of the first spring plate is in contact with the sensor, and the plate surface of the second spring plate is in contact with the sensor. The sensor is in face contact with the first spring plate and the second spring plate, a contact area is large, the sensor is subject to an even force, and the sensor is not easily damaged. In addition, when a tail end of the connecting portion of the sensor is snap-fitted into a first positioning member, the tail end may move slightly in a limiting groove. Therefore, the connecting portion of the sensor is electrically connected to an abutting portion of the first spring plate and an abutting portion of the second spring plate, and a position of the connecting portion of the sensor may be slightly adjusted to implement self-adaptation, to better abut against the first spring plate and the second spring plate, so that a connecting position of the sensor is subject to an even force, thereby ensuring that the sensor is stably connected to the first spring plate and the second spring plate, to reduce fatigue caused by an uneven force applied to the first spring plate and the second spring plate for a long period of time.

In some possible implementations, the lower cover has a first through hole, the upper cover has a second through hole, the second through hole directly faces and communicates with the first through hole, and the sensor base is snap-fitted into the lower cover and/or the upper cover, the sensor base is exposed from the first through hole and is exposed from the second through hole. In this application, the lower cover has the first through hole, and the sensor base is exposed from the first through hole, so that the sealing bottle can pass through the first through hole and is exposed from the lower cover. The upper cover has the second through hole, and the sensor base is exposed from the second through hole, so that the needle base can pass through the second through hole of the upper cover. In this way, components of the sensor sealing assembly are assembled, and then assembled with the lower cover and the upper cover, to protect the implantation portion of the sensor by the sealing bottle and reduce a damage risk.

In some possible implementations, the sensor assembly further includes a bonding member, the bonding member has a first surface and a second surface that are disposed facing away from each other, and the first surface of the bonding member is stuck on a side that is of the lower cover and that faces away from the upper cover. When the sensor assembly abuts against the organism, the second surface of the bonding member is stuck on the organism.

In some possible implementations, the physiological sign detection apparatus further includes a thin film, the housing cap is provided with a through hole, the thin film covers the through hole, and the thin film is made of a waterproof and breathable material.

In this application, the physiological sign detection apparatus may be sterilized by using epoxyethane. A waterproof and breathable thin film allows gas to pass through and isolates water vapor. An epoxyethane gas may pass through the thin film, and enter an inner side of the physiological sign detection apparatus through the through hole of the housing cap, to perform overall sterilization on all components on the inner side of the physiological sign detection apparatus.

In some possible implementations, the physiological sign detection apparatus further includes a sealing member, and the housing cap is hermetically connected to the housing by using the sealing member.

In this application, the sealing member is hermetically connected to the housing and the housing cap, to help improve sealing performance between the housing cap and the housing, and prevent a contaminant from entering the inner side of the physiological sign detection apparatus. In addition, a sealed environment is formed inside the physiological sign detection apparatus, to prevent the bonding member from being interfered by an external environment in a storage process, and help maintain bonding performance of the bonding member.

In some possible implementations, the physiological sign detection apparatus further includes a bonding member, the bonding member has a first surface and a second surface that are disposed facing away from each other, and the first surface of the bonding member is stuck on the abutting end and covers the sensor assembly. When the sensor assembly abuts against the organism, the first surface of the bonding member is stuck on the sensor assembly, and the second surface of the bonding member is stuck on the organism.

In this application, the first surface of the bonding member is stuck on the abutting end of the base, and covers the sensor assembly. The bonding member shields the puncture needle. When the user removes the housing cap, the puncture needle and the sensor cannot be seen in a field of view of the user, to help eliminate trypanophobia of the user and improve user experience.

### BRIEF DESCRIPTION OF DRAWINGS

FIG. 1 is a diagram of a physiological sign detection apparatus in some application scenarios according to this application;
FIG. 2 is a diagram of a structure of the physiological sign detection apparatus shown in FIG. 1 in some embodiments;
FIG. 3 is a diagram of a partial exploded structure of the physiological sign detection apparatus shown in FIG. 2;
FIG. 4 is a diagram of a structure of a housing of the physiological sign detection apparatus shown in FIG. 3 from another perspective;
FIG. 5A is a diagram of a structure of a base of the physiological sign detection apparatus shown in FIG. 3;
FIG. 5B is a diagram of a structure of the base shown in FIG. 5A from another perspective;
FIG. 6 is a diagram 1 of a partial cross-sectional structure after the physiological sign detection apparatus shown in FIG. 2 is cut along A-A;
FIG. 7 is a diagram 1 of a partial cross-sectional structure after the physiological sign detection apparatus shown in FIG. 2 is cut along B-B;
FIG. 8A is a diagram of a structure of an implantation base shown in FIG. 3;
FIG. 8B is a diagram of a structure of the implantation base shown in FIG. 3 from another perspective;
FIG. 9A is a diagram of a structure of a needle extraction base shown in FIG. 3;
FIG. 9B is a diagram of a structure of the needle extraction base shown in FIG. 9A from another perspective;
FIG. 10 is a diagram of a structure of a stopper member shown in FIG. 3;
FIG. 11 is a diagram 2 of a partial cross-sectional structure after the physiological sign detection apparatus shown in FIG. 2 is cut along A-A;
FIG. 12 is a diagram 2 of a partial cross-sectional structure after the physiological sign detection apparatus shown in FIG. 2 is cut along B-B;
FIG. 13 is a diagram of a structure of a sensor assembly shown in FIG. 2 in some embodiments;
FIG. 14 is a diagram of a partial exploded structure of the sensor assembly shown in FIG. 13;
FIG. 15 is a diagram of a partial exploded structure of a sensor sealing assembly shown in FIG. 14;
FIG. 16A is a diagram of an assembly structure of a sensor and a sensor base that are shown in FIG. 15 in some embodiments;
FIG. 16B is a diagram of a structure of the sensor shown in FIG. 16A;
FIG. 17A is a diagram of an assembly structure of a sensor and a sensor base that are shown in FIG. 15 in some other embodiments;
FIG. 17B is a diagram of an exploded structure of the structure shown in 17A;
FIG. 18 is a diagram of an assembly structure of a puncture needle and a needle base that are shown in FIG. 15;
FIG. 19 is a diagram of a structure of a sealing bottle shown in FIG. 15 from another perspective;
FIG. 20 is a diagram of a cross-sectional structure after a sensor sealing assembly shown in FIG. 14 is cut along C-C;
FIG. 21 is a diagram of a sterilization tooling structure of a sensor sealing assembly shown in FIG. 14;
FIG. 22 is a diagram of a partial structure of the sensor assembly shown in FIG. 13;
FIG. 23 is a diagram of an assembly structure of a sensor and a connector that are shown in FIG. 22;
FIG. 24 is a diagram of a cross-sectional structure after the sensor assembly shown in FIG. 13 is cut along D-D in some embodiments;
FIG. 25 is a diagram of a cross-sectional structure after the sensor assembly shown in FIG. 13 is cut along E-E in some embodiments;
FIG. 26 is a diagram 3 of a partial cross-sectional structure after the physiological sign detection apparatus shown in FIG. 2 is cut along B-B;
FIG. 27 is a diagram of a structure of a housing cap shown in FIG. 2 in some embodiments;
FIG. 28 is a diagram of a structure of a drying member;
FIG. 29 is a diagram of an assembly structure of a drying member and a housing cap;
FIG. 30 is a diagram of an assembly structure of a magnetic element, a drying member, and a housing cap;
FIG. 31 is a diagram 4 of a partial cross-sectional structure after the physiological sign detection apparatus shown in FIG. 2 is cut along B-B;
FIG. 32 is a diagram of a partial cross-sectional structure after the physiological sign detection apparatus shown in FIG. 2 is cut along F-F;
FIG. 33 is a diagram of a partial cross-sectional structure after the physiological sign detection apparatus shown in FIG. 2 is cut along B-B in a pressing process;
FIG. 34 is a diagram of a partial cross-sectional structure after the physiological sign detection apparatus shown in FIG. 2 is cut along A-A in a pressing process;
FIG. 35 is a diagram of a partial cross-sectional structure after the physiological sign detection apparatus shown in FIG. 2 is cut along A-A in a puncturing process;
FIG. 36 is a diagram of a partial cross-sectional structure after the physiological sign detection apparatus shown in FIG. 2 is cut along B-B in a puncturing process;
FIG. 37 is a diagram of a partial cross-sectional structure after the physiological sign detection apparatus shown in FIG. 2 is cut along B-B in a needle extraction state;
FIG. 38 is a diagram in which a sensor assembly is stuck on an organism;
FIG. 39 is a diagram of a structure of the physiological sign detection apparatus shown in FIG. 1 in some other embodiments;
FIG. 40 is a diagram of a partial exploded structure of the physiological sign detection apparatus shown in FIG. 39;
FIG. 41 is a diagram of a structure of a housing shown in FIG. 40 from another perspective;
FIG. 42 is a diagram of a structure of a base shown in FIG. 40 from another perspective;
FIG. 43 is a diagram 1 of a partial cross-sectional structure after the physiological sign detection apparatus shown in FIG. 39 is cut along G-G;
FIG. 44 is a diagram 1 of a partial cross-sectional structure after the physiological sign detection apparatus shown in FIG. 39 is cut along H-H;
FIG. 45A is a diagram of a structure of an implantation base shown in FIG. 40;
FIG. 45B is a diagram of a structure of the implantation base shown in FIG. 45A from another perspective;
FIG. 46 is a diagram 2 of a partial cross-sectional structure after the physiological sign detection apparatus shown in FIG. 39 is cut along G-G;
FIG. 47 is a diagram 2 of a partial cross-sectional structure after the physiological sign detection apparatus shown in FIG. 39 is cut along H-H;
FIG. 48 is a diagram of a partial cross-sectional structure after the physiological sign detection apparatus shown in FIG. 39 is cut along H-H in a pressing process;
FIG. 49 is a diagram of a partial cross-sectional structure after the physiological sign detection apparatus shown in FIG. 39 is cut along G-G in a pressing process;
FIG. 50 is a diagram of a partial cross-sectional structure after the physiological sign detection apparatus shown in FIG. 39 is cut along G-G in a puncturing process;
FIG. 51 is a diagram of a partial cross-sectional structure after the physiological sign detection apparatus shown in FIG. 39 is cut along H-H in a puncturing process;
FIG. 52 is a diagram of a partial cross-sectional structure after the physiological sign detection apparatus shown in FIG. 39 is cut along H-H in a needle extraction state;
FIG. 53 is a diagram of a structure of the physiological sign detection apparatus shown in FIG. 1 in some other embodiments;
FIG. 54 is a diagram of a partial exploded structure of the physiological sign detection apparatus shown in FIG. 53;
FIG. 55 is a diagram of a structure of a sensor assembly shown in FIG. 54 in some embodiments;
FIG. 56A is a diagram of a structure of a needle base shown in FIG. 55;
FIG. 56B is a diagram of an assembly structure of a snap-fit and a needle base in some embodiments;
FIG. 57 is a diagram of a structure of a sealing bottle shown in FIG. 55 from another perspective;
FIG. 58 is a diagram of a structure of the sensor sealing assembly shown in FIG. 55;
FIG. 59 is a diagram of a cross-sectional structure after the sensor assembly shown in FIG. 55 is cut along I-I;
FIG. 60 is a diagram of a cross-sectional structure after the sensor assembly shown in FIG. 55 is cut along J-J;
FIG. 61 is a diagram of a structure of a housing cap shown in FIG. 54 in some embodiments;
FIG. 62 is a diagram of a partial cross-sectional structure after the physiological sign detection apparatus shown in FIG. 53 is cut along K-K;
FIG. 63 is a diagram of a partial cross-sectional structure after the physiological sign detection apparatus shown in FIG. 53 is cut along L-L;
FIG. 64 is a diagram of a structure of a sensor assembly shown in FIG. 2 in some other embodiments;
FIG. 65 is a diagram of a partial exploded structure of the sensor assembly shown in FIG. 64;
FIG. 66 is a diagram of a partial exploded structure of a sensor sealing assembly shown in FIG. 64;
FIG. 67 is a diagram of a structure of an upper cover shown in FIG. 66 from another perspective;
FIG. 68 is a diagram of a cross-sectional structure after a sensor sealing assembly shown in FIG. 65 is cut along M-M;
FIG. 69 is a diagram of a sterilization tooling structure of a sensor sealing assembly shown in FIG. 65;
FIG. 70A is a diagram of a structure of a connector shown in FIG. 65 in some embodiments;
FIG. 70B is a diagram of a structure of the connector shown in FIG. 70A from another perspective;
FIG. 71 is a diagram of an exploded structure of the connector shown in FIG. 70B;
FIG. 72 is a diagram of a partial structure of the sensor assembly shown in FIG. 64;
FIG. 73 is a diagram of a cross-sectional structure after the sensor assembly shown in FIG. 64 is cut along N-N;
FIG. 74 is a diagram of a structure of the physiological sign detection apparatus shown in FIG. 1 in some other embodiments;
FIG. 75 is a diagram of a partial exploded structure of the physiological sign detection apparatus shown in FIG. 74;
FIG. 76 is a diagram of a partial cross-sectional structure after the physiological sign detection apparatus shown in FIG. 74 is cut along O-O; and
FIG. 77 is a diagram of a partial cross-sectional structure after the physiological sign detection apparatus shown in FIG. 74 is cut along O-O in a puncturing process.

### DESCRIPTION OF EMBODIMENTS

The following describes the technical solutions in embodiments of this application with reference to the accompanying drawings. In descriptions of embodiments of this application, unless otherwise specified, "/" indicates "or". For example, A/B may indicate A or B. The term "and/or" in this specification merely describes an association relationship for describing associated objects, and indicates that three relationships may exist. For example, A and/or B may indicate the following three cases: Only A exists, both A and B exist, and only B exists. In addition, in the descriptions of embodiments of this application, "a plurality of" means two or more.

In the following, terms such as "first" and "second" are used only for description purposes, and cannot be understood as implying or implying relative importance or implicitly indicating a quantity of indicated technical features. Therefore, a feature limited by "first" or "second" may explicitly or implicitly include one or more features.

Orientation terms mentioned in embodiments of this application, for example, "up", "down", "inside", "outside", "side surface", "top", and "bottom", are merely directions in the accompanying drawings. Therefore, the orientation terms are used to better and more clearly describe and understand embodiments of this application, instead of indicating or implying that a specified apparatus or element needs to have a specific orientation, and be constructed and operated in the specific orientation. Therefore, this cannot be understood as a limitation on embodiments of this application.

In the description of embodiments of this application, it should be noted that, unless otherwise specified and limited, terms "installation", "connecting", "connection", and "disposed on ..." should be understood in a broad sense. For example, "connection" may be a detachable connection, or may be an undetachable connection, or may be a direct connection, or may be an indirect connection by using an intermediate medium. "Fastening" means that two parties are connected to each other and a relative position relationship remains unchanged after the two parties are connected to each other. "Rotatable connection" means that two parties are connected to each other and can rotate relative to each other after the two parties are connected to each other. "Slidable connection" means that two parties are connected to each other and can slide relative to each other after the two parties are connected to each other.

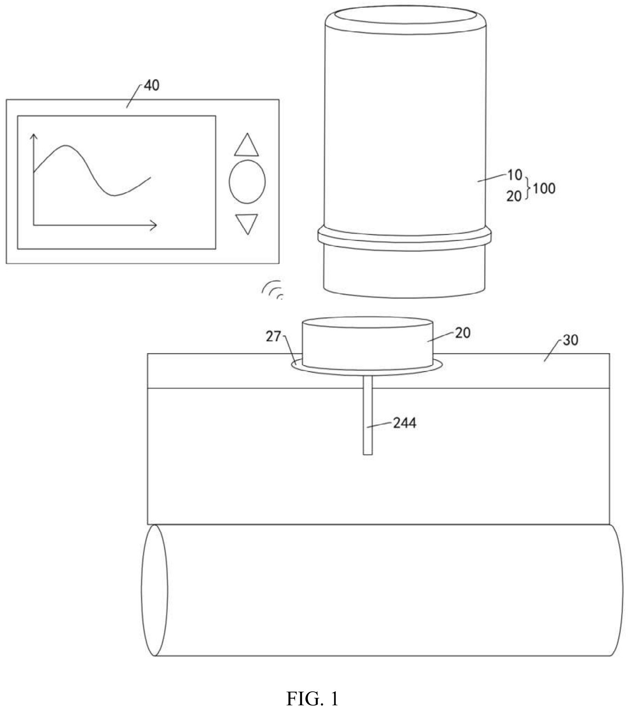

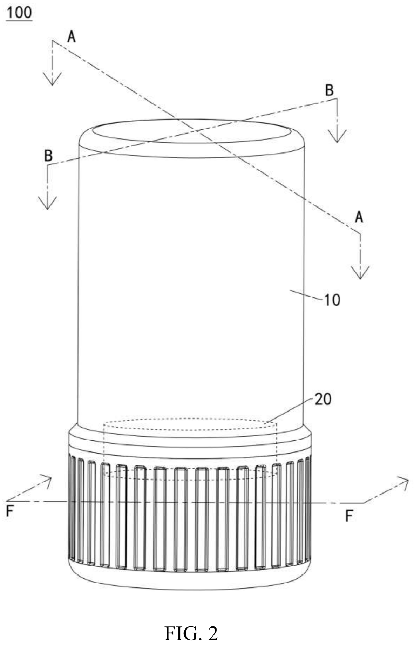

FIG. 1 is a diagram of a physiological sign detection apparatus 100 in some application scenarios according to this application. FIG. 2 is a diagram of a structure of the physiological sign detection apparatus 100 shown in FIG. 1 in some embodiments.

In some embodiments, the physiological sign detection apparatus 100 may include an implantation apparatus 10 and a sensor assembly 20. The sensor assembly 20 is detachably mounted on an inner side of the implantation apparatus 10. The implantation apparatus 10 is configured to push the sensor assembly 20 to a target monitoring position (for example, skin of a belly or a fat-rich part of an arm of the organism 30) on an organism 30. The sensor assembly 20 may include a sensor 244 and a bonding member 27. After the implantation apparatus 10 pushes away the sensor assembly 20, the sensor assembly 20 is fastened to the organism 30 by using the bonding member 27 at the bottom of the sensor assembly 20. A part of the sensor 244 of the sensor assembly 20 extends from the sensor assembly 20 and is implanted into subcutaneous tissue of skin of the organism 30, to collect and transfer blood information, so that the sensor 244 can detect and quantify one or more physiological target substances such as blood glucose, lactic acid, uric acid, dissolved oxygen, hydrogen peroxide, and ions. The sensor assembly 20 may transmit information to a terminal device 40 in a short-range transmission manner such as Bluetooth or a short-range wireless communication technology (Near Field Communication, NFC). The terminal device 40 may be an electronic device with a display, for example, a display instrument, a mobile phone, or an electronic watch. After the sensor 244 is implanted into the organism 30, setting is performed by using an application (Application, APP) on the terminal device 40, to start detection of the physiological target substance such as blood glucose. The sensor assembly 20 transfers collected physiological target substance data to the terminal device 40. The terminal device 40 is configured to observe information such as a concentration of the physiological target substance, to detect and manage a physical and chemical indicator such as blood glucose. The sensor assembly 20 is a consumable, and one sensor assembly 20 may be used for at least 14 consecutive days.

In this embodiment, the physiological sign detection apparatus 100 is an integral apparatus. The sensor assembly 20 is mounted on an inner side of the implantation apparatus 10 in advance and then packaged. A user does not need to assemble the implantation apparatus 10 and the sensor assembly 20 in a use process. The user only needs to open one package, and attach the physiological sign detection apparatus 100 to the target monitoring position on the organism 30. The sensor assembly 20 is pushed to the target monitoring position by using the implantation apparatus 10 of the physiological sign detection apparatus 100. The integral physiological sign detection apparatus 100 helps simplify steps of user operations, and can avoid a biological contamination risk caused in an assembly process of the user while facilitating the user operations. In addition, the physiological sign detection apparatus 100 needs only one package, and the sensor assembly 20 and the implantation apparatus 10 do not need to be separately packaged, to help reduce product costs and reduce packages and waste.

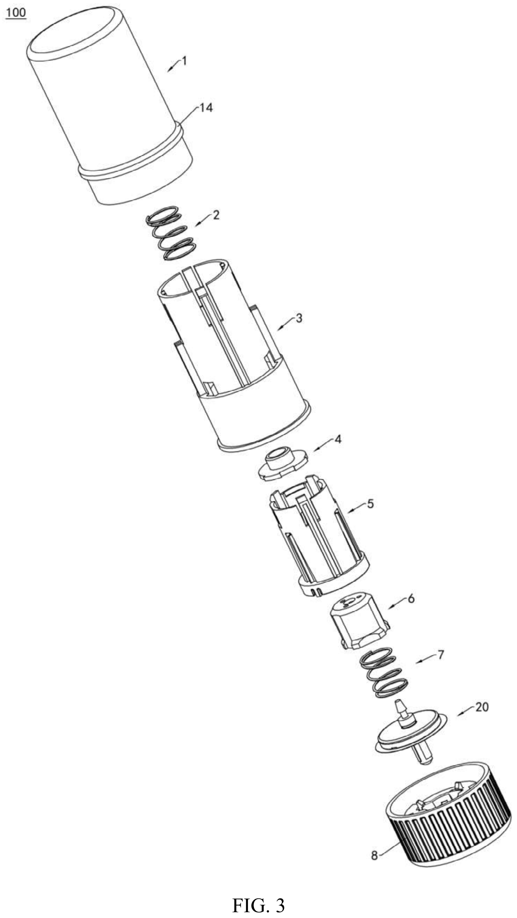

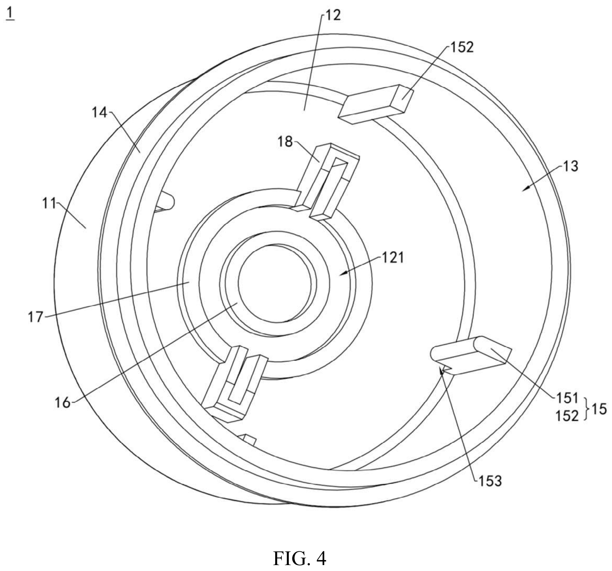

FIG. 3 is a diagram of a partial exploded structure of the physiological sign detection apparatus 100 shown in FIG. 2. FIG. 4 is a diagram of a structure of a housing 1 of the physiological sign detection apparatus 100 shown in FIG. 3 from another perspective.

In some embodiments, the implantation apparatus 10 of the physiological sign detection apparatus 100 may include the housing 1, a first elastic member 2, a base 3, a stopper member 4, an implantation base 5, a needle extraction base 6, a second elastic member 7, and a housing cap 8. In some other embodiments, the implantation apparatus 10 may include more or fewer components, or some components may be combined or split.

In some embodiments, the housing 1 may include a side wall 11 and a top wall 12, and the side wall 11 of the housing 1 is annularly connected to a circumferential edge of the top wall 12 of the housing 1. An opening 13 is formed on a side that is of the side wall 11 of the housing 1 and that is away from the top wall 12 of the housing 1. The housing 1 has an inner cavity, and the inner cavity communicates with the opening 13. The housing 1 may be generally cylindrical. The housing 1 has an axial direction, the side wall 11 of the housing 1 is disposed around the axial direction of the housing 1, and the top wall 12 of the housing 1 intersects the axial direction of the housing 1.

For example, the housing 1 may further include a first flange 14, the first flange 14 is annularly fastened to an outer side surface of the side wall 11 of the housing 1, and the first flange 14 is disposed close to the opening 13 of the housing 1 relative to the top wall 12 of the housing 1. The housing 1 has an inner side and an outer side, and the outer side surface of the side wall 11 of the housing 1 is a surface of an outer side of the side wall 11 of the housing 1. The outer side surface in the following is understood similarly. Details are not described again in this application.

For example, the housing 1 may further include a first guiding structure 15, and the first guiding structure 15 is fastened to an inner side surface of the side wall 11 of the housing 1. The inner side surface of the side wall 11 of the housing 1 is a surface of an inner side of the side wall 11 of the housing 1. The inner side surface in the following is understood similarly. Details are not described again in this application. The first guiding structure 15 may extend in the axial direction of the housing 1. There may be one or more first guiding structures 15. When there are a plurality of first guiding structures 15, the plurality of first guiding structures 15 are spaced apart around a circumferential direction of the side wall 11 of the housing 1. For example, the first guiding structure 15 may include a first sliding block 151 and a second sliding block 152, and the first sliding block 151 and the second sliding block 152 are spaced apart. Both the first sliding block 151 and the second sliding block 152 extend in the axial direction of the housing 1. Limiting space 153 is reserved between the first sliding block 151 and the top wall 12 of the housing 1. There may be one or more first sliding blocks 151 and one or more second sliding blocks 152. When there are a plurality of first sliding blocks 151 and a plurality of second sliding blocks 152, the plurality of first sliding blocks 151 and the plurality of second sliding blocks 152 are spaced apart around the circumferential direction of the side wall 11 of the housing 1, and the plurality of first sliding blocks 151 and the plurality of second sliding blocks 152 may be arranged alternately. For example, there are two first sliding blocks 151 and two second sliding blocks 152, the two first sliding blocks 151 are disposed opposite to each other, and the two second sliding blocks 152 are disposed opposite to each other.

For example, the housing 1 may further include a first limiting boss 16, a second limiting boss 17, and a toggle 18. The first limiting boss 16, the second limiting boss 17, and the toggle 18 are all fastened to a side that is of the top wall 12 of the housing 1 and that faces the opening 13 of the housing 1. For example, the first limiting boss 16 may be circular or ring-shaped. The second limiting boss 17 is located on an outer side of the first limiting boss 16, and is disposed around the first limiting boss 16. A limiting groove 121 is formed between the first limiting boss 16 and the second limiting boss 17. The toggle 18 is located on an outer side of the second limiting boss 17. There may be one or more toggles 18. When there are a plurality of toggles 18, the plurality of toggles 18 are spaced apart around a circumferential direction of the second limiting boss 17. For example, there are two toggles 18, and the two toggles 18 are disposed opposite to each other. The toggle 18 is provided with an open groove, and the open groove of the toggle 18 helps reduce consumables for manufacturing the housing 1 and reduce a weight of the housing 1.

In some embodiments, the housing 1 may be an integral mechanical part. The housing 1 may be made of engineering plastic such as polycarbonate (Polycarbonate, PC) or a polycarbonate and acrylonitrile butadiene styrene (Acrylonitrile Butadiene Styrene plastic, ABS) mixture.

In this embodiment, the polycarbonate and acrylonitrile butadiene styrene plastic have features such as heat resistance, impact resistance, good mechanical performance, high hardness, and good processing performance. The housing 1 is made of the engineering plastic such as the polycarbonate or the polycarbonate and acrylonitrile butadiene styrene mixture, to help reduce manufacturing costs of the housing 1. In addition, the housing 1 has a strong scratch resistance capability. As an outer layer structure of the physiological sign detection apparatus 100, the housing 1 can prevent a surface of the physiological sign detection apparatus 100 from being scratched, to help improve external aesthetics of using the physiological sign detection apparatus 100.

In some other embodiments, the housing 1 may alternatively be made of another material, for example, engineering plastic such as polyvinyl chloride resin or polystyrene resin, or metal such as stainless steel or aluminum alloy. This is not strictly limited in this application.

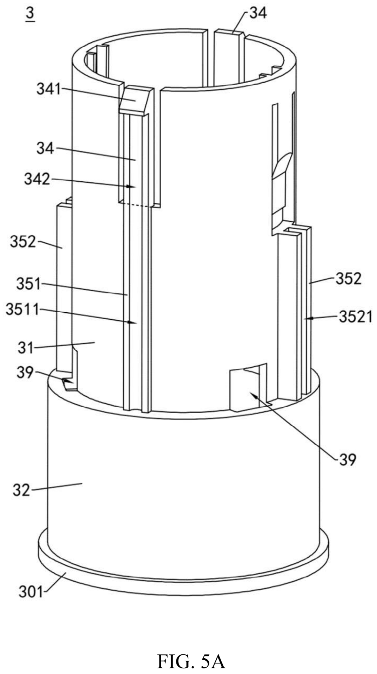

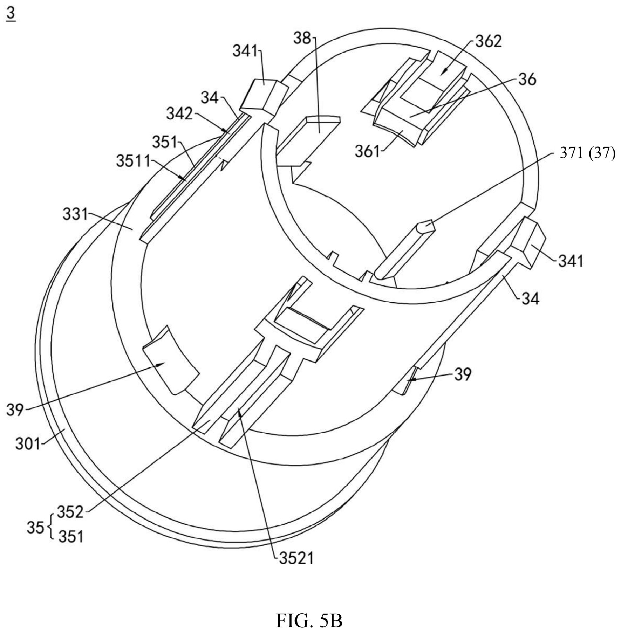

FIG. 5A is a diagram of a structure of the base 3 of the physiological sign detection apparatus 100 shown in FIG. 3. FIG. 5B is a diagram of a structure of the base 3 shown in FIG. 5A from another perspective.

In some embodiments, the base 3 may include an upper side wall 31, a lower side wall 32, and a connecting wall 33. The connecting wall 33 of the base 3 is connected to the upper side wall 31 of the base 3 and the lower side wall 32 of the base 3. On an outer side of the base 3, a step surface 331 is formed between the upper side wall 31 of the base 3 and the lower side wall 32 of the base 3, and the step surface 331 is an upper surface of the connecting wall 33 of the base 3. A direction from the upper side wall 31 of the base 3 to the lower side wall 32 of the base 3 is a top-down direction. For example, the upper side wall 31 of the base 3 is located above the lower side wall 32 of the base 3. The base 3 may be generally cylindrical. The base 3 may further include an abutting end 301, and the abutting end 301 is fastened to an end that is of the lower side wall 32 of the base 3 and that faces away from the upper side wall 31 of the base 3, and is disposed around an outer circumferential edge of the lower side wall 32 of the base 3.

In some embodiments, the base 3 further includes a first elastic arm 34, an end that is of the first elastic arm 34 and that faces away from the abutting end 301 is provided with a hook 341, and the hook 341 of the first elastic arm 34 protrudes from an outer side surface of the base 3. The other end of the first elastic arm 34 is fastened to the upper side wall 31 of the base 3. The other end of the first elastic arm 34 is an end that is of the first elastic arm 34 and that faces away from the hook 341. The first elastic arm 34 is spaced apart from a side wall of the base 3 in the circumferential direction, the hook 341 of the first elastic arm 34 is provided with an inclined end surface, and the inclined end surface of the first elastic arm 34 is inclined relative to an axial direction of the base 3.

In some embodiments, the base 3 may further include a second guiding structure 35, and the second guiding structure 35 is fastened to the outer side surface of the base 3. The second guiding structure 35 may extend in the axial direction of the base 3. For example, the second guiding structure 35 may include a first guiding arm 351, and the first guiding arm 351 is fastened to an outer side surface of the upper side wall 31 of the base 3. The first guiding arm 351 has a sliding groove 3511, and an opening of the sliding groove 3511 of the first guiding arm 351 faces away from the side wall of the base 3. The sliding groove 3511 of the first guiding arm 351 extends in the axial direction of the base 3, and the sliding groove 3511 of the first guiding arm 351 may be a strip-shaped groove. The first guiding arm 351 may extend from the step surface 331 to the first elastic arm 34, the first elastic arm 34 has a sliding groove 342, and the sliding groove 3511 of the first guiding arm 351 communicates with the sliding groove 342 of the first elastic arm 34.

In some embodiments, the second guiding structure 35 may further include a second guiding arm 352, the second guiding arm 352 is fastened to the outer side surface of the upper side wall 31 of the base 3, the second guiding arm 352 has a sliding groove 3521, and an opening of the sliding groove 3521 of the second guiding arm 352 faces away from the side wall of the base 3. The sliding groove 3521 of the second guiding arm 352 extends in the axial direction of the base 3, and the sliding groove 3521 of the second guiding arm 352 may be a strip-shaped groove. There may be one or more first guiding arms 351 and/or one or more second guiding arms 352, and the second guiding arms 352 and the first guiding arms 351 may be arranged alternately in the circumferential direction of the base 3.

In some embodiments, the base 3 may further include a second elastic arm 36, the second elastic arm 36 is located on a side that is of the base 3 and that faces away from the abutting end 301, and a tail end of the second elastic arm 36 is provided with a snap-fitting hook 361. The tail end of the second elastic arm 36 is an end that is of the second elastic arm 36 and that is close to the abutting end 301. The other end of the second elastic arm 36 is fastened to the upper side wall 31 of the base 3, and the second elastic arm 36 is spaced apart from the upper side wall 31 of the base 3 in the circumferential direction. The snap-fitting hook 361 of the second elastic arm 36 is located on an inner side of the base 3 and faces away from the abutting end 301. There may be one or more second elastic arms 36, and a plurality of second elastic arms 36 may be spaced apart around the axial direction of the base 3. For example, there are two second elastic arms 36, and the two second elastic arms 36 are disposed opposite to each other. For example, the second elastic arm 36 may be provided with a guiding groove 362, and an opening of the guiding groove 362 of the second elastic arm 36 faces the inner side of the base 3.

In some embodiments, the base 3 may further include a third guiding structure 37, and the third guiding structure 37 is fastened to an inner side surface of the base 3. The third guiding structure 37 extends in the axial direction of the base 3. For example, there may be one or more third guiding structures 37. When there are a plurality of third guiding structures 37, the plurality of third guiding structures 37 are spaced apart around the circumferential direction of the side wall 11 of the housing 1. The third guiding structure 37 may be a third sliding block 371, and the third sliding block 371 is convexly disposed on an inner side surface of the upper side wall 31 of the base 3. For example, there may be three third sliding blocks 371. The third sliding block 371 is located at an end that is of the upper side wall 31 of the base 3 and that is close to the lower side wall 32 of the base 3.

The base 3 may further include a baffle plate 38, and the baffle plate 38 is convexly disposed on the inner side surface of the upper side wall 31 of the base 3. For example, there may be one or more baffle plates 38. When there are a plurality of baffle plates 38, the plurality of baffle plates 38 are spaced apart around the circumferential direction of the base 3. The baffle plate 38 is located at an end that is of the upper side wall 31 of the base 3 and that is close to the connecting wall 33 of the base 3. The baffle plate 38 and the third sliding block 371 are spaced apart. The base 3 further has avoidance space 39. The avoidance space 39 runs through the upper side wall 31 of the base 3, and extends from a bottom side of the baffle plate 38 to the connecting wall 33 of the base 3. There may be one or more pieces of avoidance space 39. For example, there are three baffle plates 38 and three pieces of avoidance spaces 39.

In some embodiments, the base 3 may be an integral mechanical part. The base 3 is made of engineering plastic such as polycarbonate or a polycarbonate and acrylonitrile butadiene styrene mixture. In this embodiment, the polycarbonate and acrylonitrile butadiene styrene plastic have features such as heat resistance, impact resistance, good mechanical performance, high hardness, and good processing performance. The base 3 is made of the engineering plastic such as the polycarbonate or the polycarbonate and acrylonitrile butadiene styrene mixture, to help reduce manufacturing costs of the base 3, and ensure mechanical strength of the base 3.

In some other embodiments, the base 3 may alternatively be made of another material, for example, engineering plastic such as polyvinyl chloride resin or polystyrene resin, or metal such as stainless steel or aluminum alloy. This is not strictly limited in this application.

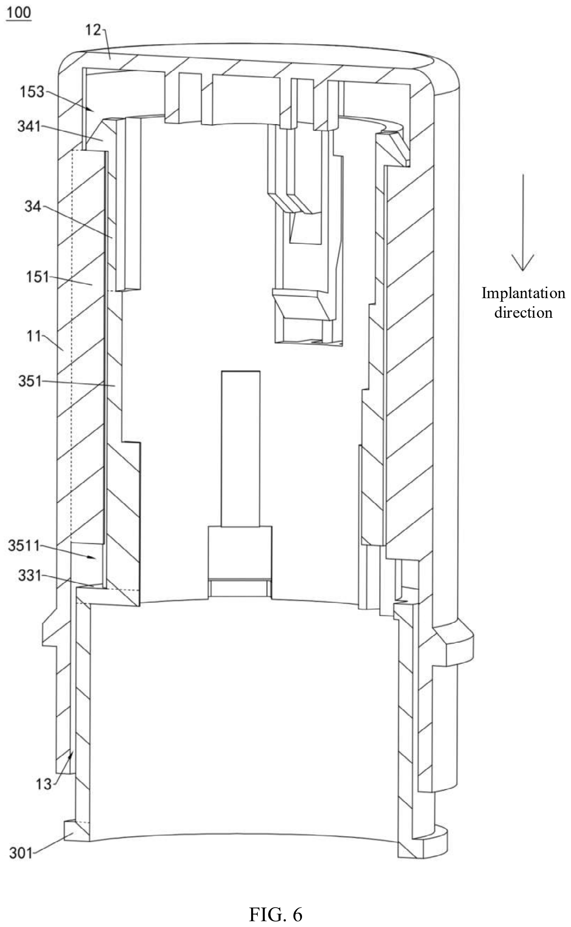

FIG. 6 is a diagram 1 of a partial cross-sectional structure after the physiological sign detection apparatus 100 shown in FIG. 2 is cut along A-A.

In some embodiments, the base 3 is mounted on an inner side of the housing 1 and is slidably connected to the side wall 11 of the housing 1, the axial direction of the base 3 coincides with the axial direction of the housing 1, and the abutting end 301 of the base 3 protrudes from the housing 1 through the opening 13 of the housing 1. The hook 341 of the first elastic arm 34 is snap-fitted into the side wall 11 of the housing 1, to limit movement of the housing 1 in a direction away from the abutting end 301 relative to the base 3. For example, the hook 341 of the first elastic arm 34 may be snap-fitted into the first sliding block 151, and the hook 341 of the first elastic arm 34 is mounted in the limiting space 153 between the first sliding block 151 and the top wall 12 of the housing 1. The first sliding block 151 abuts against the hook 341 of the first elastic arm 34, to limit movement of the housing 1 in the direction away from the abutting end 301 relative to the base 3. In this embodiment, the hook 341 of the first elastic arm 34 limits the first sliding block 151 in the axial direction, to prevent the housing 1 from moving in a direction away from the abutting end 301 relative to the base 3, and prevent the housing 1 from being detached from the base 3.

In addition, the first elastic arm 34 is spaced apart from the side wall of the base 3 in the circumferential direction, and the hook 341 of the first elastic arm 34 is provided with an inclined end surface. When the base 3 is assembled with the housing 1, the first elastic arm 34 may elastically avoid the first sliding block 151 in a radial direction, and the inclined end surface of the first elastic arm 34 avoids the first sliding block 151 in the axial direction, so that the base 3 can be smoothly mounted on the inner side of the housing 1, to reduce snap-fitting resistance, and further avoid damaging the base 3 and the housing 1 during assembly.

In some other embodiments, the side wall 11 of the housing 1 may also have snap-fitting space or a snap-fitting block, and the first elastic arm 34 can also be snap-fitted into the housing 1 by snap-fitting the hook 341 of the first elastic arm 34 into the snap-fitting space or the snap-fitting block of the housing 1.

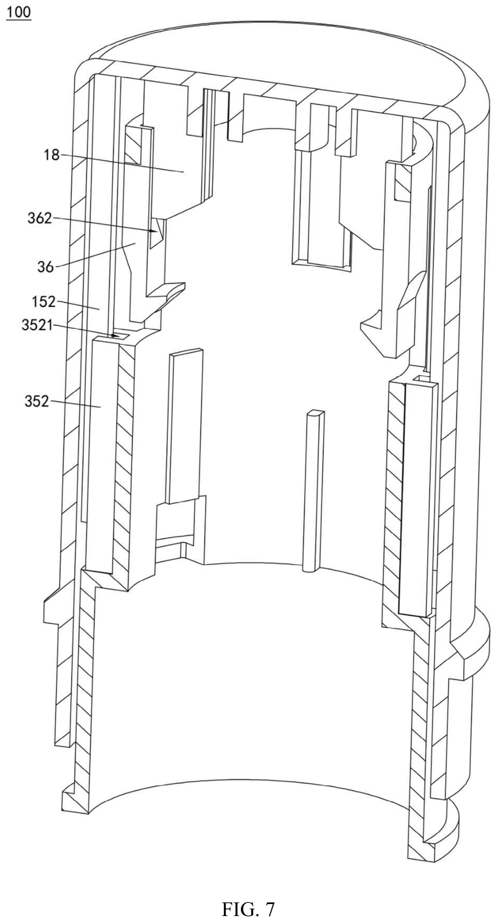

Refer to FIG. 6 and FIG. 7 together. FIG. 7 is a diagram 1 of a partial cross-sectional structure after the physiological sign detection apparatus 100 shown in FIG. 2 is cut along B-B.

In some embodiments, the first guiding structure 15 is slidably connected to the second guiding structure 35 (as shown in FIG. 4 and FIG. 5A). For example, the first sliding block 151 is mounted in the sliding groove 3511 of the first guiding arm 351 and the sliding groove 342 of the first elastic arm 34. In other words, the first sliding block 151 can slide between the step surface 331 and the hook 341 of the first elastic arm 34. A quantity of first guiding arms 351 matches a quantity of the first sliding blocks 151. The second sliding block 152 is mounted in the sliding groove 3521 of the second guiding arm 352, and the second sliding block 152 can slide in the sliding groove 3521 of the second guiding arm 352. A quantity of second guiding arms 352 matches a quantity of second sliding blocks 152.

In this embodiment, the first guiding structure 15 is slidably connected to the second guiding structure 35 for mutual cooperation, to guide the housing 1 and the base 3 to slide relative to each other in the axial direction of the housing 1, so that the housing 1 and the base 3 limit each other in the circumferential direction of the housing 1, and the base 3 is prevented from rotating relative to the housing 1 in the circumferential direction. When there are a plurality of first guiding structures 15 and a plurality of second guiding structures 35, this helps balance a force between the housing 1 and the base 3, to prevent the base 3 from being skewed relative to the housing 1. In addition, the first sliding block 151 can slide between a step surface 331 and the hook 341 of the first elastic arm 34, so that the housing 1 can move in the axial direction relative to the base 3, and movement of the housing 1 in the axial direction relative to the base 3 is limited, to limit a distance at which the housing 1 can move in an implantation direction relative to the base 3.

In some other embodiments, the first sliding block 151 may alternatively be disposed on the outer side surface of the base 3, and the first guiding arm 351 may alternatively be disposed on an inner side surface of the housing 1. The housing 1 and the base 3 can also limit each other in the circumferential direction through mutual cooperation between the first sliding block 151 of the base 3 and the first guiding arm 351 of the housing 1. This is not strictly limited in this application. In some other embodiments, the second sliding block 152 may alternatively be fastened to the outer side surface of the base 3, and the second guiding arm 352 may alternatively be fastened to the inner side surface of the side wall 11 of the housing 1. The housing 1 and the base 3 can limit each other in the circumferential direction through mutual cooperation between the second sliding block 152 and the second guiding arm 352. This is not strictly limited in this application. In some other embodiments, cooperation between the first guiding structure 15 and the second guiding structure 35 may alternatively not include cooperation between the first sliding block 151 and the first guiding arm 351, or may not include cooperation between the second sliding block 152 and the second guiding arm 352. This is not strictly limited in this application.

In some embodiments, the toggle 18 is located on a side that is of the top wall 12 of the housing 1 and that faces the base 3. When the housing 1 moves in a direction close to the abutting end 301 relative to the base 3, the toggle 18 pushes the second elastic arm 36, and the second elastic arm 36 moves toward the outer side of the base 3.

For example, a wall surface of the guiding groove 362 of the second elastic arm 36 may further include a guiding surface, the guiding surface is located on a side that is of the guiding groove 362 and that is close to the snap-fitting hook 361 of the second elastic arm 36, and the guiding surface is inclined relative to the axial direction of the base 3. At least a part of the toggle 18 is located in the guiding groove 362 of the second elastic arm 36, and an end of the toggle 18 is disposed close to the guiding surface of the guiding groove 362. When the toggle 18 moves in the direction close to the abutting end 301, the toggle 18 can move along the guiding groove 362. In this embodiment, the second elastic arm 36 is provided with the guiding groove 362, and the toggle 18 can move along the guiding groove 362, so that relative movement between the toggle 18 and the second elastic arm 36 is stable, to prevent the toggle 18 from being detached from the second elastic arm 36, thereby helping improve reliability of the physiological sign detection apparatus 100. In addition, when the toggle 18 moves in the direction close to the abutting end 301, the toggle 18 abuts against the guiding surface, and gradually pushes a tail end of the second elastic arm 36 along the guiding surface to move toward the outer side of the base 3, to help reduce movement resistance of the toggle 18, and further enable a movement action of the tail end of the second elastic arm 36 to be stable.

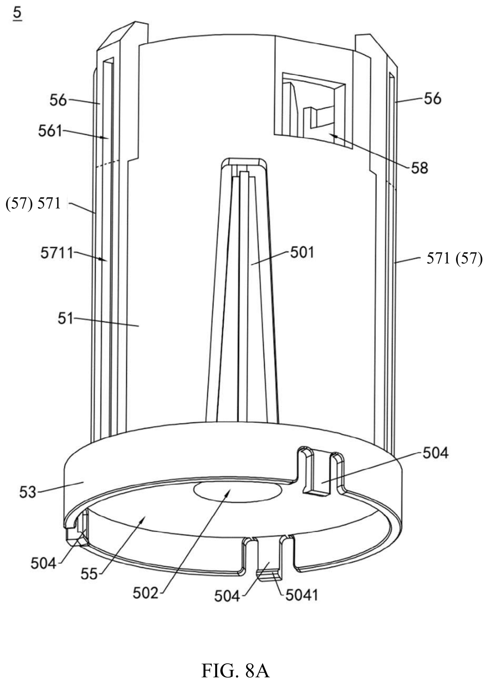

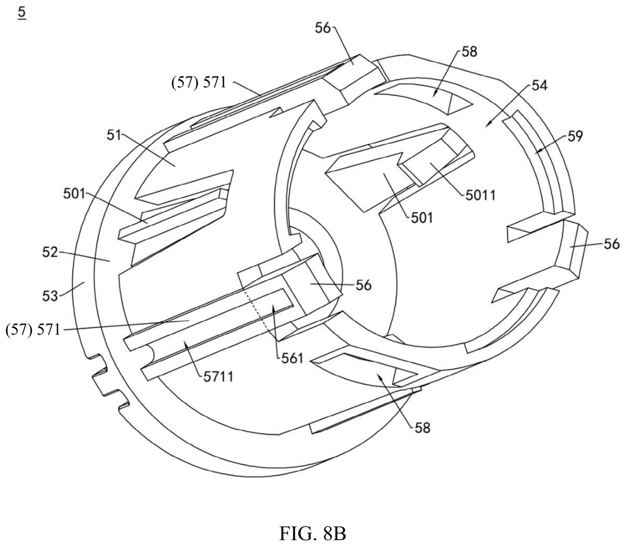

FIG. 8A is a diagram of a structure of the implantation base 5 shown in FIG. 3. FIG. 8B is a diagram of a structure of the implantation base 5 shown in FIG. 3 from another perspective.

In some embodiments, the implantation base 5 may include a side plate 51, a bottom plate 52, and a lower side plate 53, and the bottom plate 52 of the implantation base 5 is connected to the side plate 51 of the implantation base 5 and the lower side plate 53 of the implantation base 5. A circumferential edge of the side plate 51 of the implantation base 5 is fastened to the bottom plate 52 of the implantation base 5, and the side plate 51 of the implantation base 5 surrounds first accommodation space 54. The lower side plate 53 of the implantation base 5 is annularly connected to a circumferential edge of the bottom plate 52 of the implantation base 5, and the lower side plate 53 of the implantation base 5 surrounds second accommodation space 55. The implantation base 5 is generally a cylindrical combination. The implantation base 5 may further include a third elastic arm 56, an end of the third elastic arm 56 is fastened to the side plate 51 of the implantation base 5, and the third elastic arm 56 is located at an end that is of the side plate 51 of the implantation base 5 and that faces away from the bottom plate 52 of the implantation base 5. The third elastic arm 56 is spaced apart from the side plate 51 of the implantation base 5 in the circumferential direction, an end that is of the third elastic arm 56 and that faces away from the bottom plate 52 of the implantation base 5 is provided with an inclined end surface, and the inclined end surface of the third elastic arm 56 is inclined relative to an axial direction of the implantation base 5.

The implantation base 5 may further include a fourth guiding structure 57, and the fourth guiding structure 57 is fastened to an outer side surface of the implantation base 5. For example, the fourth guiding structure 57 may include a third guiding arm 571, and the third guiding arm 571 is fastened to an outer side surface of the side plate 51 of the implantation base 5. The third guiding arm 571 has a sliding groove 5711, and an opening of the sliding groove 5711 of the third guiding arm 571 faces an outer side of the implantation base 5. The sliding groove 3511 of the first guiding arm 351 extends in the axial direction of the implantation base 5, and the sliding groove 5711 of the third guiding arm 571 may be a strip-shaped groove. In some embodiments, the third guiding arm 571 may extend from the bottom plate 52 of the implantation base 5 to the third elastic arm 56, the third elastic arm 56 also has a sliding groove 561, the sliding groove 5711 of the third guiding arm 571 communicates with the sliding groove 561 of the third elastic arm 56, and the third elastic arm 56 is also a part of the fourth guiding structure 57. There may be one or more third elastic arms 56 and one or more third guiding arms 571. When there are a plurality of third elastic arms 56 and a plurality of third guiding arms 571, the plurality of third elastic arms 56 and the plurality of third guiding arms 571 are disposed around a circumferential direction of the implantation base 5.

The implantation base 5 is provided with snap-fitting space 58, and the snap-fitting space 58 is located at the end that is of the side plate 51 of the implantation base 5 and that faces away from the bottom plate 52 of the implantation base 5. For example, the snap-fitting space 58 may be a snap-fitting groove. An opening of the snap-fitting groove faces the outer side of the implantation base 5. In addition, the snap-fitting space 58 may alternatively be a snap-fitting hole that runs through the side plate 51 of the implantation base 5. The snap-fitting space 58 and the third elastic arm 56 are spaced apart. The implantation base 5 may further include a plurality of mounting grooves 59, the plurality of mounting grooves 59 are located at an end that is of the side plate 51 of the implantation base 5 and that faces away from the bottom plate 52 of the implantation base 5, the plurality of mounting grooves 59 are spaced apart, and the plurality of mounting grooves 59 may be formed by recessing the side plate 51 of the implantation base 5 from an inner side to an outer side. For example, there may be three mounting grooves 59. For example, the mounting groove 59 is spaced apart from each of the snap-fitting space 58 and the third elastic arm 56.

The implantation base 5 may further include a fourth elastic arm 501, the fourth elastic arm 501 is spaced apart from the side plate 51 of the implantation base 5 in the circumferential direction, and an end of the fourth elastic arm 501 is fastened to the bottom plate 52 of the implantation base 5 or the side plate 51 of the implantation base 5. The other end of the fourth elastic arm 501 is provided with a snap-fitting hook 5011, and the other end of the fourth elastic arm 501 is an end that is of the first elastic arm 34 and that faces away from the bottom plate 52 of the implantation base 5. The snap-fitting hook 5011 of the fourth elastic arm 501 faces an inner side of the implantation base 5. There may be one or more fourth elastic arms 501. When there are a plurality of fourth elastic arms 501, the plurality of fourth elastic arms 501 are disposed around the circumferential direction of the implantation base 5. The fourth elastic arm 501 may be a T-shaped elastic arm, to help increase a cross-sectional area of the fourth elastic arm 501 in a radial direction of the implantation base 5, and improve rigidity of the fourth elastic arm 501, so that the fourth elastic arm 501 is not easy to break off, and has higher reliability.

The bottom plate 52 of the implantation base 5 has a mounting hole 502. The implantation base 5 may further include a fifth elastic arm 504, and the fifth elastic arm 504 is spaced apart from the lower side plate 53 of the implantation base 5 in the circumferential direction. An end of the fifth elastic arm 504 is fastened to the bottom plate 52 of the implantation base 5 or the lower side plate 53 of the implantation base 5, the other end of the fifth elastic arm 504 is provided with a snap-fitting hook 5041, and the other end of the fifth elastic arm 504 is an end that is of the fifth elastic arm 504 and that faces away from the bottom plate 52 of the implantation base 5. There may be one or more fifth elastic arms 504. When there are a plurality of fifth elastic arms 504, the plurality of fifth elastic arms 504 are spaced apart around the circumferential direction of the implantation base 5. For example, there may be three fifth elastic arms 504.

In some embodiments, the implantation base 5 may be an integral mechanical part. The implantation base 5 is made of engineering plastic such as polycarbonate or a polycarbonate and acrylonitrile butadiene styrene mixture. In this embodiment, the polycarbonate and acrylonitrile butadiene styrene plastic have features such as heat resistance, impact resistance, good mechanical performance, high hardness, and good processing performance. The implantation base 5 is made of the engineering plastic such as the polycarbonate or the polycarbonate and acrylonitrile butadiene styrene mixture, to help reduce manufacturing costs of the implantation base 5, and ensure mechanical strength of the implantation base 5.

In some other embodiments, the implantation base 5 may alternatively be made of another material, for example, engineering plastic such as polyvinyl chloride resin or polystyrene resin, or metal such as stainless steel or aluminum alloy. This is not strictly limited in this application.

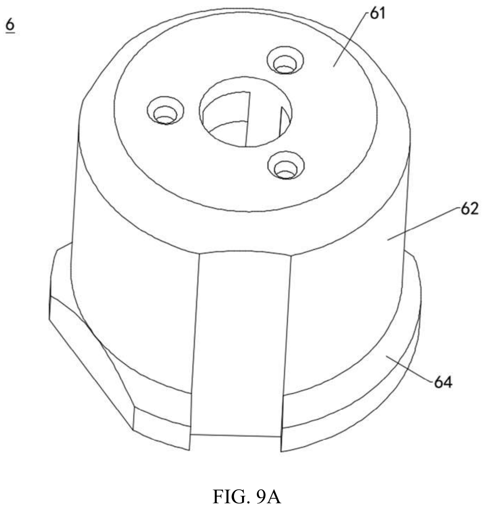

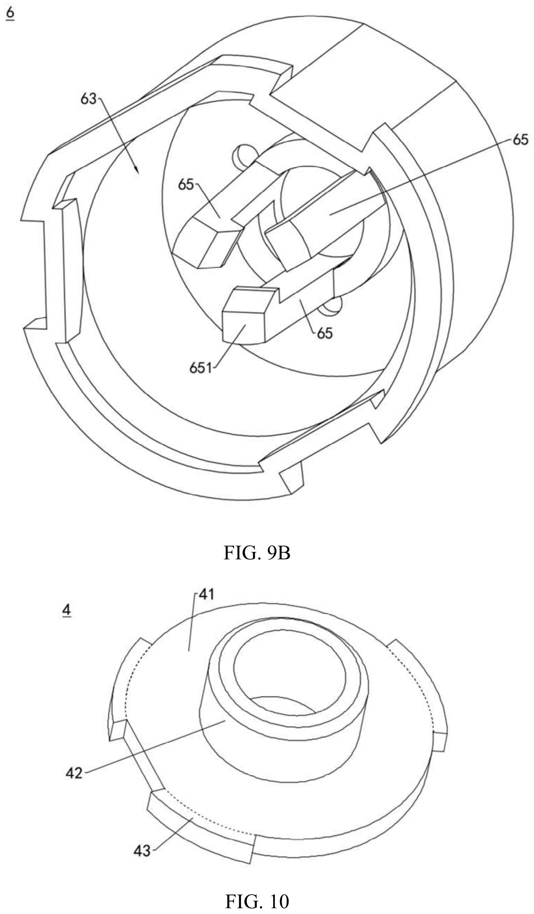

FIG. 9A is a diagram of a structure of the needle extraction base 6 shown in FIG. 3. FIG. 9B is a diagram of a structure of the needle extraction base 6 shown in FIG. 9A from another perspective. In some embodiments, the needle extraction base 6 may include a top plate 61 and a side plate 62, and the side plate 62 of the needle extraction base 6 is disposed around the top plate 61 of the needle extraction base 6. The needle extraction base 6 is generally cylindrical, and the side plate 62 of the needle extraction base 6 surrounds third accommodation space 63. The needle extraction base 6 may further include a limiting flange 64, the limiting flange 64 is located at an end that is of the side plate 62 of the needle extraction base 6 and that faces away from the top plate 61 of the needle extraction base 6, and the limiting flange 64 is disposed around an outer circumferential side of the side plate 62 of the needle extraction base 6. The needle extraction base 6 may further include a first cantilever 65, an end of the first cantilever 65 is fastened to a side that is of the top plate 61 of the needle extraction base 6 and that faces the third accommodation space 63, the other end of the first cantilever 65 is provided with a snap-fitting hook 651, the other end of the first cantilever 65 is an end that is of the first cantilever 65 and that faces away from the top plate 61 of the needle extraction base 6, and the snap-fitting hook 651 of the first cantilever 65 faces the top plate 61 of the needle extraction base 6. For example, there may be one or more first cantilevers 65. When there are a plurality of first cantilevers 65, the plurality of first cantilevers 65 are spaced apart around an axis of the needle extraction base 6. For example, there may be three first cantilevers 65.

In some embodiments, the needle extraction base 6 may be an integral mechanical part. The needle extraction base 6 is made of engineering plastic such as polycarbonate or a polycarbonate and acrylonitrile butadiene styrene mixture. In this embodiment, the polycarbonate and acrylonitrile butadiene styrene plastic have features such as heat resistance, impact resistance, good mechanical performance, high hardness, and good processing performance. The needle extraction base 6 is made of the engineering plastic such as the polycarbonate or the polycarbonate and acrylonitrile butadiene styrene mixture, to help reduce manufacturing costs of the needle extraction base 6, and ensure mechanical strength of the needle extraction base 6.

In some other embodiments, the needle extraction base 6 may alternatively be made of another material, for example, engineering plastic such as polyvinyl chloride resin or polystyrene resin, or metal such as stainless steel or aluminum alloy. This is not strictly limited in this application.

FIG. 10 is a diagram of a structure of the stopper member 4 shown in FIG. 3.

In some embodiments, the stopper member 4 may include an abutting portion 41, a third limiting boss 42, and a plurality of stopping blocks 43. The abutting portion 41 is generally in a disc shape, the third limiting boss 42 is fastened to a surface of a side of the abutting portion 41, the plurality of stopping blocks 43 are fastened to a circumferential edge of the abutting portion 41, and the plurality of stopping blocks 43 are spaced apart. For example, there may be three stopping blocks 43. The stopper member 4 may be made of engineering plastic such as polycarbonate or a polycarbonate and acrylonitrile butadiene styrene mixture.

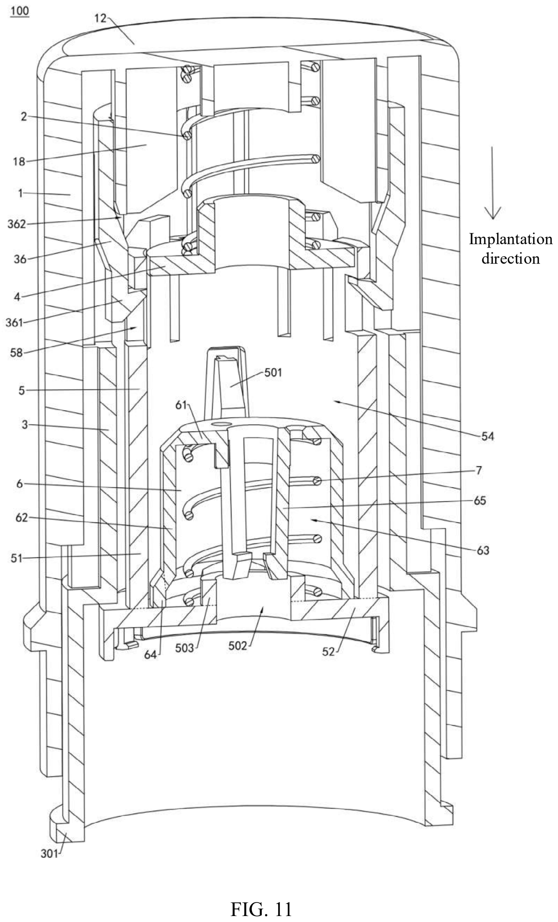

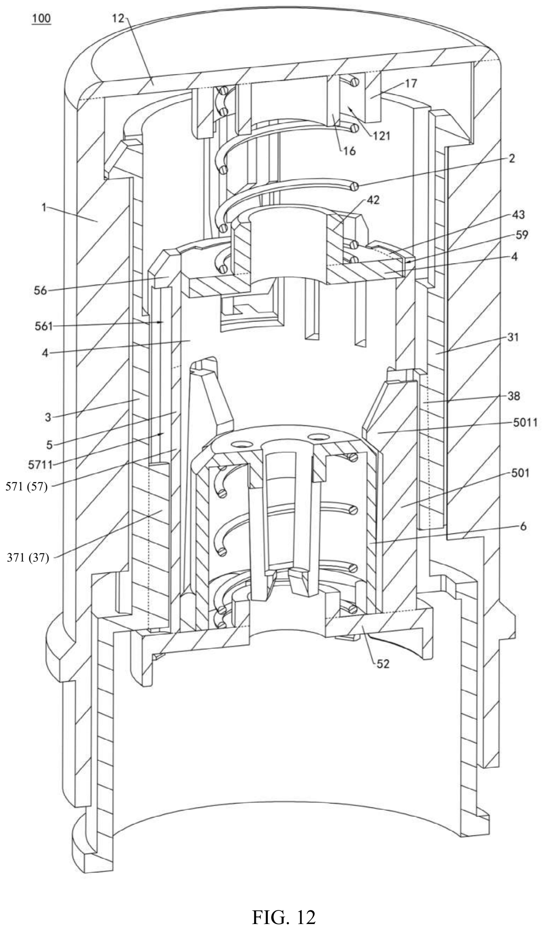

FIG. 11 is a diagram 2 of a partial cross-sectional structure after the physiological sign detection apparatus 100 shown in FIG. 2 is cut along A-A. FIG. 12 is a diagram 2 of a partial cross-sectional structure after the physiological sign detection apparatus 100 shown in FIG. 2 is cut along B-B.

In some embodiments, the implantation base 5 is mounted on an inner side of the base 3, and the axial direction of the implantation base 5 coincides with the axial direction of the base 3. The implantation base 5 may further include a fourth limiting boss 503, and the fourth limiting boss 503 is disposed around a circumferential edge of the mounting hole 502, and is fastened to a side that is of the bottom plate 52 of the implantation base 5 and that faces the first accommodation space 54. Both the needle extraction base 6 and the second elastic member 7 are mounted on the inner side of the implantation base 5.

The second elastic member 7 is located between the implantation base 5 and the needle extraction base 6. Both an axial direction of the needle extraction base 6 and an axial direction of the second elastic member 7 may coincide with the axial direction of the implantation base 5. The second elastic member 7 is in a compressed state. For example, the needle extraction base 6 is mounted in the first accommodation space 54 of the implantation base 5, and the side plate 62 of the needle extraction base 6 is located on an outer side of the fourth limiting boss 503. The second elastic member 7 is mounted in the third accommodation space 63 of the needle extraction base 6. The second elastic member 7 may be but is not limited to a spring, an elastic rubber, or the like. The second elastic member 7 is located between the bottom plate 52 of the implantation base 5 and the top plate 61 of the needle extraction base 6, and is sleeved on a periphery of the first cantilever 65 and a periphery of the fourth limiting boss 503. The second elastic member 7 is configured to push the needle extraction base 6 away from the bottom plate 52 of the implantation base 5. The first cantilever 65 and the fourth limiting boss 503 limit and position the second elastic member 7, to prevent the second elastic member 7 from being skewed in a compression and rebounding process, thereby stabilize a position of the second elastic member 7, to improve reliability of the physiological sign detection apparatus 100.

In some embodiments, the needle extraction base 6 is mounted between the bottom plate 52 of the implantation base 5 and the snap-fitting hook 5011 of the fourth elastic arm 501. There may be three fourth elastic arms 501, so that the snap-fitted needle extraction base 6 is subject to a balanced force. In this embodiment, the needle extraction base 6 is located between the bottom plate 52 of the implantation base 5 and the snap-fitting hook 5011 of the fourth elastic arm 501, so that a stable position relationship is maintained between the needle extraction base 6 and the implantation base 5, and the fourth elastic arm 501 may push the needle extraction base 6 to move with the implantation base 5.

The fourth elastic arm 501 abuts against the baffle plate 38 on the inner side of the base 3, or a gap between the fourth elastic arm 501 and the baffle plate 38 is small. In this embodiment, when the physiological sign detection apparatus 100 is in an initial state, the snap-fitting hook 5011 of the fourth elastic arm 501 needs to well limit the needle extraction base 6, to prevent the needle extraction base 6 from moving toward a side that is away from the bottom plate 52 of the implantation base 5. The second elastic member 7 has a driving force for the needle extraction base 6, and the end that is of the fourth elastic arm 501 and that is provided with the snap-fitting hook is a free end. When driven by the needle extraction base 6, the fourth elastic arm 501 has a trend of moving toward a side of the upper side wall 31 of the base 3. The baffle plate 38 may block the fourth elastic arm 501, to avoid a failure of the physiological sign detection apparatus 100 because the needle extraction base 6 is detached from the implantation base 5 when the physiological sign detection apparatus 100 is in the initial state. This helps improve reliability of the physiological sign detection apparatus 100.

In some embodiments, the side plate 62 of the needle extraction base 6 is located between the fourth limiting boss 503 and the side plate 51 of the implantation base 5, the limiting flange 64 of the needle extraction base 6 is close to the side plate 51 of the implantation base 5, and a spacing between the limiting flange 64 and the side plate 51 of the implantation base 5 is small. In this embodiment, the limiting flange 64 is disposed on the side plate 62 of the needle extraction base 6, so that a gap between the side plate 62 of the needle extraction base 6 and the side plate 51 of the implantation base 5 is smaller, to help control of assembly precision between the needle extraction base 6 and the implantation base 5, reduce misalignment, and improve reliability and implantation accuracy of the physiological sign detection apparatus 100. In addition, the limiting flange 64 may further prevent the needle extraction base 6 from shaking greatly relative to the implantation base 5 in a packaging and transportation process of the physiological sign detection apparatus 100, so that the stable position relationship is maintained between the needle extraction base 6 and the implantation base 5.

In some embodiments, the first elastic member 2 is connected between the top wall 12 of the housing 1 and the implantation base 5, and an axial direction of the first elastic member 2 may coincide with the axial direction of the housing 1. The stopper member 4 is fastened to an end that is of the implantation base 5 and that faces away from the needle extraction base 6. For example, the stopping block 43 of the stopper member 4 is mounted in the mounting groove 59 of the implantation base 5. The plurality of stopping blocks 43 of the stopper member 4 are disposed in a one-to-one correspondence with the plurality of mounting grooves 59 of the implantation base 5, and the implantation base 5 can stably support the stopper member 4. The first elastic member 2 is mounted between the stopper member 4 and the top wall 12 of the housing 1. The first elastic member 2 may be but is not limited to a spring, an elastic rubber, or the like. An end of the first elastic member 2 is located in the limiting groove 121 of the top wall 12 of the housing 1, and the other end of the first elastic member 2 is sleeved on a periphery of the third limiting boss 42 of the stopper member 4. The first limiting boss 16 and the second limiting boss 17 of the housing 1 and the third limiting boss 42 of the stopper member 4 all limit and position the first elastic member 2, to prevent the first elastic member 2 from being skewed in a compression and rebounding process, thereby stabilizing a position of the first elastic member 2, to improve reliability of the physiological sign detection apparatus 100. The first elastic member 2 may be in a compressed state, to form pre-pressure on the stopper member 4, so that a position of the stopper member 4 relative to the base 3 is stable. Alternatively, the first elastic member 2 may be in a natural state. This is not strictly limited in this embodiment of this application.

Refer to FIG. 8B, FIG. 11, and FIG. 12 together. In some embodiments, the implantation base 5 is slidably connected to the base 3. The third guiding structure 37 of the base 3 is slidably connected to the fourth guiding structure 57 of the implantation base 5. For example, the third sliding block 371 of the base 3 is mounted in the sliding groove 561 of the third elastic arm 56 and the sliding groove 5711 of the third guiding arm 571 of the implantation base 5, and the third sliding block 371 can slide in the sliding groove 561 of the third elastic arm 56 and the sliding groove 5711 of the third guiding arm 571. There may be three third elastic arms 56 and three third guiding arms 571, to ensure that a connection between the implantation base 5 and the base 3 is subject to a balanced force, and maintain the implantation base 5 and the base 3 relatively stable in the radial direction. In some other embodiments, the third guiding structure 37 includes a sliding groove, the fourth guiding structure 57 includes a sliding block, and the third guiding structure 37 and the fourth guiding structure 57 are still slidably connected to each other.

In this embodiment, the third guiding structure 37 of the base 3 is slidably connected to the fourth guiding structure 57 of the implantation base 5 for mutual cooperation, so that the base 3 and the implantation base 5 limit each other in the circumferential direction, and the implantation base 5 is prevented from rotating relative to the base 3 in the circumferential direction. The third sliding block 371 can slide in the sliding groove 561 of the third elastic arm 56 and the third guiding arm 571, so that the implantation base 5 can move in the axial direction relative to the base 3, and the third elastic arm 56 may further limit movement of the implantation base 5 in the axial direction. The third elastic arm 56 is spaced apart from the side plate 51 of the implantation base 5 in the circumferential direction, and the other end that is of the third elastic arm 56 and that faces away from the bottom plate 52 of the implantation base 5 is provided with the inclined end surface. When the implantation base 5 is assembled with the base 3, the third elastic arm 56 may elastically avoid the third sliding block 371 in the radial direction, and the inclined end surface of the third elastic arm 56 avoids the third sliding block 371 in the axial direction, so that the implantation base 5 is smoothly mounted on the inner side of the base 3, to reduce snap-fitting resistance, and further avoid damaging the implantation base 5 and the base 3 during assembly.

In some embodiments, at least a part of the snap-fitting hook 361 of the second elastic arm 36 is located in the snap-fitting space 58 of the implantation base 5, so that the second elastic arm 36 is snap-fitted into the implantation base 5, and the base 3 limits the implantation base 5. At least a part of the toggle 18 of the housing 1 is located in the guiding groove 362 of the second elastic arm 36. When the housing 1 moves in the implantation direction, the toggle 18 moves along the guiding groove 362, and the toggle 18 pushes the second elastic arm 36, so that the second elastic arm 36 moves toward a side away from the implantation base 5. The snap-fitting hook 361 of the second elastic arm 36 is detached from the snap-fitting space 58, the implantation base 5 is free from a limit of the base 3, and the implantation base 5 can move in the implantation direction under an elastic force of the first elastic member 2. The implantation direction is a direction in which the top wall 12 of the housing 1 points to the abutting end 301 of the base 3. The housing 1 moves in the implantation direction. To be specific, the housing 1 moves toward the abutting end 301 relative to the base 3.

In this embodiment, a limit of the base 3 on the implantation base 5 is removed through cooperation between the toggle 18 and the second elastic arm 36, so that the implantation base 5 is detached from the base 3 under an elastic force of the first elastic member 2. When the housing 1 moves in the implantation direction, the toggle 18 gradually pushes a second cantilever 19 to move toward an outer side of the side plate 51 of the implantation base 5, and compresses the first elastic member 2. When the snap-fitting hook 361 of the second elastic arm 36 is detached from the snap-fitting space 58, the implantation base 5 moves in the implantation direction under the elastic force of the first elastic member 2. A limiting mechanism and a limit release mechanism of the implantation base 5 of the physiological sign detection apparatus 100 are simple and are arranged compactly, and a transmission speed between the housing 1 and the base 3 is fast.

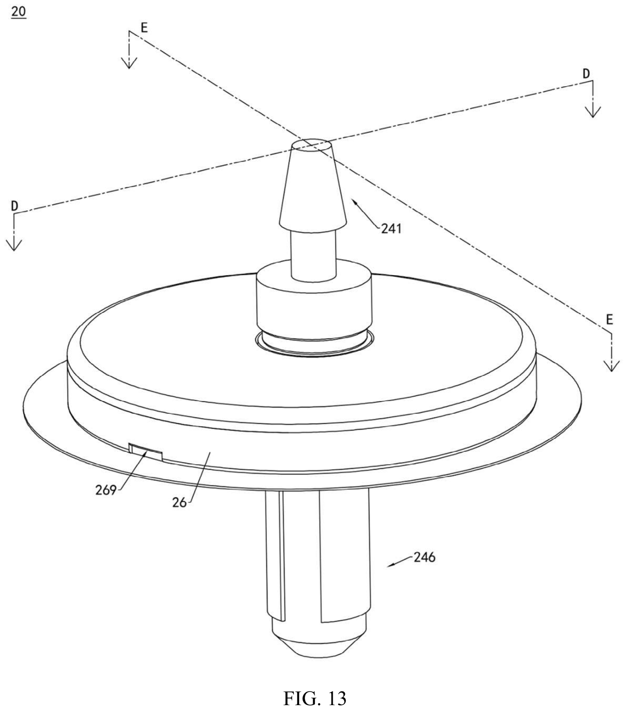

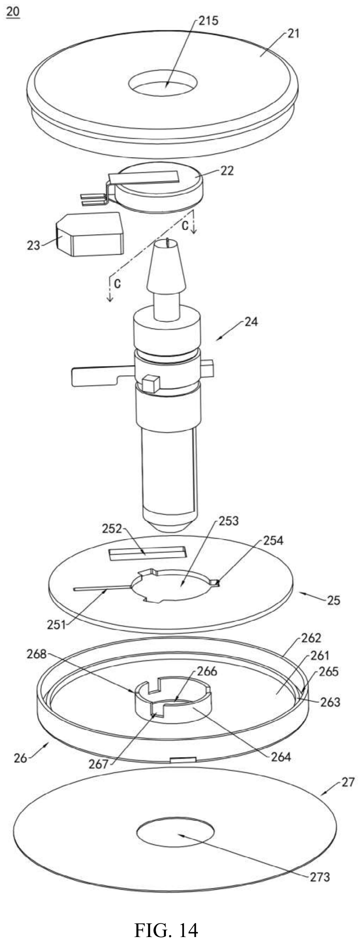

FIG. 13 is a diagram of a structure of the sensor assembly 20 shown in FIG. 2 in some embodiments. FIG. 14 is a diagram of a partial exploded structure of the sensor assembly 20 shown in FIG. 13.

In some embodiments, the sensor assembly 20 may include an upper cover 21, a battery 22, a connector 23, a sensor sealing assembly 24, a circuit board 25 (Printed Circuit Board, PCB), a lower cover 26, and a bonding member 27. Both the upper cover 21 and the lower cover 26 may be made of engineering plastic such as polycarbonate or acrylonitrile butadiene styrene. Electronic elements such as a chip, a capacitor, a resistor, a near-range wireless communication (Near Field Communication, NFC) component, or a Bluetooth component may be disposed on the circuit board 25.

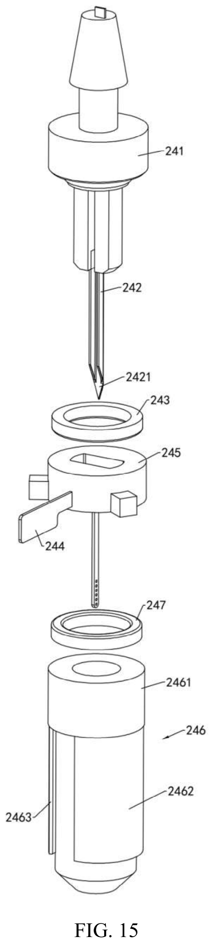

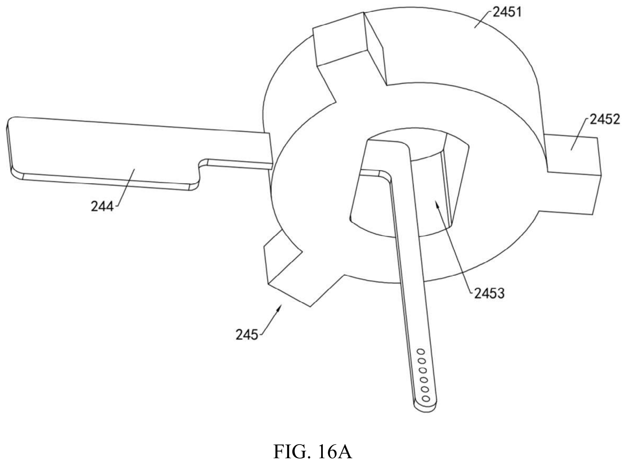

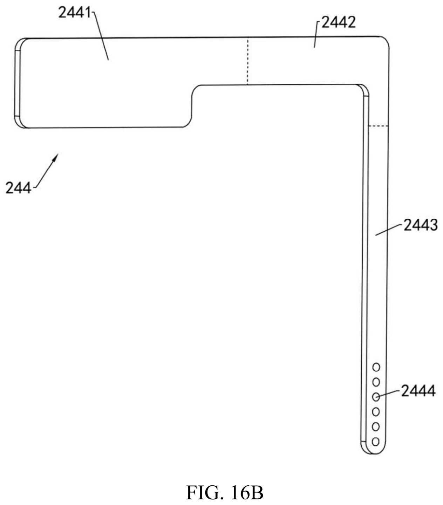

FIG. 15 is a diagram of a partial exploded structure of the sensor sealing assembly 24 shown in FIG. 14. FIG. 16A is a diagram of an assembly structure of a sensor 244 and a sensor base 245 that are shown in FIG. 15 in some embodiments. FIG. 16B is a diagram of a structure of the sensor 244 shown in FIG. 16A.

In some embodiments, the sensor sealing assembly 24 may include a needle base 241, a puncture needle 242, a first sealing member 243, a sensor 244, a sensor base 245, a second sealing member 247, and a sealing bottle 246. The sensor 244 is fastened to the sensor base 245. The sensor 244 may include a connecting portion 2441, a fastening portion 2442, and an implantation portion 2443. The fastening portion 2442 is connected to the implantation portion 2443 and the connecting portion 2441. The connecting portion 2441 of the sensor 244 is configured to be electrically connected to the connector 23 (as shown in FIG. 14), the sensor base 245 is fastened to the fastening portion 2442 of the sensor 244, and the implantation portion 2443 of the sensor 244 is configured to be punctured into the organism 30.

The sensor 244 may include a substrate, an electrode layer (not shown in FIG. 16B), and a sensing layer 2444. For example, a material of the substrate is polyethylene terephthalate (Polyethylene terephthalate, PET). The electrode layer is fastened to a surface of the substrate. The electrode layer may be an electrode system (for example, two electrodes or a plurality of electrodes) formed on the surface of the substrate by using a process such as ink-jet printing or silk-screen printing. The electrode layer may include a first part located in the implantation portion 2443, a second part located in the fastening portion 2442, and a third part located in the connecting portion 2441. The second part of the electrode layer is connected to the first part of the electrode layer and the third part of the electrode layer. The sensing layer 2444 is fastened to a partial region in the first part of the electrode layer. In other words, the sensing layer 2444 is located in the implantation portion 2443. The sensing layer 2444 may be formed by applying a sensing reagent to a surface of the first part of the electrode layer through ink-jet printing or drop-casting. The sensing reagent may be a mixed solution including a bioproteinase (for example, glucose oxidase), an electron transfer medium, and a cross-linking agent. After printing of the sensor 244 is completed, a shape may be formed through femtosecond or picosecond laser cutting.

In some embodiments, the sensor 244 and the sensor base 245 are integral mechanical parts. For example, the sensor 244 and the sensor base 245 are integrally formed by using an in-mold injection molding process. A region that is of the sensor 244 and that is in contact with the sensor base 245 may be covered with a heat insulation layer. For example, a surface of the fastening portion 2442 of the sensor 244 may be covered with a heat insulation layer. The heat insulation layer may be formed by using a process such as an ink-jet printing process or a silk-screen printing process.

In this embodiment, the sensor 244 and the sensor base 245 are integrally formed by using the in-mold injection molding process, and the region that is of the sensor 244 and that is in contact with the sensor base 245 is covered with the heat insulation layer, to reduce heat conduction in a process of performing in-mold injection molding on the sensor 244 and the sensor base 245, and prevent heat from being transferred from the fastening portion 2442 of the sensor 244 to the implantation portion 2443, so as to prevent an enzyme at the sensing layer 2444 of the implantation portion 2443 from being deactivated due to a high temperature, thereby helping improve detection accuracy of the physiological sign detection apparatus 100.

In some embodiments, the sensor base 245 may include a body 2451, a blocking block 2452, and a mounting through hole 2453. The sensor 244 may be fastened to the body 2451 of the sensor base 245. The blocking block 2452 is fastened to an outer circumferential edge of the body 2451. There may be one or more blocking blocks 2452, and a plurality of blocking blocks 2452 are spaced apart around a circumferential direction of the body 2451. For example, there may be three blocking blocks 2452. The mounting through hole 2453 passes through the body 2451, and the mounting through hole 2453 may be a strip hole with radians at two ends. The sensor base 245 may be an integral mechanical part. The sensor base 245 may be made of engineering plastic such as polycarbonate or acrylonitrile butadiene styrene.

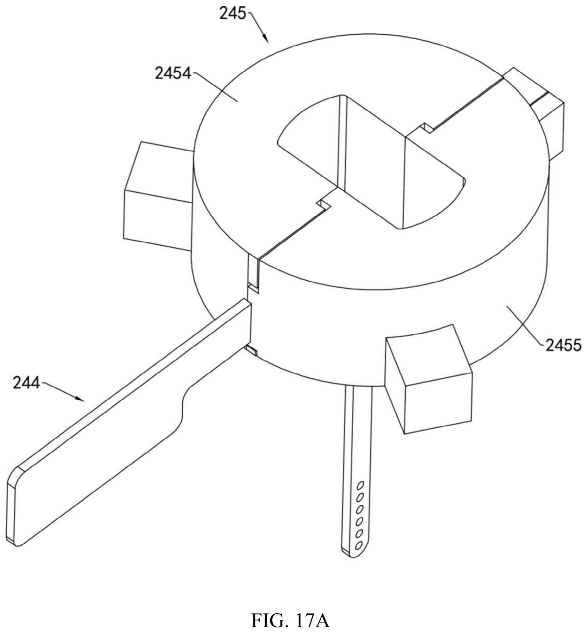

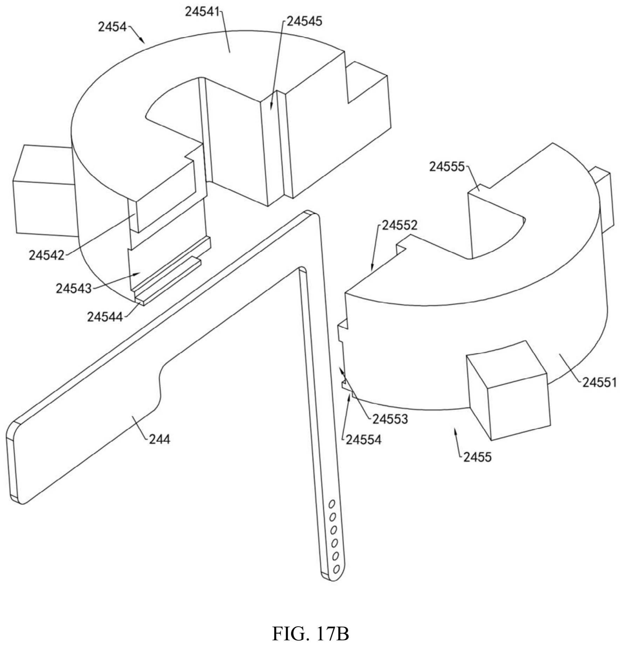

FIG. 17A is a diagram of an assembly structure of the sensor 244 and the sensor base 245 that are shown in FIG. 15 in some other embodiments. FIG. 17B is a diagram of an exploded structure of the structure shown in 17A. This embodiment may include most of the technical solutions in the foregoing embodiments. The following mainly describes differences between this embodiment and the foregoing embodiments, and most same content of this embodiment and the foregoing embodiments is not described again.

In some embodiments, the sensor base 245 may include a first part 2454 and a second part 2455. The first part 2454 is snap-fitted into the second part 2455, and the fastening portion 2442 of the sensor 244 is mounted in a limiting through hole between the first part 2454 and the second part 2455. For example, the first part 2454, the sensor 244, and the second part 2455 may be stuck by using a gel dispensing process, to form an integral mechanical part. The first part 2454 may include a first body 24541, a first bump 24542, a first groove 24543, a second bump 24544, and a second groove 24545. The first bump 24542, the first groove 24543, the second groove 24545, and the second bump 24544 are located on a side that is of the first body 24541 and that faces the second part 2455. The first bump 24542 and the second bump 24544 are fastened to the first body 24541. The first groove 24543 and the second groove 24545 may be formed by recessing the first body 24541 in a direction facing away from the second part 2455. The second part 2455 may include a second body 24551, a third groove 24552, a fourth groove 24553, a fifth groove 24554, and a third bump 24555. The third groove 24552, the fourth groove 24553, the fifth groove 24554, and the third bump 24555 are all located on a side that is of the second body 24551 and that faces the first part 2454. The third bump 24555 is fastened to the second body 24551. The third groove 24552, the fourth groove 24553, and the fifth groove 24554 may be formed by recessing the second body 24551 in a direction facing away from the first part 2454. The first bump 24542 is mounted in the third groove 24552, the second bump 24544 is mounted in the fifth groove 24554, and the third bump 24555 is mounted in the second groove 24545, so that the first part 2454 is snap-fitted into the second part 2455. The first groove 24543 and the fourth groove 24553 are disposed opposite to each other and communicate with each other, to jointly form a limiting through hole of the sensor 244.

In this embodiment, the sensor 244 is stuck between the first part 2454 and the second part 2455 of the sensor base 245, and a temperature of an assembly environment is moderate, to prevent an enzyme at the sensing layer 2444 of the sensor 244 from being deactivated due to temperature impact. No heat insulation layer needs to be disposed in the region that is of the sensor 244 and that is in contact with the sensor base 245, and assembly between the sensor 244 and the sensor base 245 is simpler, to help reduce production costs of the physiological sign detection apparatus 100.

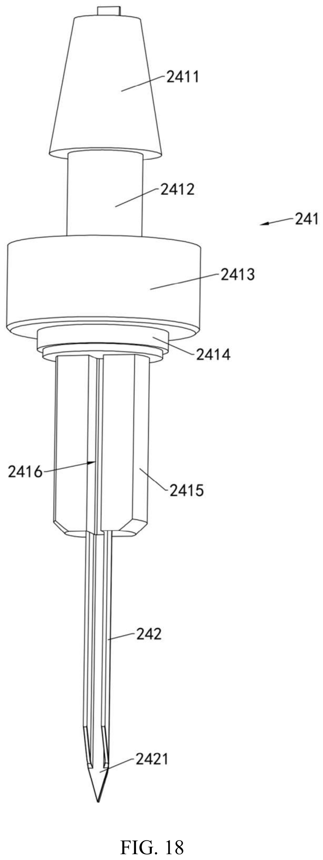

FIG. 18 is a diagram of an assembly structure of the puncture needle 242 and the needle base 241 that are shown in FIG. 15.

In some embodiments, the needle base 241 may include a limiting snap-fit 2411, a snap-fitting portion 2412, a limiting portion 2413, a sealing portion 2414, and a guiding portion 2415. The limiting snap-fit 2411, the snap-fitting portion 2412, the limiting portion 2413, the sealing portion 2414, and the guiding portion 2415 are sequentially connected. The limiting snap-fit 2411 may be generally in a shape of a circular truncated cone, the snap-fitting portion 2412 and the limiting portion 2413 may be generally in a shape of a cylinder, and a diameter of the snap-fitting portion 2412 is less than a diameter of a bottom surface of the limiting snap-fit 2411 and a diameter of the limiting portion 2413. The guiding portion 2415 has a recessed groove 2416, and the recessed groove 2416 extends in an axial direction of the needle base 241.

The puncture needle 242 is fastened to the needle base 241, and extends in the axial direction of the needle base 241. A puncture end 2421 of the puncture needle 242 protrudes from the guiding portion 2415 of the needle base 241. A part that is of the puncture needle 242 and that is fastened to the guiding portion 2415 wraps the recessed groove 2416, and a shape of the recessed groove 2416 matches a shape of the puncture needle 242. The puncture needle 242 and the needle base 241 may be integrally formed. For example, the needle base 241 and the puncture needle 242 may be integrally formed by using the in-mold injection molding process. The needle base 241 may be made of engineering plastic such as polycarbonate or acrylonitrile butadiene styrene. The puncture needle 242 may be made of a metal material such as 316 stainless steel, 304 stainless steel, or medical-grade stainless steel (for example, SUS316L).

A cross section of the puncture needle 242 may be generally of a U-shaped structure, and the puncture needle 242 is configured to puncture and push away tissue of the organism 30. The puncture end 2421 of the puncture needle 242 may be configured to wrap the implantation portion 2443 of the sensor 244. When the puncture needle 242 is punctured into the organism 30, implantation space may be formed, so that the implantation portion 2443 of the sensor 244 is punctured into the organism 30. In addition, the puncture end 2421 of the puncture needle 242 may further partially wrap the implantation portion 2443 of the sensor 244, and the implantation portion 2443 of the sensor 244 may also be punctured into the organism 30 with the puncture needle 242. This is not strictly limited in this application.

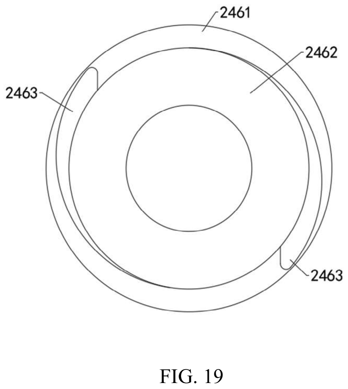

Refer to FIG. 15 and FIG. 19 together. FIG. 19 is a diagram of a structure of the sealing bottle 246 shown in FIG. 15 from another perspective.

In some embodiments, the sealing bottle 246 may include an abutting portion 2461, an accommodation portion 2462, and a ratchet 2463, the accommodation portion 2462 is connected to the abutting portion 2461, and the ratchet 2463 is convexly disposed on an outer side surface of the sealing bottle 246. There may be one or more ratchets 2463, and a plurality of ratchets 2463 are spaced apart around an axis of the sealing bottle 246. For example, the ratchet 2463 is fastened to an outer side surface of the accommodation portion 2462, and there is a smooth transition between an outer side surface of the ratchet 2463 and the outer side surface of the accommodation portion 2462. There may be two ratchets 2463, and the two ratchets 2463 are disposed opposite to each other.

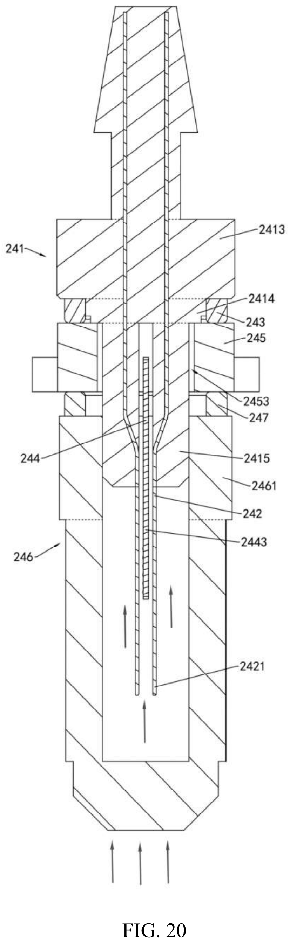

FIG. 20 is a diagram of a cross-sectional structure after the sensor sealing assembly 24 shown in FIG. 14 is cut along C-C.

In some embodiments, the sensor base 245 is located between the needle base 241 and the sealing bottle 246, an end of the needle base 241 passes through the sensor base 245, the sealing bottle 246 is detachably fastened to the needle base 241 and is hermetically connected to the sensor base 245, and the needle base 241 is hermetically connected to the sensor base 245. The guiding portion 2415 of the needle base 241 passes through the sensor base 245. For example, the guiding portion 2415 is inserted into the mounting through hole 2453 of the sensor base 245. The guiding portion 2415 is detachably fastened to the sealing bottle 246. For example, the needle base 241 may be threadedly connected to the sealing bottle 246, an outer side surface of the guiding portion 2415 is provided with an external thread, an inner side surface of the abutting portion 2461 is provided with an internal thread (not shown in FIG. 20), and the guiding portion 2415 is threadedly connected to the abutting portion 2461. The first sealing member 243 is sleeved on an outer side of the sealing portion 2414 of the needle base 241, and is located between the limiting portion 2413 and the sensor base 245. The first sealing member 243 is hermetically connected to the sealing portion 2414 of the needle base 241 and the sensor base 245. The second sealing member 247 is located between the sealing bottle 246 and the sensor base 245, and is hermetically connected to the sealing bottle 246 and the sensor base 245. In this case, the puncture needle 242 wraps the implantation portion 2443 of the sensor 244. The sealing bottle 246 has a hollow cavity, and the implantation portion 2443 of the sensor 244 and the puncture end 2421 of the puncture needle 242 are located in the hollow cavity of the sealing bottle 246.

In an existing sensor assembly, a sensor is exposed and is directly assembled with another component of the sensor assembly. In an assembly process, the sensor is easily touched, to damage a sensing material of an implantation portion of the sensor. Consequently, detection performance of the sensor is reduced, and detection accuracy is reduced. In this embodiment, the needle base 241 is threadedly connected to the sealing bottle 246, to securely connect the needle base 241, the first sealing member 243, the sensor base 245, the second sealing member 247, and the sealing bottle 246, so that the first sealing member 243 is hermetically connected to the needle base 241 and the sensor base 245, the second sealing member 247 is hermetically connected to the sensor base 245 and the sealing bottle 246, and the implantation portion 2443 of the sensor 244 is located in sealed space formed by using the sensor sealing assembly 24. In addition, the implantation portion 2443 of the sensor 244 is located in the hollow cavity of the sealing bottle 246, and the sealing bottle 246 may isolate and protect the implantation portion 2443 of the sensor 244, to avoid damage to the sensing layer 2444 of the implantation portion 2443 in a subsequent assembly process of the sensor 244 and another component, thereby helping improve reliability and detection accuracy of the physiological sign detection apparatus 100, and ensuring batch consistency of the sensor 244 in a production process.

The first sealing member 243 may be made of an elastic polymer material such as nitrile butadiene rubber, silicon rubber, or fluorine rubber. In some embodiments, the needle base 241 and the first sealing member 243 may be integrally formed by using a process such as a vulcanization process or a dual-shot injection molding process, so that the first sealing member 243 is formed on the sealing portion 2414 of the needle base 241. The needle base 241 and the first sealing member 243 are integrally formed, so that the first sealing member 243 and the needle base 241 are connected more stably, to help improve sealing performance between the needle base 241 and the first sealing member 243. In addition, when the needle base 241 is detached from the sensor base 245, the needle base 241 may be detached from the sensor base 245 together with the first sealing member 243. In some other embodiments, the first sealing member 243 may alternatively be fastened to the needle base 241 through bonding, or the like.

The second sealing member 247 may be made of an elastic polymer material such as nitrile butadiene rubber, silicon rubber, or fluorine rubber. In some embodiments, the second sealing member 247 and the sealing bottle 246 may be integrally formed by using a process such as a vulcanization process or a dual-shot injection molding process, so that the second sealing member 247 is fastened to a side that is of the sealing bottle 246 and that is close to the sensor base 245. The sealing bottle 246 and the second sealing member 247 are integrally formed, so that the second sealing member 247 and the sealing bottle 246 are connected more stably, to help improve sealing performance between the sealing bottle 246 and the second sealing member 247. In addition, when the sealing bottle 246 is detached from the needle base 241, the second sealing member 247 may be detached from the sensor base 245 with the sealing bottle 246. In some other embodiments, the second sealing member 247 may alternatively be fastened to the sealing bottle 246 through bonding, or the like.

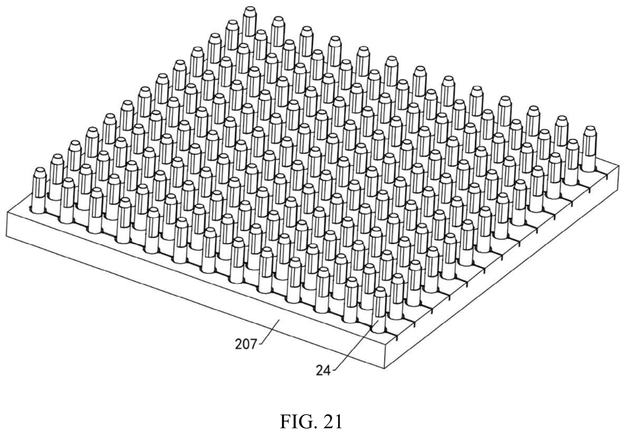

Refer to FIG. 20 and FIG. 21 together. FIG. 21 is a diagram of a structure of a sterilization tooling structure of the sensor sealing assembly 24 shown in FIG. 14.

This application provides a sterilization solution, to perform irradiation sterilization on puncture ends 2421 of puncture needles 242 and sensors 244 in batch sensor sealing assemblies 24 through ionizing radiation. In some embodiments, at least a part of the sealing bottle 246 allows ionizing radiation to pass through. For example, a part or all of regions of the sealing bottle 246 may allow ionizing radiation to pass through. The sealing bottle 246 may be made of engineering plastic such as polymethyl methacrylate (Polymethyl methacrylate, PMMA) and polycarbonate, and the engineering plastic such as methyl methacrylate and polycarbonate can both allow ionizing radiation to pass through.

Ionizing radiation may be one or a combination of electron beam radiation, gamma-ray radiation, and X-ray radiation. Ionizing radiation (as shown by an arrow in FIG. 20) passes through the sealing bottle 246, to sterilize the puncture end 2421 of the puncture needle 242 and the implantation portion 2443 of the sensor 244. When irradiation sterilization is performed on the sensor sealing assembly 24, the sensor sealing assembly 24 is placed in a positioning structure of a tray 207, the positioning structure of the tray 207 may be arranged in an array, a plurality of sensor sealing assemblies 24 may be placed on one tray 207, and the tray 207 is placed in a sterilization room for irradiation sterilization.

In the conventional technology, when radiation sterilization is performed on an assembled sensor assembly or physiological sign detection apparatus, a dedicated sterilization apparatus needs to be customized to implement fixed-point sterilization on a puncture end of a puncture needle and an implantation portion of a sensor. Fixed-point sterilization has a high requirement on a radiation device, and is difficult to be implemented in batched. In another manner in the conventional technology, when irradiation sterilization is performed on the entire sensor assembly or the entire physiological sign detection apparatus, a specific tooling needs to be additionally designed, and the sensor assembly or the physiological sign detection apparatus is assembled with the specific tooling, so that the specific tooling performs shielding protection on an internal circuit of the sensor assembly, and then irradiation sterilization can be performed. Consequently, sterilization operations are complex and efficiency is low.

In this embodiment, at least the partial region of the sealing bottle 246 allows ionizing radiation to pass through, so that the sensor sealing assembly 24 can sterilize the puncture end 2421 of the puncture needle 242 and the implantation portion 2443 of the sensor 244 through irradiation. In addition, after sterilization, the puncture end 2421 of the puncture needle 242 and the implantation portion 2443 of the sensor 244 are still located in the sealed space formed by using the sensor sealing assembly 24. As a sterile barrier, the sealing bottle 246 may form good isolation protection for the puncture end 2421 of the puncture needle 242 and the implantation portion 2443 of the sensor 244. The puncture end 2421 of the puncture needle 242 and the implantation portion 2443 of the sensor 244 are not contaminated for a second time. In subsequent packaging, a sterilized package for ensuring that the sensor 244 is located in a sterile environment does not need to be additionally added to the physiological sign detection apparatus 100, to reduce costs. In addition, because the sensor sealing assembly 24 may be independent of an internal circuit of the sensor assembly 20, it is possible to perform only radiation sterilization on the sensor sealing assembly 24, and no additional shielding protection needs to be set. Sterilization operations are simple, and an internal circuit of the sensor 244 is not damaged. In addition, the sensor sealing assembly 24 has a small volume, and a plurality of sensor sealing assemblies 24 can be sterilized simultaneously through one radiation sterilization operation, to greatly improve sterilization efficiency and reducing sterilization costs.

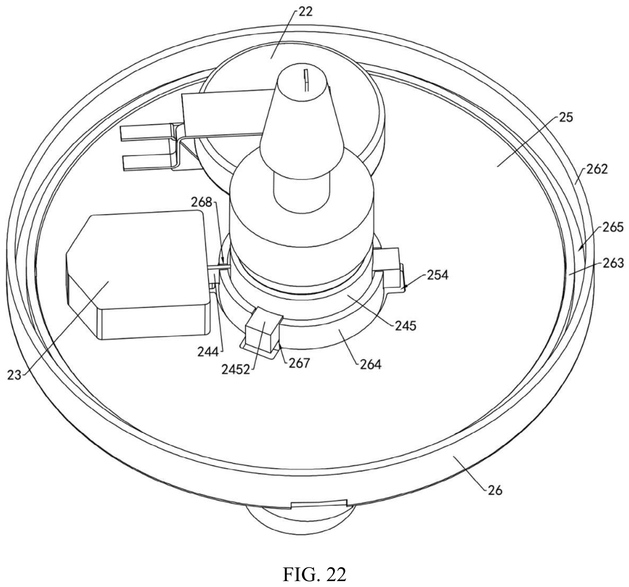

Refer to FIG. 14 and FIG. 22 together. FIG. 22 is a diagram of a partial structure of the sensor assembly 20 shown in FIG. 13.

In some embodiments, the lower cover 26 may include a bottom plate 261, a first side plate 262, a second side plate 263, and a blocking flange 264. The bottom plate 261 of the lower cover 26 may be generally in a disc shape. The first side plate 262 of the lower cover 26 surrounds and is connected to a circumferential edge of the bottom plate 261 of the lower cover 26, the second side plate 263 is located on an inner side of the first side plate 262 and is located on a same side of the bottom plate 261 of the lower cover 26 as the first side plate 262, a circumferential edge of the second side plate 263 is fastened to the bottom plate 261 of the lower cover 26, and a mounting groove 265 is formed between the first side plate 262 and the second side plate 263.

The lower cover 26 is provided with a first through hole 266, the first through hole 266 is located on an inner side of the second side plate 263 and runs through the bottom plate 261 of the lower cover 26, the blocking flange 264 is disposed around the first through hole 266, and the blocking flange 264 is located on a same side of the bottom plate 261 of the lower cover 26 as the first side plate 262. A side that is of the blocking flange 264 and that faces away from the bottom plate 261 of the lower cover 26 has a first avoidance groove 267. There may be one or more first avoidance grooves 267, and a plurality of first avoidance grooves 267 are spaced apart in a circumferential direction of the blocking flange 264. For example, there may be three first avoidance grooves 267. The side that is of the blocking flange 264 and that faces away from the bottom plate 261 of the lower cover 26 is further provided with a blocking groove 268, and the blocking groove 268 and the first avoidance groove 267 are spaced apart.

In some embodiments, the circuit board 25 has first mounting space 251, second mounting space 252, and a third through hole 253. The first mounting space 251 and the second mounting space 252 are spaced apart, and the third through hole 253 communicates with the first mounting space 251 and is spaced apart from the second mounting space 252. The first mounting space 251 and the second mounting space 252 may be through holes, or may be open grooves, and an opening of the open groove is disposed facing away from the bottom plate 261 of the lower cover 26. The circuit board 25 has a second avoidance groove 254, there are one or more second avoidance grooves 254, and a plurality of second avoidance grooves 254 are located on a circumferential side of the third through hole 253 and communicate with the third through hole 253. For example, there may be three second avoidance grooves 254.

The circuit board 25 is fastened to the bottom plate 261 of the lower cover 26, and is located on an inner side of the second side plate 263. The blocking flange 264 is inserted into the third through hole 253, the first mounting space 251 is disposed opposite to the blocking groove 268 in the radial direction, and the plurality of second avoidance grooves 254 are disposed opposite to the plurality of first avoidance grooves 267 in a one-to-one correspondence in the radial direction. The circuit board 25 and the lower cover 26 may form an integral mechanical part by using an adhesive process. For example, a contact surface between the bottom plate 261 of the lower cover 26 and the circuit board 25 may be coated with glue, so that the circuit board 25 is stuck and fastened to the lower cover 26.

The sensor sealing assembly 24 is inserted into the first through hole 266 of the lower cover 26, and the blocking block 2452 of the sensor base 245 is snap-fitted into the first avoidance groove 267, so that the sensor base 245 is snap-fitted into the lower cover 26. The sensor 244 passes through the blocking groove 268 and is connected to the connector 23. The connector 23 is fastened to the circuit board 25 and is electrically connected to the circuit board 25. The sensor 244 is electrically connected to the connector 23, to be electrically connected to the circuit board 25 by using the connector 23. The battery 22 is fastened to the circuit board 25 and is electrically connected to the circuit board 25, to supply power to the sensor assembly 20. There may be one or more batteries 22. For example, a single battery 22 or two batteries 22 connected in series may be used as a power supply of the sensor assembly 20, and a voltage of the battery 22 may be 1.5 volts (volt, V).

In this embodiment, the circuit board 25 and the lower cover 26 form an integral mechanical part by using an adhesive process, so that the circuit board 25 and a bottom plate 261 of the lower cover 26 are stably fastened, to avoid a collision caused by movement of the circuit board 25 relative to the lower cover 26 in a transportation process of the physiological sign detection apparatus 100, and help improve reliability of the physiological sign detection apparatus 100. The first mounting space 251 and the second avoidance groove 254 on the circuit board 25 may be used for positioning and/or limiting, so that the circuit board 25 is aligned with and assembled with the lower cover 26. In addition, the second avoidance groove 254 of the circuit board 25 may further avoid the blocking block 2452 of the sensor base 245, to prevent the blocking block 2452 from abutting against the circuit board 25 and cause a failure of the circuit board 25.

In some embodiments, a center of the first through hole 266 of the lower cover 26 is located or approximately located on an axis of the sensor assembly 20, and the sensor sealing assembly 24 is snap-fitted into the blocking flange 264, so that the puncture needle 242 and the implantation portion 2443 of the sensor 244 are located or close to the axis of the sensor assembly 20, and the puncture needle 242 and the implantation portion 2443 of the sensor 244 can extend in the middle of the lower cover 26. Based on the foregoing structural design, when the puncture needle 242 and the implantation portion 2443 of the sensor 244 are punctured into the organism 30, the puncture needle 242 and the implantation portion 2443 of the sensor 244 are subject to an even action force, and the puncture needle 242 is not easily skewed. This helps ensure that the puncture needle 242 is quickly punctured into the organism 30, and the organism 30 feels a small pain feeling.

In addition, in the sensor assembly 20, the sensor sealing assembly 24 is arranged in the middle, the circuit board 25 is arranged around the sensor sealing assembly 24, the battery 22, the connector 23, and another component are arranged on a circumferential side of the sensor sealing assembly 24, and a plurality of components of the sensor assembly 20 are arranged compactly, to facilitate miniaturization.

A diameter of the sensor assembly 20 may be less than 21 millimeters (mm), and a thickness is less than 3 millimeters. For example, a diameter of the sensor assembly 20 may be 20.6 millimeters, 20 millimeters, 19.7 millimeters, 19.5 millimeters, 18.8 millimeters, 18.3 millimeters, or 18 millimeters. The thickness of the sensor assembly 20 may be 2.95 millimeters, 2.9 millimeters, 2.85 millimeters, 2.8 millimeters, 2.78 millimeters, or the like. The sensor assembly 20 has a small volume, to help reduce a volume of the implantation apparatus 10 configured to implant the sensor assembly 20.

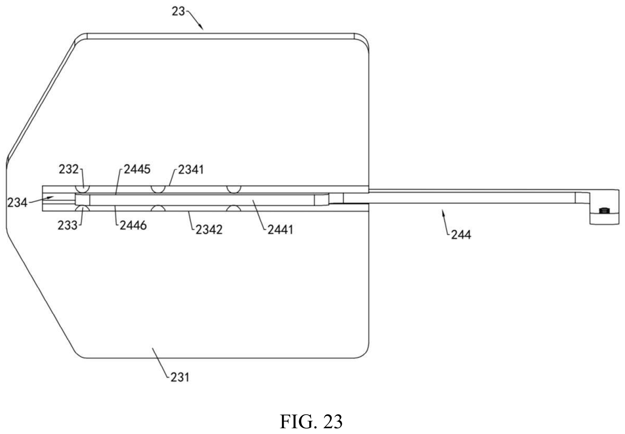

FIG. 23 is a diagram of an assembly structure of a sensor 244 and a connector 23 that are shown in FIG. 22.

In some embodiments, the connector 23 may include a connector body 231, a plurality of first spring plates 232, and a plurality of second spring plates 233. The connector body 231 is provided with a connecting groove 234, and the connecting groove 234 may include a first groove wall 2341 and a second groove wall 2342 that are disposed opposite to each other. The plurality of first spring plates 232 and the plurality of second spring plates 233 are both fastened to the connector body 231, at least a part of the plurality of first spring plates 232 is convexly disposed on the first groove wall 2341, at least a part of the plurality of second spring plates 233 is convexly disposed on the second groove wall 2342, and a gap is formed between the plurality of first spring plates 232 and the plurality of second spring plates 233. The connecting portion 2441 of the sensor 244 is snap-fitted into the connecting groove 234 of the connector 23, and is electrically connected to the plurality of first spring plates 232 and the plurality of second spring plates 233. For example, the sensor 244 may be generally plate-like, the sensor 244 may include a first surface 2445 and a second surface 2446 that are disposed facing away from each other, and the first surface 2445 and the second surface 2446 each are provided with a conductive layer. The plurality of first spring plates 232 of the sensor 244 are electrically connected to a conductive layer of the first surface 2445, and the plurality of second spring plates 233 of the sensor 244 are electrically connected to a conductive layer of the second surface 2446.

In the conventional technology, when a sensor is electrically connected to a circuit board, a connecting portion of the sensor needs to be folded, so that the connecting portion of the sensor is parallel to the circuit board, to be welded with a pad of the circuit board. Consequently, costs of the sensor are increased, and a folding position of the sensor is easily damaged. In addition, only one surface of the sensor can be welded to the circuit board, and a large connection area needs to be set for each of the sensor and the circuit board. Consequently, a volume of the sensor and a volume of the circuit board are large. In this embodiment, at least a part of the plurality of first spring plates 232 of the connector 23 is convexly disposed on the first groove wall 2341 of the connecting groove 234, and at least a part of the plurality of second spring plates 233 is convexly disposed on the second groove wall 2342 of the connecting groove 234. A conductive layer on a surface of the sensor 244 can be electrically connected to the plurality of first spring plates 232 and the plurality of second spring plates 233 only by snap-fitting the sensor 244 into the connecting groove 234 of the connector 23. Assembly between the sensor 244 and the connector 23 is simpler, and an electrical connection between the sensor 244 and the connector 23 is more stable. In addition, the sensor 244 can maintain a plate-like structure, and does not need to be folded additionally due to an electrical connection requirement, so that the sensor 244 has high reliability. In addition, because spring plates are convexly disposed on groove walls on two sides of the connecting groove 234, the connector 23 has more contacts configured to be electrically connected to the sensor 244, to reduce an arrangement area of the first spring plate 232 and the second spring plate 233, thereby reducing a volume of the connector 23, helping miniaturize the sensor assembly 20. In addition, miniaturization of the sensor assembly 20 also helps miniaturize the physiological sign detection apparatus 100.

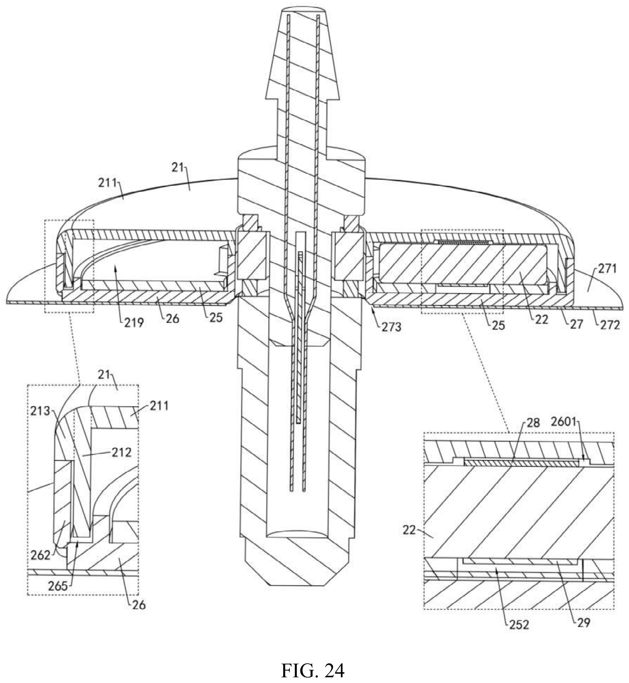

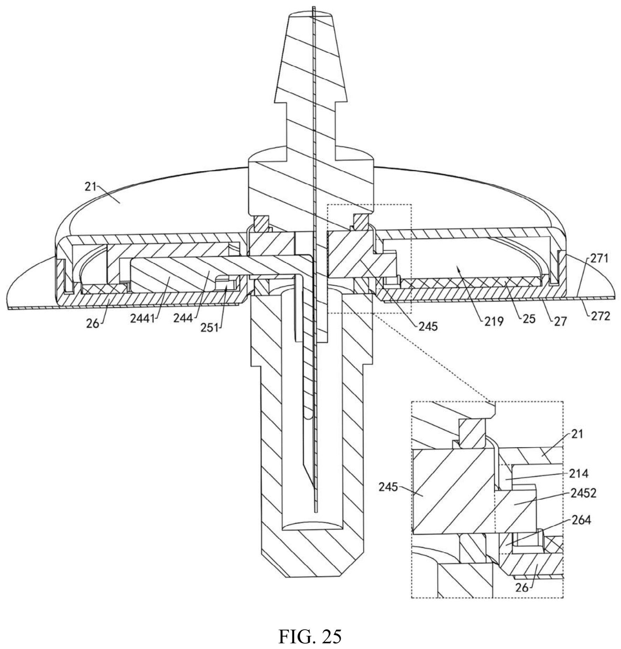

Refer to FIG. 14, FIG. 24, and FIG. 25 together. FIG. 24 is a diagram of a cross-sectional structure after the sensor assembly 20 shown in FIG. 13 is cut along D-D in some embodiments. FIG. 25 is a diagram of a cross-sectional structure after the sensor assembly 20 shown in FIG. 13 is cut along E-E in some embodiments.

In some embodiments, the upper cover 21 is fastened to the lower cover 26, accommodation space 219 is formed between the upper cover 21 and the lower cover 26, and the circuit board 25, the battery 22, the connector 23, the connecting portion 2441 of the sensor 244, and at least a part of the sensor base 245 are located in the accommodation space 219. The upper cover 21 may include a top plate 211, a side plate 212, a side plate flange 213, and a snap-fitting flange 214, the side plate 212 of the upper cover 21 surrounds and is connected to a circumferential edge of the top plate 211 of the upper cover 21, and the side plate flange 213 surrounds and is connected to an outer circumferential side of the side plate 212 of the upper cover 21. The side plate 212 of the upper cover 21 is mounted in the mounting groove 265 of the lower cover 26, so that the upper cover 21 is fastened to the lower cover 26. The side plate flange 213 abuts against the first side plate 262 of the lower cover 26. The upper cover 21 and the lower cover 26 may form an integral mechanical part by using an adhesive process. For example, all contact surfaces between the lower cover 26 and the upper cover 21 are coated with glue, so that the lower cover 26 and the upper cover 21 are stuck and fastened.

In this embodiment, the mounting groove 265 of the lower cover 26 has positioning and limiting roles, so that the upper cover 21 is mounted and fastened to the lower cover 26. In addition, the side plate flange 213 of the upper cover 21 abuts against the first side plate 262 of the lower cover 26, and the upper cover 21 and the lower cover 26 may be integrated by using an adhesive, so that sealing performance between the upper cover 21 and the lower cover 26 is better, to avoid a case in which a contaminant enters the accommodation space 219 from a gap between the upper cover 21 and the lower cover 26, and consequently, an electronic element 204 located in the accommodation space 219 is damaged.

The connecting portion 2441 of the sensor 244 is mounted in the first mounting space 251 of the circuit board 25. Components of the sensor 244 may further include a positive spring plate 28 and a negative spring plate 29. A side that is of the top plate 211 of the upper cover 21 and that faces the lower cover 26 has an accommodation groove 2601, an end of the positive spring plate 28 is mounted in the accommodation groove 2601 and is in contact with and electrically connected to the battery 22, and an end of the negative spring plate 29 is mounted in the second mounting space 252 of the circuit board 25, and is in contact with and electrically connected to the battery 22. The other end of the positive spring plate 28 and the other end of the negative spring plate 29 may be fastened and electrically connected to the circuit board 25, to conduct the battery 22 and the circuit board 25.

The upper cover 21 has a second through hole 215, the second through hole 215 is located on the top plate 211 of the upper cover 21, and the second through hole 215 directly faces and communicates with the first through hole 266. The sensor base 245 is snap-fitted into the lower cover 26 and/or the upper cover 21. To be specific, the sensor base 245 is snap-fitted into the lower cover 26, or the sensor base 245 is snap-fitted into the lower cover 26 and the upper cover 21, or the sensor base 245 is snap-fitted into the upper cover 21. For example, after the circuit board 25, the sensor sealing assembly 24, the connector 23, and the battery 22 are all first mounted on the lower cover 26, the needle base 241 passes through the second through hole 215 of the upper cover 21, and then the upper cover 21 is mounted on the lower cover 26. The blocking block 2452 of the sensor base 245 is located between the blocking flange 264 of the lower cover 26 and the snap-fitting flange 214 of the upper cover 21, and the blocking block 2452 is snap-fitted into the snap-fitting flange 214 and/or the blocking flange 264, so that the sensor base 245 is snap-fitted into the lower cover 26 and/or the upper cover 21. For example, there are three blocking blocks 2452, to ensure that the sensor base 245 is stably snap-fitted into the lower cover 26 and/or the upper cover 21. The sensor base 245 is exposed from the first through hole 266 and exposed from the second through hole 215. A part of the sensor base 245 may be located in the first through hole 266 and the second through hole 215.

In this embodiment, the sensor base 245 is snap-fitted into the lower cover 26 and/or the upper cover 21, so that the sensor base 245 is stably fastened to the lower cover 26 and the upper cover 21, to avoid a case in which the sensor 244 is loose relative to the lower cover 26 and/or the upper cover 21 when the sealing bottle 246 and the needle base 241 are detached from the sensor base 245. The lower cover 26 has the first through hole 266, and the sensor base 245 is exposed from the first through hole 266, so that the sealing bottle 246 can pass through the first through hole 266 and is exposed from the lower cover 26. The upper cover 21 has the second through hole 215, and the sensor base 245 is exposed from the second through hole 215, so that the needle base 241 can pass through the second through hole 215 of the upper cover 21. In this way, components of the sensor sealing assembly 24 are assembled, and then assembled with the lower cover 26 and the upper cover 21, to protect the implantation portion 2443 of the sensor 244 by the sealing bottle 246 and reduce a damage risk.

In some embodiments, the bonding member 27 has a first surface 271 and a second surface 272 that are disposed facing away from each other, the first surface 271 of the bonding member 27 is stuck on a side that is of the lower cover 26 and that faces away from the upper cover 21, and the second surface 272 of the bonding member 27 is configured to be stuck on the organism 30 when the sensor assembly 20 abuts against the organism 30, so that the sensor assembly 20 is stuck on a skin surface of the organism 30. For example, the bonding member 27 may be generally plate-like, the bonding member 27 has a fourth through hole 273, the fourth through hole 273 is disposed opposite to the first through hole 266, and the sensor base 245 is exposed from the fourth through hole 273, so that the sealing bottle 246 can pass through the fourth through hole 273 and be hermetically connected to the sensor base 245. The bonding member 27 may be a medical back adhesive, and the bonding member 27 can maintain stable bonding for at least 14 days.

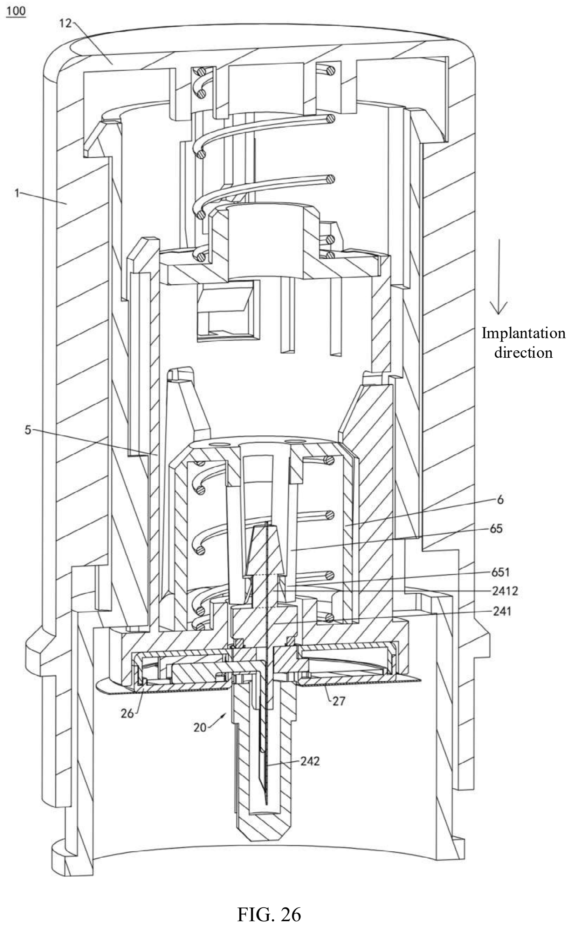

Refer to FIG. 8A, FIG. 13, and FIG. 26 together. FIG. 26 is a diagram 3 of a partial cross-sectional structure after the physiological sign detection apparatus 100 shown in FIG. 2 is cut along B-B.

In some embodiments, the sensor assembly 20 is fastened to the implantation base 5, and is located on a side that is of the implantation base 5 and that faces away from the top wall 12 of the housing 1. An axial direction of the sensor assembly 20 may coincide with the axial direction of the implantation base 5. For example, the sensor assembly 20 is mounted in the second accommodation space 55 of the implantation base 5. A snap-fitting hook groove 269 may exist on an outer side surface of the lower cover 26 of the sensor assembly 20. The snap-fitting hook 5041 of the fifth elastic arm 504 of the implantation base 5 is snap-fitted into the snap-fitting hook groove 269 of the lower cover 26. When there are a plurality of fifth elastic arms 504, the snap-fitted sensor assembly 20 can be enabled to be subject to a balanced force and be stably connected to the implantation base 5.

For example, the needle extraction base 6 is snap-fitted into the needle base 241. The needle base 241 may pass through the mounting hole 502 of the implantation base 5 and extend to the needle extraction base 6, and the snap-fitting hook 651 of the first cantilever 65 is snap-fitted into the snap-fitting portion 2412 of the needle base 241, so that the needle base 241 can move synchronously with the needle extraction base 6. When there are three first cantilevers 65, the snap-fitted needle base 241 can be subject to a balanced force. When the implantation base 5 moves in the implantation direction, the implantation base 5 pushes the sensor assembly 20 to also move in the implantation direction, so that the bonding member 27 of the sensor assembly 20 can be stuck on the organism. In addition, the implantation base 5 pushes the needle base 241 to the organism through the needle extraction base 6, so that the puncture needle 242 can be punctured into the organism. When the needle extraction base 6 moves in a reverse direction of the implantation direction, the first cantilever 65 drives, through the needle base 241, the puncture needle 242 to move in the reverse direction of the implantation direction, so that the puncture needle 242 is detached from the organism.

In some embodiments, a center of the mounting hole 502 of the implantation base 5 is located or close to an axis of the physiological sign detection apparatus 100, and the plurality of first cantilever 65 are disposed around the axis of the physiological sign detection apparatus 100, so that when the needle base 241 of the sensor assembly 20 passes through the mounting hole 502 and is snap-fitted into the first cantilever 65, an extension direction of the puncture needle 242 is located on or close to the axis of the physiological sign detection apparatus 100. Therefore, when the implantation base 5 pushes the sensor assembly 20 to move in the implantation direction, the puncture needle 242 is subject to a more concentrated force, to help reduce a puncture pain feeling of the user.

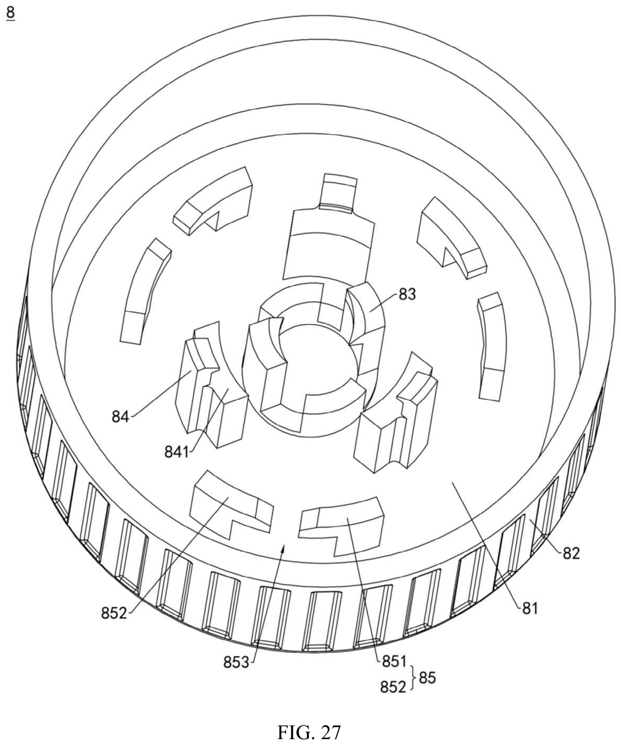

FIG. 27 is a diagram of a structure of the housing cap 8 shown in FIG. 2 in some embodiments.

In some embodiments, the housing cap 8 may include a bottom wall 81, a side wall 82, a pawl 83, a blocking member 84, and a limiting member 85. The side wall 82 of the housing cap 8 surrounds and is connected to a circumferential edge of the bottom wall 81 of the housing cap 8. The pawl 83, the limiting member 85, the blocking member 84, and the side wall 82 of the housing cap 8 are all located on a same side of the bottom wall 81 of the housing cap 8. The pawl 83, the blocking member 84, and the limiting member 85 are all located on an inner side of the side wall 82 of the housing cap 8. The housing cap 8 may be generally cylindrical. An end of the pawl 83 is fastened to the bottom wall 81 of the housing cap 8, the other end that is of the pawl 83 and that faces away from the bottom wall 81 of the housing cap 8 is provided with a snap-fitting hook, and the snap-fitting hook of the pawl 83 may be formed by a smooth protrusion from a side to the other side. There may be one or more pawls 83, and a plurality of pawls 83 are spaced apart around an axis of the housing cap 8. For example, there are two pawls 83, and extension directions of the two pawls 83 are the same. The blocking member 84 is located on an outer side of the pawl 83, and is spaced apart from the pawl 83. An end of the blocking member 84 is fastened to the bottom wall 81 of the housing cap 8. The other end that is of the blocking member 84 and that faces away from the bottom wall 81 of the housing cap 8 is provided with a snap-fitting hook. The blocking member 84 may further include a platform 841, and the platform 841 is spaced apart from the snap-fitting hook of the blocking member 84 in an axial direction of the housing cap 8. There may be one or more blocking members 84, and a plurality of blocking members 84 are spaced apart around the axis of the housing cap 8. For example, there are three blocking members 84, and platforms 841 of the three blocking members 84 have a same height.

The limiting member 85 is located on an outer side of the blocking member 84, and is spaced apart from the blocking member 84. An end of the limiting member 85 is fastened to the bottom wall 81 of the housing cap 8. There may be one or more limiting members 85, and a plurality of limiting members 85 are spaced apart around the axis of the housing cap 8. For example, there are three limiting members 85, to prevent a snap-fitted part from being detached from the limiting member 85. The limiting member 85 may include a first limiting block 851 and a second limiting block 852, the first limiting block 851 and the second limiting block 852 are spaced apart, and blocking space 853 is formed between the first limiting block 851 and the second limiting block 852.

In some embodiments, the housing cap 8 may be an integral mechanical part. The housing cap 8 is made of engineering plastic such as polycarbonate or a polycarbonate and acrylonitrile butadiene styrene mixture. In this embodiment, the polycarbonate and acrylonitrile butadiene styrene plastic have features such as heat resistance, impact resistance, good mechanical performance, high hardness, and good processing performance. The housing cap 8 is made of the engineering plastic such as the polycarbonate or the polycarbonate and acrylonitrile butadiene styrene mixture, to help reduce manufacturing costs of the housing cap 8. In addition, the housing cap 8 has a strong scratch resistance capability. As an outer layer structure of the physiological sign detection apparatus 100, the housing cap 8 can prevent a surface of the physiological sign detection apparatus 100 from being scratched, to help improve external aesthetics of using the physiological sign detection apparatus 100.

In some other embodiments, the housing cap 8 may alternatively be made of another material, for example, engineering plastic such as polyvinyl chloride resin or polystyrene resin, or metal such as stainless steel or aluminum alloy. This is not strictly limited in this application.

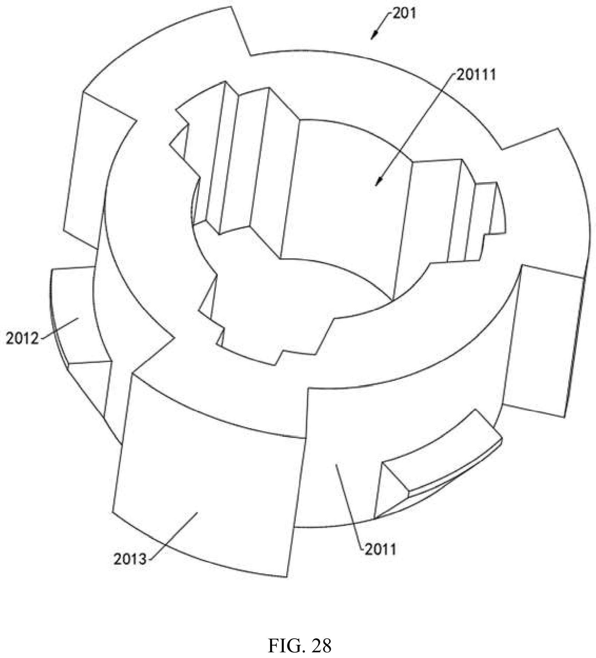

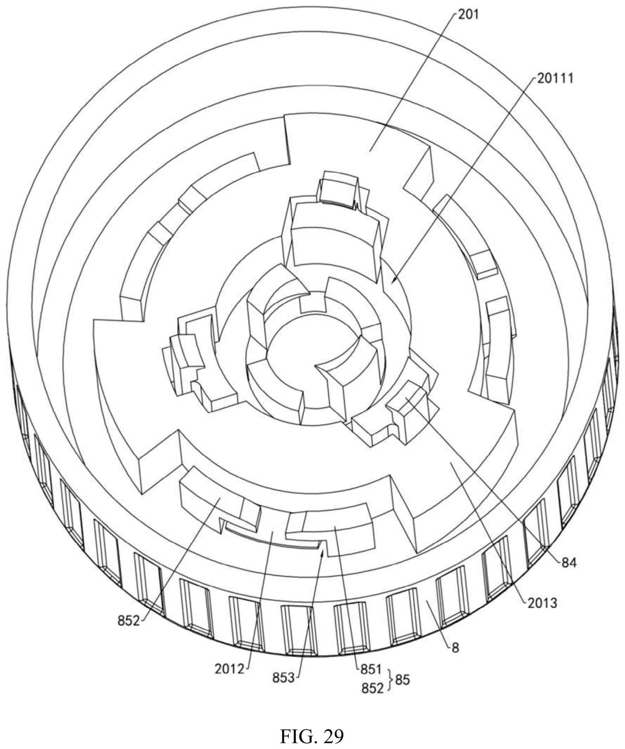

FIG. 28 is a diagram of a structure of a drying member 201. FIG. 29 is a diagram of an assembly structure of the drying member 201 and the housing cap 8.

In some embodiments, the physiological sign detection apparatus 100 may further include the drying member 201, and the drying member 201 is snap-fitted into the housing cap 8. For example, the drying member 201 may be made of a material having an adsorption function, for example, silica gel, artificial fiber, or molecular sieve. The drying member 201 is configured to absorb moisture, so that humidity of the inner side of the physiological sign detection apparatus 100 is kept within a proper range. In addition, disposing of the drying member 201 does not affect a sterilization operation of the physiological sign detection apparatus 100. The drying member 201 may include a main body portion 2011, a blocking bump 2012, and a limiting bump 2013. There may be three blocking bumps 2012 and three limiting bumps 2013. The three blocking bumps 2012 and the three limiting bumps 2013 are spaced apart around the axis of the housing cap 8. The main body portion 2011 has a through hole 20111, and the blocking member 84 is inserted into the through hole 20111 of the main body portion 2011. The three limiting bumps 2013 are mounted in spacings between the three limiting members 85 in a one-to-one correspondence, the three blocking bumps 2012 are mounted in blocking space 853 between the three limiting members 85 in a one-to-one correspondence, and the first limiting block 851 and the second limiting block 852 are snap-fitted into the blocking bump 2012.

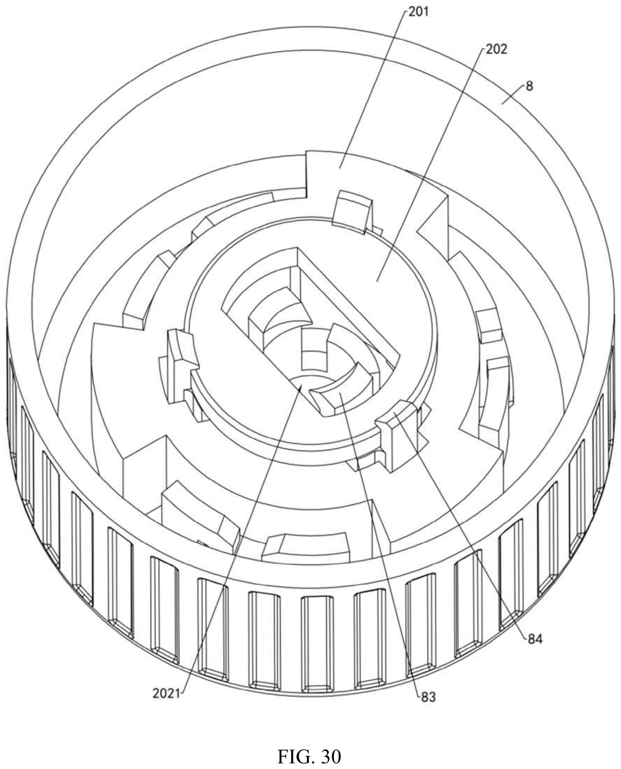

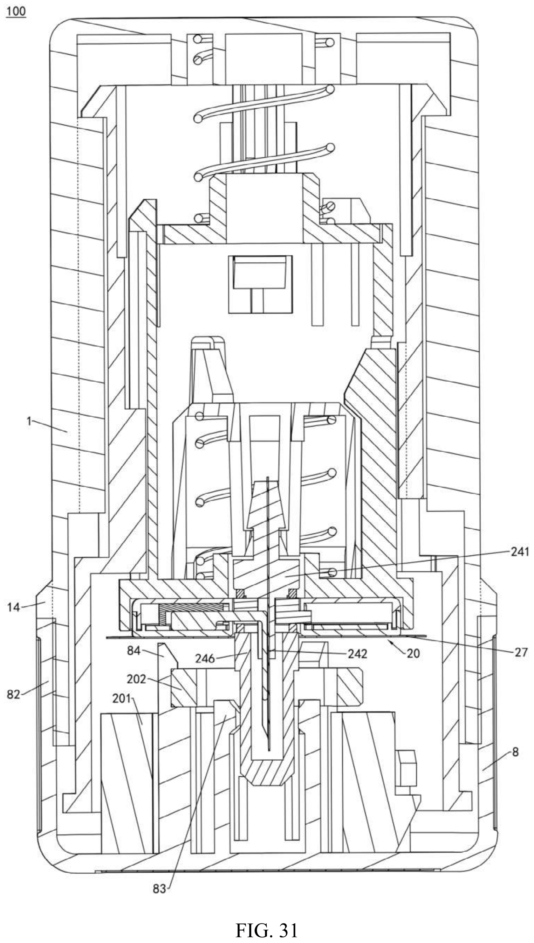

FIG. 30 is a diagram of an assembly structure of a magnetic element 202, a drying member 201, and a housing cap 8. FIG. 31 is a diagram 4 of a partial cross-sectional structure after the physiological sign detection apparatus 100 shown in FIG. 2 is cut along B-B.

In some embodiments, the housing cap 8 is detachably fastened to an end that is of the side wall 11 of the housing 1 and that faces away from the top wall 12 of the housing 1, and the housing cap 8 wraps the abutting end 301 and closes the opening 13 of the housing 1. For example, a detachable connection relationship between the housing 1 and the housing cap 8 may be implemented in a connection manner such as a magnetic connection, a snap-fitting connection, an interference connection, or a threaded connection. This is not strictly limited in this embodiment of this application.

In some embodiments, the physiological sign detection apparatus 100 may further include the magnetic element 202, and the magnetic element 202 is snap-fitted into the housing cap 8. For example, the magnetic element 202 may be a magnet. The magnetic element 202 has a through hole 2021, the pawl 83 of the housing cap 8 is inserted into the through hole 2021 of the magnetic element 202, and the magnetic element 202 is placed on the platform 841 of the blocking member 84 (as shown in FIG. 27), and is snap-fitted into the snap-fitting hook of the blocking member 84. The magnetic element 202 can form a magnetic field around the magnetic element 202, and serves as a magnetic switch. A magnetic switch (not shown in FIG. 31) is disposed on the inner side of the sensor assembly 20. When the housing cap 8 is fastened to the housing 1, the magnetic element 202 is close to the sensor assembly 20, the magnetic switch is located in a magnetic field of the magnetic element 202, the magnetic switch controls the sensor assembly 20 to be in a standby state, and power consumption of the sensor assembly 20 is extremely low, to help reduce power consumption of the battery in the sensor assembly 20 and prolong product use time. When a product is unpacked and the magnetic element 202 is detached from the housing 1 with the housing cap 8, the magnetic switch cannot sense the magnetic field, and the magnetic switch controls the sensor assembly 20 to be activated, so that the sensor assembly 20 is started and is in a working state.

In this embodiment, the magnetic element 202 is snap-fitted into the housing cap 8, assembly of the magnetic element 202 and the housing cap 8 is simple, and the magnetic element 202 may be mounted in the housing 1 or detached from the housing 1 with the housing cap 8, to implement control of the magnetic switch in the sensor assembly 20.

In some embodiments, the physiological sign detection apparatus 100 may further include a sealing member (not shown in FIG. 31), and the housing cap 8 is hermetically connected to the housing 1 by using the sealing member. For example, the sealing member may be mounted between a first flange 14 of the housing 1 and the side wall 82 of the housing cap 8, and the side wall 82 of the housing cap 8 is hermetically connected to the first flange 14 of the housing 1 by using the sealing member. The sealing member may be made of an elastic polymer material such as nitrile butadiene rubber, silicon rubber, or fluorine rubber.

In this embodiment, the sealing member is hermetically connected to the housing 1 and the housing cap 8, to help improve sealing performance between the housing cap 8 and the housing 1, and prevent a contaminant from entering the inner side of the physiological sign detection apparatus 100. In addition, a sealed environment is formed inside the physiological sign detection apparatus 100, to prevent the bonding member 27 from being interfered by an external environment in a storage process, and help maintain bonding performance of the bonding member 27.

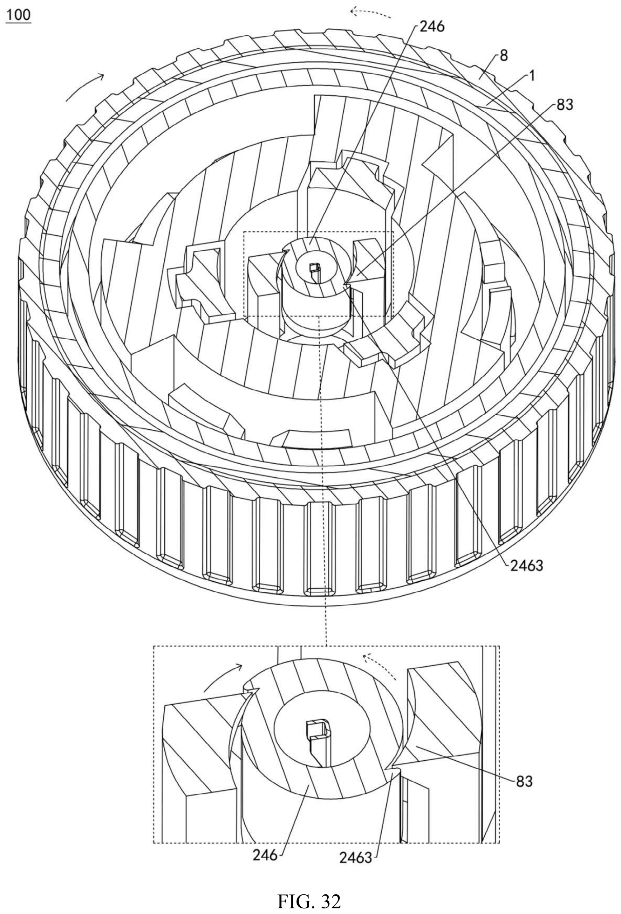

Refer to FIG. 31 and FIG. 32 together. FIG. 32 is a diagram of a partial cross-sectional structure after the physiological sign detection apparatus 100 shown in FIG. 2 is cut along F-F.

In some embodiments, the housing cap 8 is threadedly connected to the housing 1, and the pawl 83 of the housing cap 8 and the ratchet 2463 of the sealing bottle 246 are disposed through cooperation. For example, an internal thread (not shown in FIG. 31) is disposed on an inner side surface of the side wall 82 of the housing cap 8, an external thread (not shown in FIG. 31) is disposed on an outer side surface of the side wall 11 of the housing 1, and the internal thread of the housing cap 8 cooperates with the external thread of the housing 1, so that the housing cap 8 is threadedly connected to the housing 1. When the housing cap 8 is fastened to the housing 1, the side wall 82 of the housing cap 8 abuts against the first flange 14, and the first flange 14 limits a mounting depth of mounting the housing cap 8 on the housing 1, to enhance sealing performance between the housing cap 8 and the housing 1, and prevent a contaminant from entering the inner side of the physiological sign detection apparatus 100. A knurl may be disposed on the outer side surface of the side wall 82 of the housing cap 8, and the knurl may increase a friction force, so that the housing cap 8 can be screwed on the housing 1 or the housing cap 8 can be screwed off the housing 1. A rotation direction of an internal thread of the housing cap 8 is the same as a rotation direction of an internal thread of the sealing bottle 246. In a process in which the housing cap 8 is screwed on the housing 1 (moves in a direction shown by a dashed arrow in FIG. 32), the pawl 83 is slidably connected to the ratchet 2463. In other words, in the process in which the housing cap 8 is screwed on the housing 1, the pawl 83 does not push the ratchet 2463 to move, to avoid a case in which the sealing bottle 246 is loose relative to the needle base 241 when the housing cap 8 is assembled to the housing 1. In a process in which the housing cap 8 is detached from the housing 1 (moves in a direction shown by a solid arrow in FIG. 32), the pawl 83 is snap-fitted into the ratchet 2463, and pushes the sealing bottle 246 to rotate. Because the sealing bottle 246 is threadedly connected to the needle base 241, the sealing bottle 246 is screwed off relative to the needle base 241, so that the sealing bottle 246 is detached from the needle base 241. A quantity of circles of internal threads of the housing cap 8 may be greater than a quantity of circles of internal threads of the sealing bottle 246, so that the sealing bottle 246 may be detached from the needle base 241 before the housing cap 8 is detached from the housing 1.

In this embodiment, the housing cap 8 is threadedly connected to the housing 1, the sealing bottle 246 is threadedly connected to the needle base 241, and the pawl 83 of the housing cap 8 cooperates with the ratchet 2463 of the sealing bottle 246. When the housing cap 8 is detached from the housing 1, the sealing bottle 246 may be pushed to be detached from the needle base 241, so that the puncture needle 242 and the sensor 244 are exposed, to help simplify operation steps of the physiological sign detection apparatus 100, and improve use experience of the user.

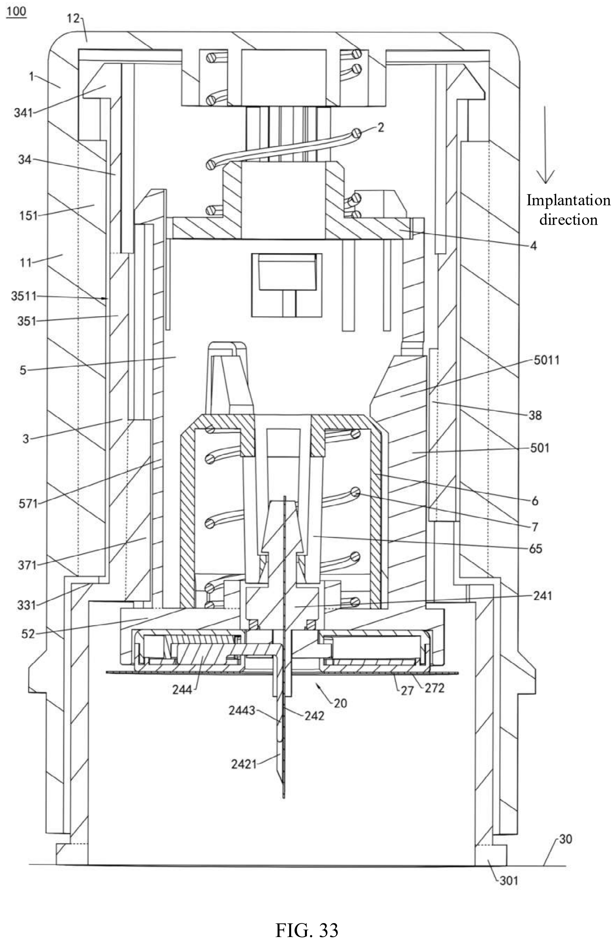

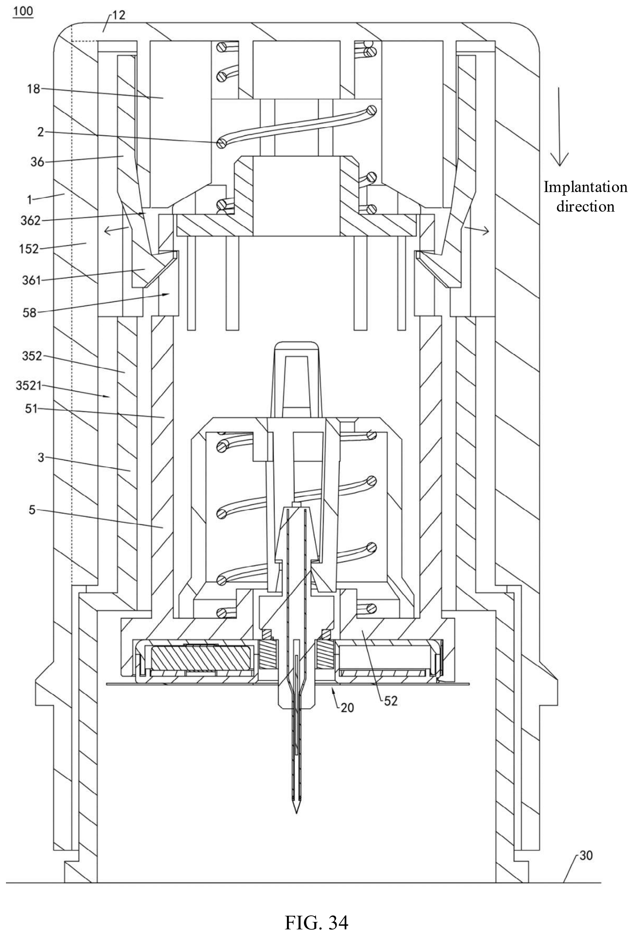

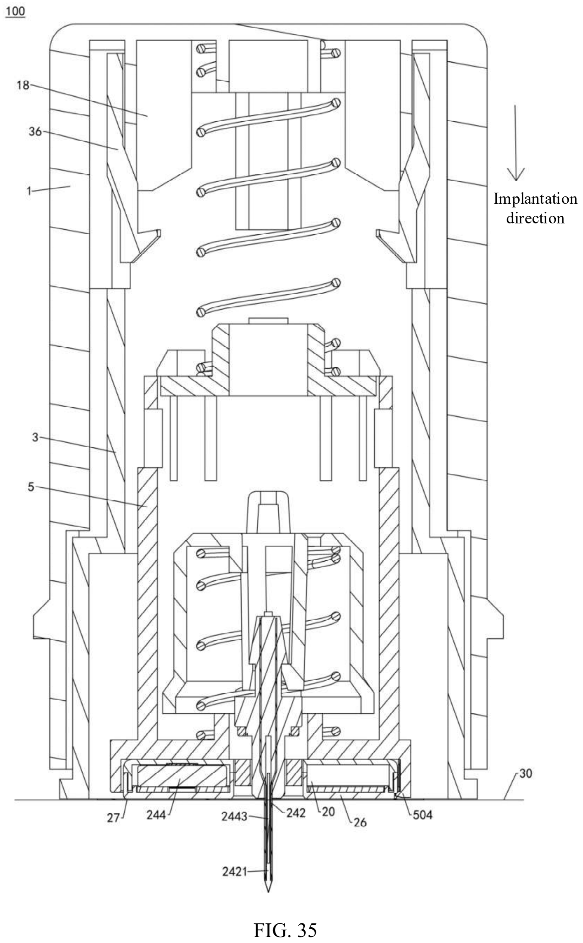

FIG. 33 is a diagram of a partial cross-sectional structure after the physiological sign detection apparatus 100 shown in FIG. 2 is cut along B-B in a pressing process. FIG. 34 is a diagram of a partial cross-sectional structure after the physiological sign detection apparatus 100 shown in FIG. 2 is cut along A-A in a pressing process. FIG. 35 is a diagram of a partial cross-sectional structure after the physiological sign detection apparatus 100 shown in FIG. 2 is cut along A-A in a puncturing process.

In some embodiments, the physiological sign detection apparatus 100 has a pressing process, a puncturing process, and a needle withdrawal process in an implantation process. In the implantation process of the physiological sign detection apparatus 100, the housing cap 8 is detached from the housing 1 (as shown in FIG. 31), and the abutting end 301 of the base 3 abuts against the organism 30, and maintains a position unchanged relative to the organism 30. The abutting end 301 may be configured to increase a contact area of abutting against skin of the organism 30 by the base 3, to reduce pressure, thereby helping reduce a pain feeling when the base 3 abuts against the skin of the organism 30, and improving user experience. When the physiological sign detection apparatus 100 is in the pressing process, and the housing 1 moves in a direction close to the organism 30 relative to the base 3, that is, when the housing 1 moves in the implantation direction, the first sliding block 151 is detached from the hook 341 of the first elastic arm 34, and moves in a direction close to the organism 30 along the sliding groove 3511 of the first guiding arm 351, the second sliding block 152 moves in a direction close to the organism 30 along the sliding groove 3521 of the second guiding arm 352, the top wall 12 of the housing 1 compresses the first elastic member 2 in the implantation direction, and the first elastic member 2 pushes the stopper member 4 and the implantation base 5 to move in the implantation direction, to push the implantation base 5 and the sensor assembly 20 toward the organism 30. The third guiding arm 571 moves in the implantation direction relative to the third sliding block 371. The first elastic member 2 may push the stopper member 4 and the implantation base 5 to move by releasing elastic potential energy. The step surface 331 and the hook 341 of the first elastic arm 34 limit movement of the first sliding block 151 in the axial direction, to help control implantation strength and an implantation depth of the physiological sign detection apparatus 100.

When the housing 1 moves in the direction close to the organism 30 relative to the base 3, the toggle 18 moves in the insertion direction along the guiding groove 362 of the second elastic arm 36, and the toggle 18 pushes the second elastic arm 36, so that the end that is of the second elastic arm 36 and that is provided with the snap-fitting hook 361 moves toward an outer side of the side plate 51 of the implantation base 5 (as shown by an arrow in FIG. 34), and the snap-fitting hook 361 of the second elastic arm 36 is detached from the snap-fitting space 58 of the implantation base 5. The implantation base 5 is free from a limit of the second elastic arm 36 of the base 3, and continues to move toward the organism 30 under an action of the elastic force of the first elastic member 2.

The bottom plate 52 of the implantation base 5 pushes the sensor assembly 20 to get close to the organism 30. The baffle plate 38 of the base 3 abuts against the fourth elastic arm 501 of the implantation base 5, and limits the fourth elastic arm 501 in the radial direction, to prevent the needle extraction base 6 from pushing away the snap-fitting hook 5011 of the fourth elastic arm 501 under an action of the second elastic member 7. When the implantation base 5 moves in the direction close to the organism 30, the fourth elastic arm 501 of the implantation base 5 pushes the needle extraction base 6 to move synchronously in the implantation direction with the implantation base 5. The first cantilever 65 of the needle extraction base 6 pushes the needle base 241 to move synchronously in the implantation direction with the implantation base 5. The implantation base 5 pushes the needle base 241 to the organism 30 by using the needle extraction base 6, so that the needle base 241 and the sensor 244 move synchronously in the direction close to the organism 30. The implantation base 5 pushes the sensor assembly 20 to move in the implantation direction until the sensor assembly 20 abuts against the organism 30, the puncture end 2421 of the puncture needle 242 and the implantation portion 2443 of the sensor 244 are punctured into the organism 30, and the second surface 272 of the bonding member 27 is stuck on the organism 30.

In the conventional technology, an implantation apparatus of a physiological sign detection apparatus starts an implantation process of a sensor by using an exposed button. In a process of product assembly, packaging, transportation, or the like, when a product drops or a user holds the physiological sign detection apparatus, the button is easily touched. Consequently, the implantation process is triggered incorrectly, and a sensor assembly cannot be ejected directly facing a detection position. Consequently, the sensor cannot be implanted into the detection position, and the physiological sign detection apparatus fails.

In this embodiment, the housing 1 is provided with no button. After the housing 1 and the housing cap 8 (as shown in FIG. 31) are assembled, the housing 1 and the housing cap 8 form a sealed cavity, to form good isolation and protection for an implantation portion 2443 and the sensor assembly 20 on an inner side. When the housing cap 8 is not detached from the housing 1, the implantation apparatus 10 does not trigger an implantation action, and the sensor assembly 20 is not ejected. In addition, the sensor assembly 20 is ejected only when the user holds the base 3 against the organism 30 and pushes the housing 1 toward the organism 30. Therefore, a risk of false triggering of the physiological sign detection apparatus 100 is low, and reliability is higher. In addition, in the implantation process, a movement direction of the housing 1 is consistent with a movement direction of the puncture needle 242, an implantation direction of the sensor assembly 20 is easy to control, an implantation force of the sensor assembly 20 is controlled by the first elastic member 2, and a magnitude of the implantation force is stable and is not affected by another external force. The physiological sign detection apparatus 100 has a moderate implantation force and a fast implantation speed, to help reduce a pain feeling of the user.

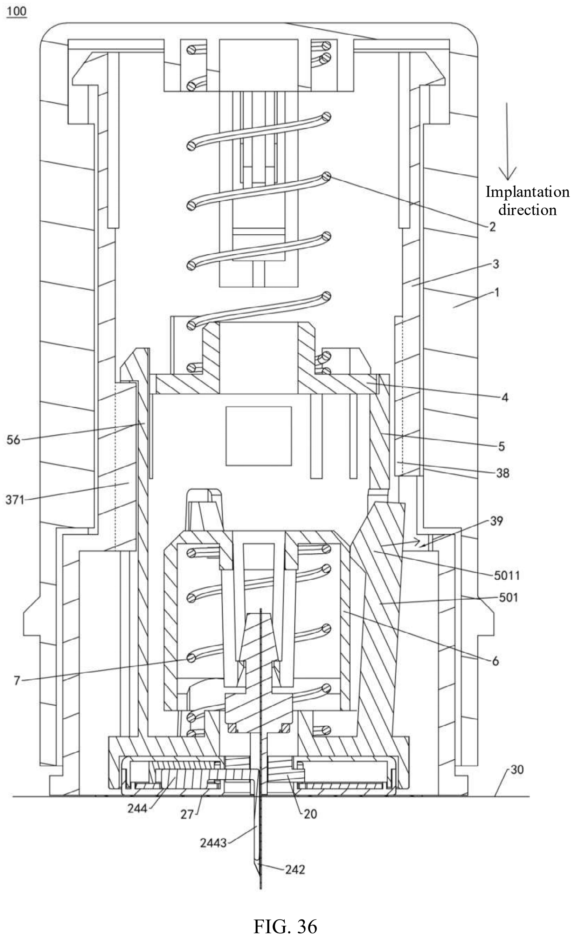

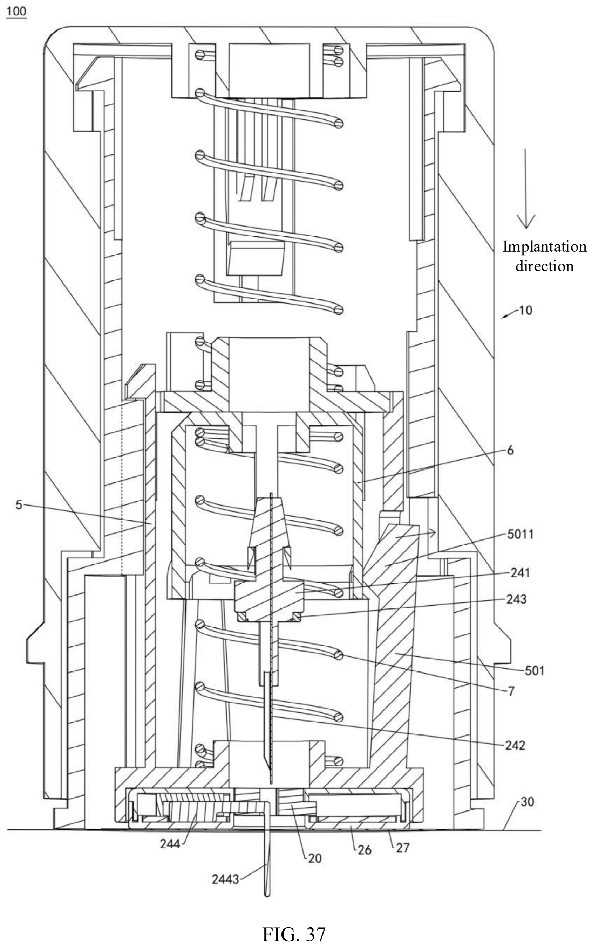

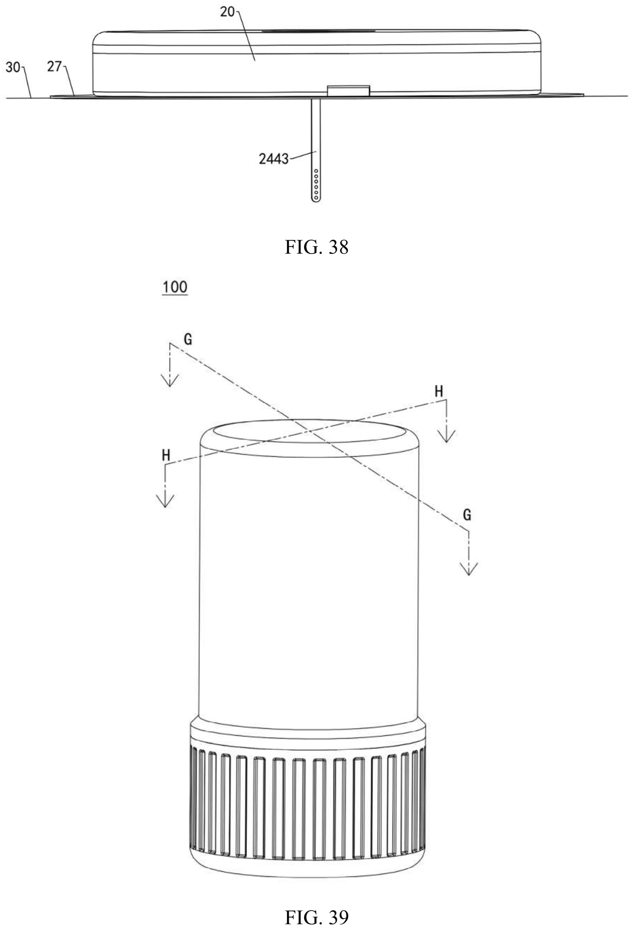

FIG. 36 is a diagram of a partial cross-sectional structure after the physiological sign detection apparatus 100 shown in FIG. 2 is cut along B-B in a puncturing process. FIG. 37 is a diagram of a partial cross-sectional structure after the physiological sign detection apparatus 100 shown in FIG. 2 is cut along B-B in a needle extraction state. FIG. 38 is a diagram in which a sensor assembly 20 is stuck on an organism 30.

In some embodiments, when the housing 1 moves in a direction close to the organism 30 relative to the base 3, the first elastic member 2 is compressed. When the first elastic member 2 applies an action force, the stopper member 4 pushes the implantation base 5 to move in the direction close to the organism 30. The third elastic arm 56 of the implantation base 5 moves in the implantation direction relative to the third sliding block 371, the implantation base 5 pushes the sensor assembly 20 to move in the implantation direction, and the puncture needle 242 is punctured into the organism 30. When the snap-fitting hook of the third elastic arm 56 abuts against the third sliding block 371, motion of the implantation base 5 relative to the base 3 is limited. In this case, the bonding member 27 of the sensor assembly 20 reaches a surface of the organism 30, and a depth of puncturing the puncture needle 242 and the implantation portion 2443 of the sensor 244 into the organism 30 is just proper. In this embodiment, the snap-fitting hook of the third elastic arm 56 cooperates with the third sliding block 371, to limit a movement distance of the implantation base 5 in the implantation direction to a proper range, to avoid excessive impact on the organism 30 caused by free movement of the implantation base 5 under an action of the first elastic member 2, thereby causing a pain to the user.

In some embodiments, when the physiological sign detection apparatus 100 is in the puncturing process, and the sensor 244 is implanted into the organism 30 under guidance of the puncture needle 242, the snap-fitting hook 5011 of the fourth elastic arm 501 reaches the avoidance space 39 of the base 3, the snap-fitting hook 5011 of the fourth elastic arm 501 is no longer blocked by the baffle plate 38, and a speed at which the puncture needle 242 moves in the implantation direction decreases. When the physiological sign detection apparatus 100 is in the needle withdrawal process, the second elastic member 7 pushes the needle extraction base 6 to a side away from the organism 30, the snap-fitting hook 5011 of the fourth elastic arm 501 retracts to the avoidance space 39 (as shown by an arrow in FIG. 36), the needle extraction base 6 is detached from the snap-fitting hook 5011 of the fourth elastic arm 501, the needle extraction base 6 drives the needle base 241 and the puncture needle 242 to move toward the side away from the organism 30, and the puncture needle 242 is rapidly detached from the organism 30. The second elastic member 7 may push the needle extraction base 6 to move by releasing elastic potential energy. The first sealing member 243 is fastened to the needle base 241, and is detached from the sensor assembly 20 with the needle base 241. The implantation portion 2443 of the sensor 244 is reserved on an inner side of the organism 30, the lower cover 26 of the sensor assembly 20 is stuck on the organism 30 by using the bonding member 27, the implantation apparatus 10 is removed, the sensor assembly 20 is detached from the fifth elastic arm 504 of the implantation base 5 (referring to FIG. 35), and the sensor assembly 20 is reserved on the surface of the organism 30 to detect a physiological target substance such as blood glucose. In some possible embodiments, total time required in the entire implantation process of the physiological sign detection apparatus 100 is at a millisecond level, and the user almost feels no pain or has a very weak pain feeling.

In this embodiment, when the sensor 244 is implanted into the organism 30, the avoidance space 39 of the base 3 provides retraction space for the snap-fitting hook 5011 of the fourth elastic arm 501. In addition, an elastic force of the second elastic member 7 enables the needle extraction base 6 to push away and be detached from the fourth elastic arm 501, so that the needle extraction base 6 moves toward the reverse direction of the implantation direction, and drives the puncture needle 242 to be detached from the organism 30. A needle extraction force of the needle extraction base 6 is provided only by the second elastic member 7, so that the physiological sign detection apparatus 100 can automatically and quickly retract a needle, to reduce user operations, and help improve use experience of the user.

In some embodiments, a shock absorption pad (not shown in FIG. 37) may be further disposed between the needle extraction base 6 and the stopper member 4. The shock absorption pad may be made of a vinyl-vinyl acetate copolymer (Ethylene Vinyl Acetate copolymer, EVA), polyvinyl (polyethylene, PE) foam, chloroprene rubber, polyurethane (Polyurethane, PU) foam, or the like, to reduce a problem that the user is afraid due to too large noise caused by a collision of the stopper member 4 in a needle extraction process of the needle extraction base 6.

A procedure of using the physiological sign detection apparatus 100 is as follows: First, the to-be-implanted portion 2443 of the organism 30 is wiped with alcohol. After the alcohol is dry, the housing cap 8 of the physiological sign detection apparatus 100 is removed. Then, the physiological sign detection apparatus 100 is placed in the to-be-implanted portion 2443 of the organism 30, so that the base 3 abuts against the organism 30, and the housing 1 is pushed to move toward the organism 30. In this way, the sensor 244 is implanted into the organism 30 under guidance of the puncture needle 242, the sensor assembly 20 is stuck on the surface of the organism 30, and the implantation apparatus 10 is withdrawn. Implantation is completed.

In this embodiment of this application, in the physiological sign detection apparatus 100, a miniaturization design of the sensor assembly 20 and a compact arrangement of all components of an implanter enable a small overall volume of the physiological sign detection apparatus 100, to facilitate miniaturization. In some embodiments, a diameter of the physiological sign detection apparatus 100 may be less than 40 millimeters, and a height may be less than 70 millimeters. For example, the diameter of the physiological sign detection apparatus 100 may be 39 millimeters, 38.6 millimeters, 37 millimeters, 36.3 millimeters, 35 millimeters, 33 millimeters, or 32.7 millimeters. A height of the physiological sign detection apparatus 100 may be 69 millimeters, 68.6 millimeters, 68 millimeters, 67.3 millimeters, 65 millimeters, 64 millimeters, 63 millimeters, or the like.

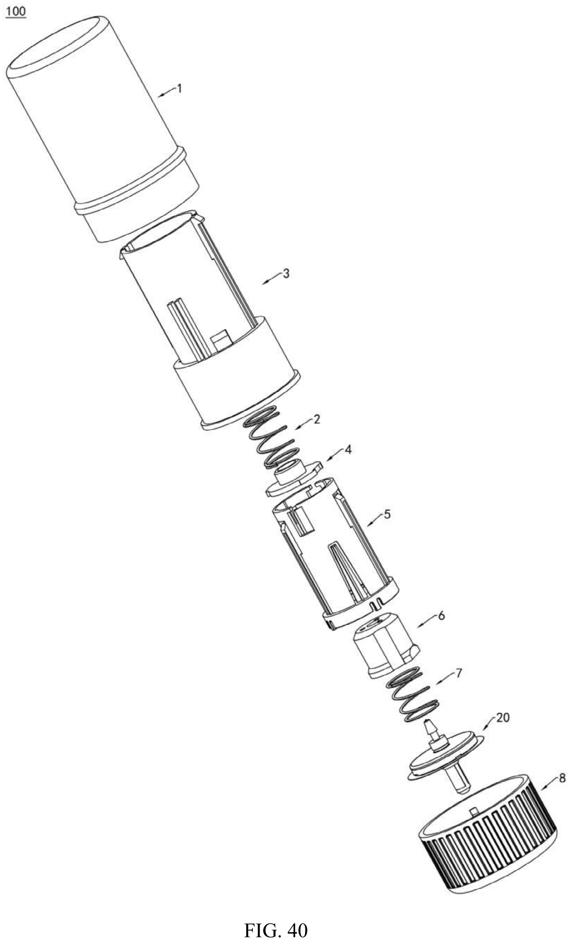

FIG. 39 is a diagram of a structure of the physiological sign detection apparatus 100 shown in FIG. 1 in some other embodiments. FIG. 40 is a diagram of a partial exploded structure of the physiological sign detection apparatus 100 shown in FIG. 39. This embodiment may include most of the technical solutions in the foregoing embodiments. The following mainly describes differences between this embodiment and the foregoing embodiments, and most same content of this embodiment and the foregoing embodiments is not described again.

In some embodiments, an implantation apparatus 10 of the physiological sign detection apparatus 100 may include the housing 1, a first elastic member 2, a base 3, a stopper member 4, an implantation base 5, a needle extraction base 6, a second elastic member 7, and a housing cap 8. The physiological sign detection apparatus 100 may further include a sensor assembly 20. The first elastic member 2, the stopper member 4, the needle extraction base 6, the second elastic member 7, the sensor assembly 20, and the housing cap 8 may be disposed with reference to the foregoing embodiments. Details are not described again below. A main difference between this embodiment and the foregoing embodiments lies in that the housing 1 and the base 3 are different.

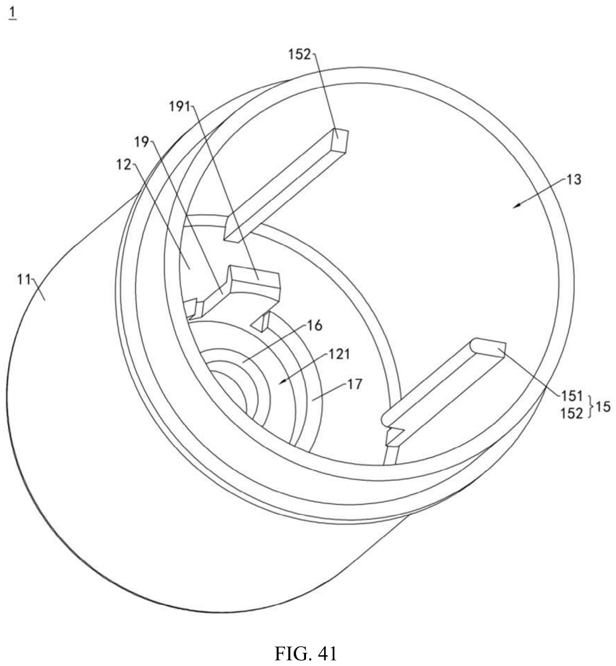

FIG. 41 is a diagram of a structure of the housing 1 shown in FIG. 40 from another perspective. In some embodiments, the housing 1 may include a side wall 11, a top wall 12, an opening 13, a first guiding structure 15, a first limiting boss 16, a second limiting boss 17, and a limiting groove 121. The first guiding structure 15 may include a first sliding block 151 and a second sliding block 152. In this embodiment, the side wall 11, the top wall, the first guiding structure 15, the first limiting boss 16, the second limiting boss 17, the limiting groove 121, the first sliding block 151, and the second sliding block 152 of the housing 1 are disposed. A material of the housing 1 may be set with reference to the foregoing embodiments. Details are not described below. The housing 1 may further include a second cantilever 19, and the second cantilever 19 is fastened to a side that is of the top wall 12 of the housing 1 and that faces the opening 13. There may be one or more second cantilevers 19. When there are a plurality of second cantilevers 19, the plurality of second cantilevers 19 are spaced apart in a circumferential direction of the second limiting boss 17. A tail end of the second cantilever 19 is provided with a snap-fitting hook 191, and snap-fitting hooks 191 of the plurality of second cantilever 19 are disposed facing away from each other.

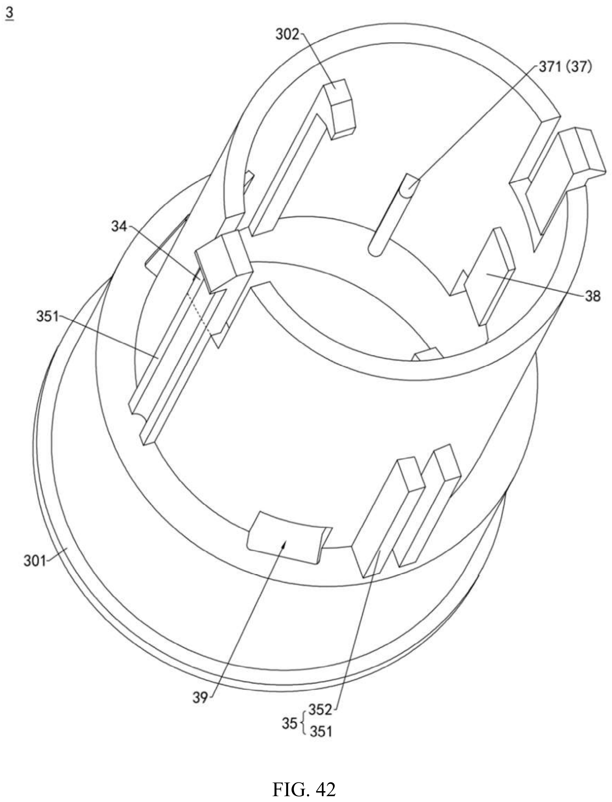

FIG. 42 is a diagram of a structure of the base 3 shown in FIG. 40 from another perspective.

In some embodiments, the base 3 may include an upper side wall 31, a lower side wall 32, a connecting wall 33, abutting end 301, a first elastic arm 34, a second guiding structure 35, a third guiding structure 37, a baffle plate 38, and avoidance space 39. The second guiding structure 35 may include a first guiding arm 351 and a second guiding arm 352, and the third guiding structure 37 may include a third sliding block 371. In this embodiment, the upper side wall 31, the lower side wall 32, the connecting wall 33, the abutting end 301, the first elastic arm 34, the second guiding structure 35, the third guiding structure 37, the baffle plate 38, the avoidance space 39, the first guiding arm 351, the second guiding arm 352, and the third sliding block 371 of the base 3 may be disposed with reference to the foregoing embodiments. Details are not described again below. The base 3 may further include a push block 302, and the push block 302 is convexly disposed on an inner side surface of the base 3.

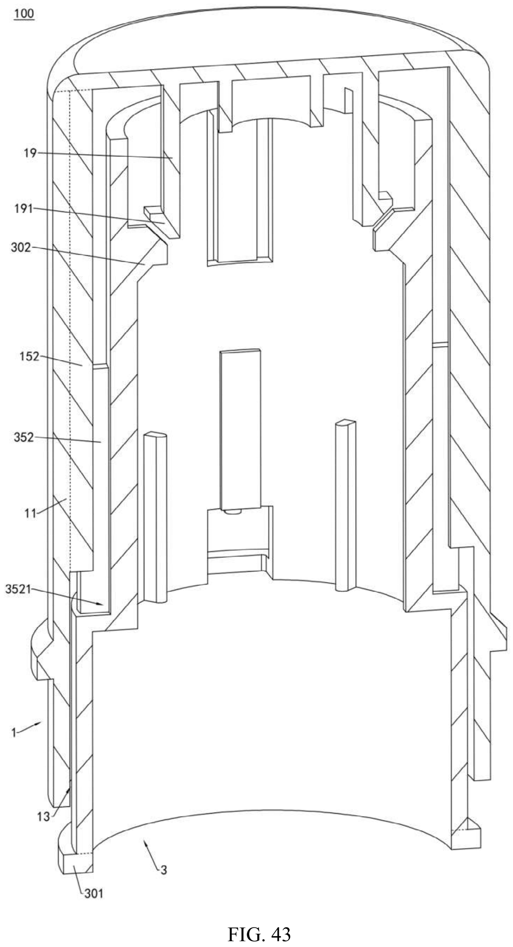

FIG. 43 is a diagram 1 of a partial cross-sectional structure after the physiological sign detection apparatus 100 shown in FIG. 39 is cut along G-G.

In some embodiments, the base 3 is mounted on an inner side of the housing 1 and is slidably connected to the side wall 11 of the housing 1, the abutting end 301 of the base 3 protrudes from the housing 1 through the opening 13 of the housing 1, and an axial direction of the base 3 coincides with an axial direction of the housing 1. The push block 302 is disposed opposite to the snap-fitting hook 191 of the second cantilever 19 in the axial direction. A quantity of push blocks 302 matches a quantity of second cantilevers 19. For example, there are two push blocks 302, and the two push blocks 302 are disposed opposite to each other. The second sliding block 152 is mounted in a sliding groove 3521 of the second guiding arm 352, and the second sliding block 152 can slide in a sliding groove 3521 of the second guiding arm 352. In this embodiment, the second guiding arm 352 cooperates with the second sliding block 152, so that the housing 1 and the base 3 limit each other in a circumferential direction, and the base 3 is prevented from rotating relative to the housing 1 in the circumferential direction. In addition, the second sliding block 152 can slide in the sliding groove 3521 of the second guiding arm 352, so that the housing 1 can move relative to the base 3 in the axial direction.

In some other embodiments, the second sliding block 152 may alternatively be fastened to an outer side surface of the base 3, and the second guiding arm 352 may alternatively be fastened to an inner side surface of the side wall 11 of the housing 1. The housing 1 and the base 3 can limit each other in the circumferential direction through mutual cooperation between the second sliding block 152 and the second guiding arm 352. This is not strictly limited in this application.

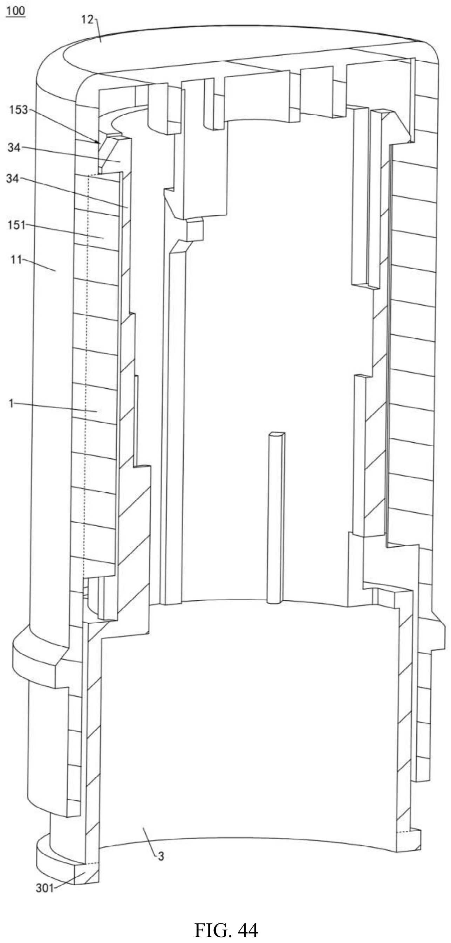

Refer to FIG. 42 and FIG. 44 together. FIG. 44 is a diagram 1 of a partial cross-sectional structure after the physiological sign detection apparatus 100 shown in FIG. 39 is cut along H-H.

In some embodiments, a hook 341 of the first elastic arm 34 of the base 3 may be snap-fitted into the first sliding block 151 of the housing 1, and the hook 341 of the first elastic arm 34 is mounted in limiting space 153 between the first sliding block 151 and the top wall 12 of the housing 1. The first sliding block 151 abuts against the hook 341 of the first elastic arm 34, to limit movement of the housing 1 in a direction away from the abutting end 301 relative to the base 3. In this embodiment, the hook 341 of the first elastic arm 34 limits the first sliding block 151 in the axial direction, to prevent the housing 1 from moving in the direction away from the abutting end 301 relative to the base 3, and prevent the housing 1 from being detached from the base.

In some other embodiments, the side wall 11 of the housing 1 may also have snap-fitting space or a snap-fitting block, and the first elastic arm 34 can also be snap-fitted into the housing 1 by snap-fitting the hook 341 of the first elastic arm 34 into the snap-fitting space or the snap-fitting block of the housing 1.

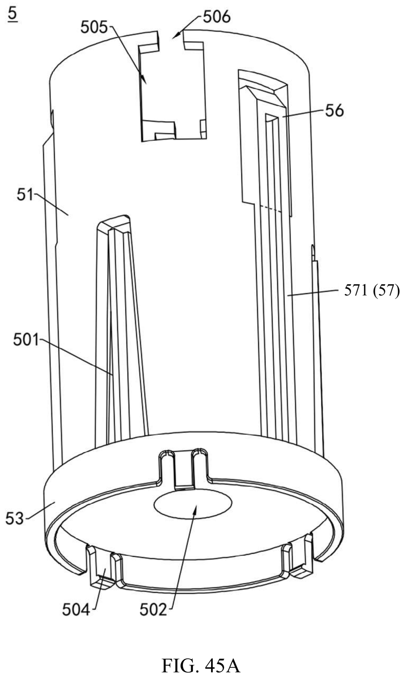

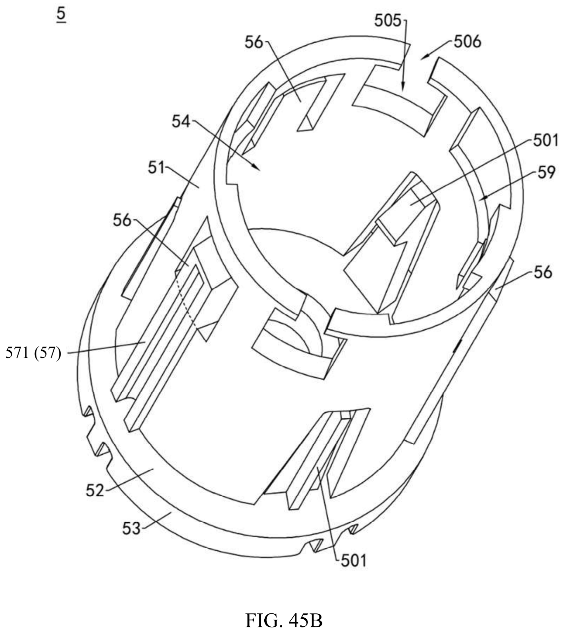

FIG. 45A is a diagram of a structure of the implantation base 5 shown in FIG. 40. FIG. 45B is a diagram of a structure of the implantation base 5 shown in FIG. 45A from another perspective.

In some embodiments, the implantation base 5 may include a side plate 51, a bottom plate 52, a lower side plate 53, first accommodation space 54, a third elastic arm 56, a fourth guiding structure 57, a fourth elastic arm 501, a mounting groove 59, and a fifth elastic arm 504. The fourth guiding structure 57 may include a third guiding arm 571. In this embodiment, the side plate 51, the bottom plate 52, the lower side plate 53, the first accommodation space 54, the third elastic arm 56, the fourth guiding structure 57, the third guiding arm 571, the fourth elastic arm 501, the mounting groove 59, and the fifth elastic arm 504 of the implantation base 5 may be disposed with reference to the foregoing embodiments. Details are not described again below. The implantation base 5 has a snap-fitting hole 505 and a notch 506. The snap-fitting hole 505 is located at an end that is of the side plate 51 of the implantation base 5 and that faces away from the lower side plate 53 of the implantation base 5, and the notch 506 is located at a side that is of the snap-fitting hole 505 and that faces away from the lower side plate 53 of the implantation base 5, and communicates with the snap-fitting hole 505.

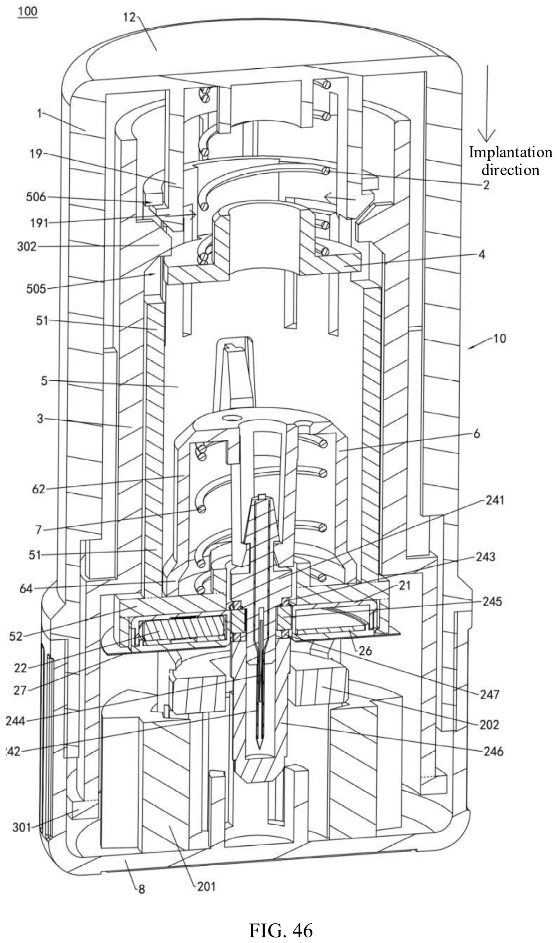

FIG. 46 is a diagram 2 of a partial cross-sectional structure after the physiological sign detection apparatus 100 shown in FIG. 39 is cut along G-G.

In some embodiments, the base 3 is mounted on the inner side of the housing 1, and the axial direction of the base 3 coincides with the axial direction of the housing 1. The implantation base 5 is mounted on an inner side of the base 3, and the axial direction of the implantation base 5 coincides with the axial direction of the base 3. The snap-fitting hole 505 is located at an end that is of the side plate 51 of the implantation base 5 and that is close to the top wall 12 of the housing 1, and the notch 506 is located at a side that is of the snap-fitting hole 505 and that is close to the top wall 12 of the housing 1. At least a part of the snap-fitting hook 191 of the second cantilever 19 of the housing 1 is located in the snap-fitting hole 505 of the implantation base 5, so that the second cantilever 19 is snap-fitted into the implantation base 5. In this way, the base 3 limits the implantation base 5, to prevent the implantation base 5 from moving toward a side close to the abutting end 301, thereby causing a failure of the implantation apparatus 10 of the physiological sign detection apparatus 100. For example, there are two second cantilevers 19, and the two second cantilevers 19 are disposed opposite to each other, to ensure that the snap-fitted implantation base 5 is subject to a balanced force. At least a part of the push block 302 of the base 3 is snap-fitted into the snap-fitting hole 505, and a length of the push block 302 in a circumferential direction is less than a length of the notch 506 in the circumferential direction. Therefore, the push block 302 can be detached from the snap-fitting hole 505 from the notch 506. When the housing 1 moves in a direction close to the organism relative to the base 3 (in other words, the housing 1 moves in an implantation direction), the second cantilever 19 is squeezed by the push block 302, the second cantilever 19 moves toward an inner side of the side plate 51 of the implantation base 5 (as shown by an arrow in FIG. 46), the snap-fitting hook 191 of the second cantilever 19 is detached from the snap-fitting hole 505, and the implantation base 5 is free from a limit of the base 3. Under an elastic force of the first elastic member 2, the implantation base 5 can move in the direction close to the organism relative to the base 3, and the push block 302 is detached from the snap-fitting hole 505 from the notch 506.

In this embodiment, a limit of the base 3 on the implantation base 5 is removed through cooperation between the push block 302 and the second cantilever 19, so that the implantation base 5 is detached from the base 3 under the elastic force of the first elastic member 2. When the housing 1 moves in the implantation direction, the push block 302 gradually pushes the second cantilever 19 to move toward the inner side of the side plate 51 of the implantation base 5, and compresses the first elastic member 2. When the snap-fitting hook 191 of the second cantilever 19 is detached from the snap-fitting hole 505, the implantation base 5 immediately moves in the implantation direction under the elastic force of the first elastic member 2. A limiting mechanism and a limit release mechanism of the implantation base 5 of the physiological sign detection apparatus 100 are simple and are arranged compactly, and a transmission speed between the housing 1 and the base 3 is fast.

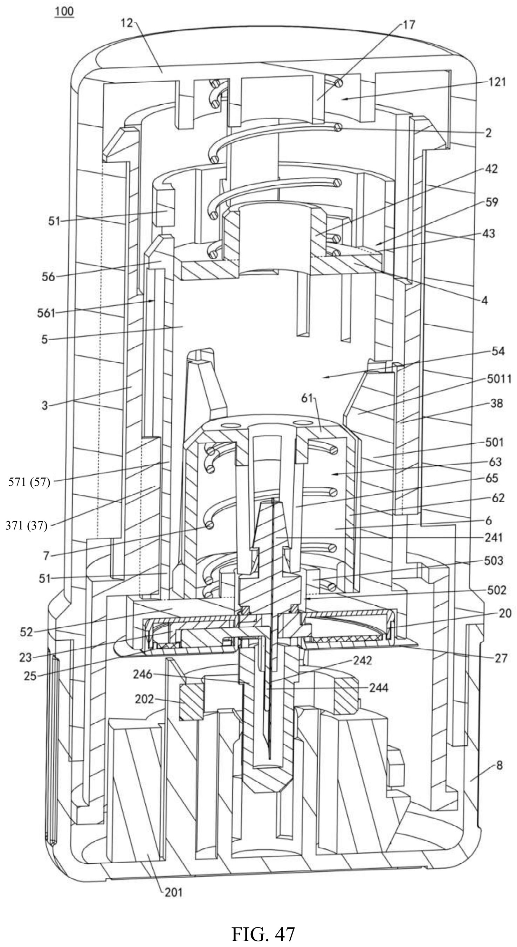

Refer to FIG. 45B and FIG. 47 together. FIG. 47 is a diagram 2 of a partial cross-sectional structure after the physiological sign detection apparatus 100 shown in FIG. 39 is cut along H-H.

In some embodiments, the implantation base 5 is slidably connected to the base 3, and the third guiding structure 37 of the base 3 is slidably connected to the fourth guiding structure 57 of the implantation base 5. For example, the third sliding block 371 of the base 3 is mounted in the sliding groove 561 of the third elastic arm 56 of the implantation base 5, and the third sliding block 371 can slide in the sliding groove 561 of the third elastic arm 56. There may be three third elastic arms 56 and three third guiding arms 571, to ensure that a connection between the implantation base 5 and the base 3 is subject to a balanced force, and maintain the implantation base 5 and the base 3 relatively stable in the radial direction. In some other embodiments, the third guiding structure 37 includes a sliding groove, the fourth guiding structure 57 includes a sliding block, and the third guiding structure 37 and the fourth guiding structure 57 are still slidably connected to each other. In this embodiment, the third sliding block 371 of the base 3 is slidably connected to the third elastic arm 56 of the implantation base 5 for mutual cooperation, so that the base 3 and the implantation base 5 limit each other in the circumferential direction, and the implantation base 5 is prevented from rotating relative to the base 3 in the circumferential direction. The third sliding block 371 can slide in the sliding groove 561 of the third elastic arm 56, so that the implantation base 5 can move in the axial direction relative to the base 3, and the third elastic arm 56 may further limit movement of the implantation base 5 in the axial direction.

The third elastic arm 56 is spaced apart from the side plate 51 of the implantation base 5 in both the circumferential direction and the axial direction, and the other end that is of the third elastic arm 56 and that faces away from the bottom plate 52 of the implantation base 5 is provided with the inclined end surface. When the implantation base 5 is assembled with the base 3, the third elastic arm 56 may elastically avoid the third sliding block 371 in the radial direction, and the inclined end surface of the third elastic arm 56 avoids the third sliding block 371 in the axial direction, so that the implantation base 5 is smoothly mounted on the inner side of the base 3, to reduce snap-fitting resistance, and further avoid damaging the implantation base 5 and the base 3 during assembly.

In some embodiments, the implantation base 5 may further include a mounting hole 502 and a fourth limiting boss 503, and the fourth limiting boss 503 is disposed around a circumferential edge of the mounting hole 502, and is fastened to a side that is of the bottom plate 52 of the implantation base 5 and that faces the first accommodation space 54. Both the needle extraction base 6 and the second elastic member 7 are mounted on the inner side of the implantation base 5. The second elastic member 7 is located between the implantation base 5 and the needle extraction base 6. Both an axial direction of the needle extraction base 6 and an axial direction of the second elastic member 7 may coincide with the axial direction of the implantation base 5. The second elastic member 7 is in a compressed state. For example, the needle extraction base 6 is mounted in the first accommodation space 54 of the implantation base 5, and the side plate 62 of the needle extraction base 6 is located on an outer side of the fourth limiting boss 503. The second elastic member 7 is mounted in the third accommodation space 63 of the needle extraction base 6. The second elastic member 7 may be but is not limited to a spring, an elastic rubber, or the like. The second elastic member 7 is located between the bottom plate 52 of the implantation base 5 and the top plate 61 of the needle extraction base 6, and is sleeved on a periphery of the first cantilever 65 and a periphery of the fourth limiting boss 503. The second elastic member 7 is configured to push the needle extraction base 6 away from the bottom plate 52 of the implantation base 5. The first cantilever 65 and the fourth limiting boss 503 limit and position the second elastic member 7, to prevent the second elastic member 7 from being skewed in a compression and rebounding process, thereby stabilize a position of the second elastic member 7, to improve reliability of the physiological sign detection apparatus 100.

In some embodiments, the needle extraction base 6 is mounted between the bottom plate 52 of the implantation base 5 and the snap-fitting hook 5011 of the fourth elastic arm 501. There may be three fourth elastic arms 501, so that the snap-fitted needle extraction base 6 is subject to a balanced force. In this embodiment, the needle extraction base 6 is located between the bottom plate 52 of the implantation base 5 and the snap-fitting hook 5011 of the fourth elastic arm 501, so that a stable position relationship is maintained between the needle extraction base 6 and the implantation base 5, and the fourth elastic arm 501 may push the needle extraction base 6 to move with the implantation base 5.

The fourth elastic arm 501 abuts against the baffle plate 38 on the inner side of the base 3, or a gap between the fourth elastic arm 501 and the baffle plate 38 is small. In this embodiment, when the physiological sign detection apparatus 100 is in an initial state, the snap-fitting hook 5011 of the fourth elastic arm 501 needs to well limit the needle extraction base 6, to prevent the needle extraction base 6 from moving toward a side that is away from the bottom plate 52 of the implantation base 5. The second elastic member 7 has a driving force for the needle extraction base 6, and the end that is of the fourth elastic arm 501 and that is provided with the snap-fitting hook is a free end. When driven by the needle extraction base 6, the fourth elastic arm 501 has a trend of moving toward a side of the upper side wall 31 of the base 3. The baffle plate 38 may block the fourth elastic arm 501, to avoid a failure of the physiological sign detection apparatus 100 because the needle extraction base 6 is detached from the implantation base 5 when the physiological sign detection apparatus 100 is in the initial state. This helps improve reliability of the physiological sign detection apparatus 100.

In some embodiments, the side plate 62 of the needle extraction base 6 is located between the fourth limiting boss 503 and the side plate 51 of the implantation base 5, the limiting flange 64 of the needle extraction base 6 is close to the side plate 51 of the implantation base 5, and a spacing between the limiting flange 64 and the side plate 51 of the implantation base 5 is small. In this embodiment, the limiting flange 64 is disposed on the side plate 62 of the needle extraction base 6, so that a gap between the side plate 62 of the needle extraction base 6 and the side plate 51 of the implantation base 5 is smaller, to help control of assembly precision between the needle extraction base 6 and the implantation base 5, reduce misalignment, and improve reliability and implantation accuracy of the physiological sign detection apparatus 100. In addition, the limiting flange 64 may further prevent the needle extraction base 6 from shaking greatly relative to the implantation base 5 in a packaging and transportation process of the physiological sign detection apparatus 100, so that the stable position relationship is maintained between the needle extraction base 6 and the implantation base 5.

In some embodiments, the first elastic member 2 is connected between the top wall 12 of the housing 1 and the implantation base 5, and an axial direction of the first elastic member 2 may coincide with the axial direction of the housing 1. The stopper member 4 is fastened to an end that is of the implantation base 5 and that faces away from the needle extraction base 6. For example, the stopping block 43 of the stopper member 4 is mounted in the mounting groove 59 of the implantation base 5. The plurality of stopping blocks 43 of the stopper member 4 are disposed in a one-to-one correspondence with the plurality of mounting grooves 59 of the implantation base 5, and the implantation base 5 can stably support the stopper member 4. The first elastic member 2 is mounted between the stopper member 4 and the top wall 12 of the housing 1. The first elastic member 2 may be but is not limited to a spring, an elastic rubber, or the like. An end of the first elastic member 2 is located in the limiting groove 121 of the top wall 12 of the housing 1, and the other end of the first elastic member 2 is sleeved on a periphery of the third limiting boss 42 of the stopper member 4. The first limiting boss 16 and the second limiting boss 17 of the housing 1 and the third limiting boss 42 of the stopper member 4 all limit and position the first elastic member 2, to prevent the first elastic member 2 from being skewed in a compression and rebounding process, thereby stabilizing a position of the first elastic member 2, to improve reliability of the physiological sign detection apparatus 100. The first elastic member 2 may be in a compressed state, to form pre-pressure on the stopper member 4, so that a position of the stopper member 4 relative to the base 3 is stable. Alternatively, the first elastic member 2 may be in a natural state. This is not strictly limited in this embodiment of this application.

Refer to FIG. 45A, FIG. 46, and FIG. 47 together. In some embodiments, the sensor assembly 20 may include an upper cover 21, a sensor sealing assembly 24, a battery 22, a connector 23, a circuit board 25, a lower cover 26, and a bonding member 27. The sensor sealing assembly 24 may include a needle base 241, a first sealing member 243, a sensor 244, a sensor base 245, a puncture needle 242, a second sealing member 247, and a sealing bottle 246. In this embodiment, the upper cover 21, the battery 22, the connector 23, the circuit board 25, the lower cover 26, the bonding member 27, the needle base 241, the first sealing member 243, the sensor 244, the sensor base 245, the puncture needle 242, the second sealing member 247, and the sealing bottle 246 of the sensor assembly 20 may be disposed with reference to the foregoing embodiments. For materials of all components of the sensor assembly 20, an assembly relationship of the components, and an assembly relationship of the sensor assembly 20, the implantation base 5, and the needle extraction base 6, refer to the foregoing embodiments. Details are not described again in this application.

The physiological sign detection apparatus 100 may further include a housing cap 8, a sealing member (not shown in FIG. 46), a drying member 201, and a magnetic element 202. In this embodiment, the housing cap 8, the sealing member, the drying member 201, and the magnetic element 202 may be disposed with reference to the foregoing embodiments. The housing cap 8 is hermetically connected to the housing 1 by using the sealing member. Materials and the assembly relationship of the housing cap 8, the drying member 201, and the magnetic element 202, and the assembly relationship of the housing cap 8, the housing 1, and the sealing bottle 246 may be set with reference to the foregoing embodiments. Details are not described again in this application.

A sterilization manner of the physiological sign detection apparatus 100 in this embodiment may be set with reference to the foregoing embodiments. Details are not described herein again.

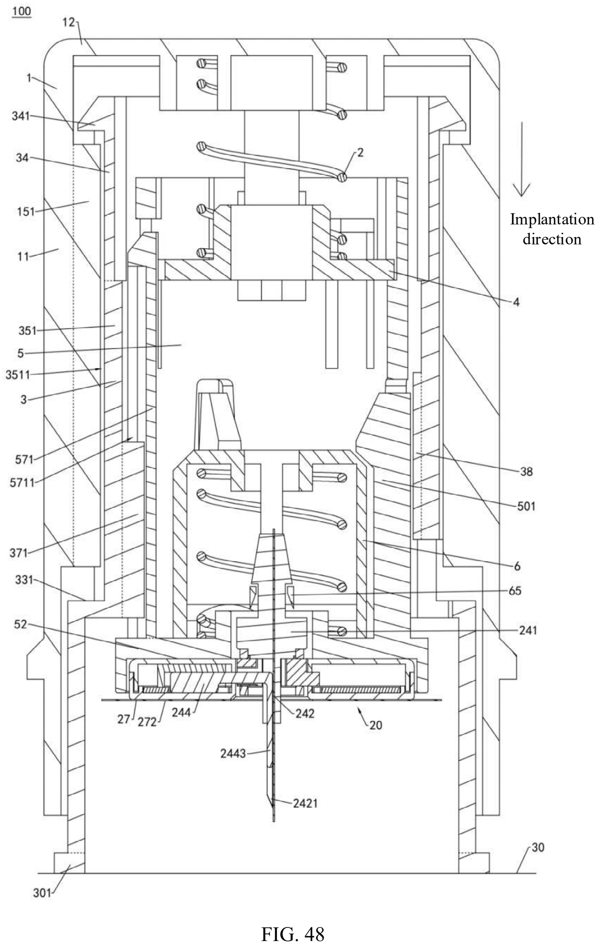

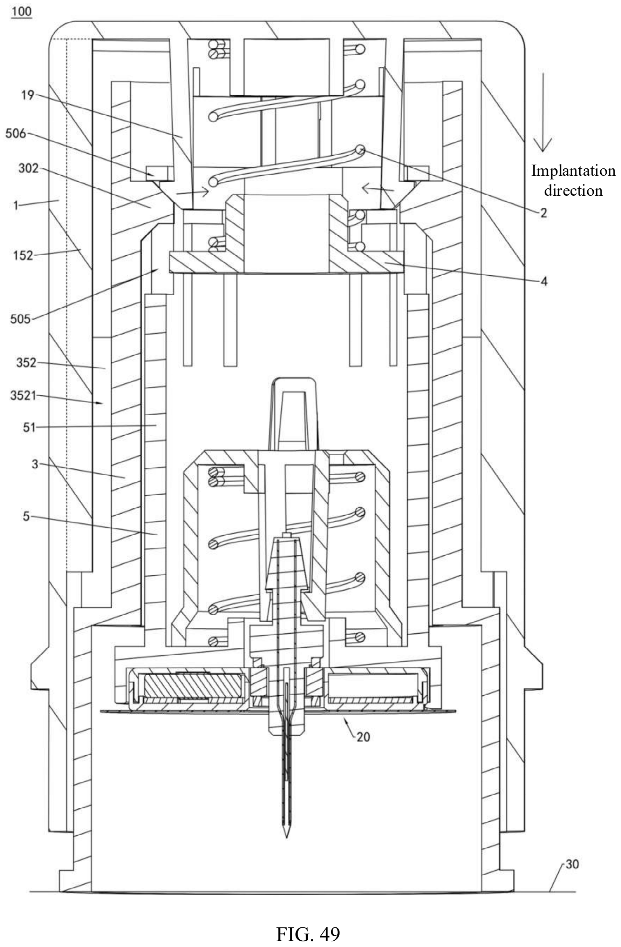

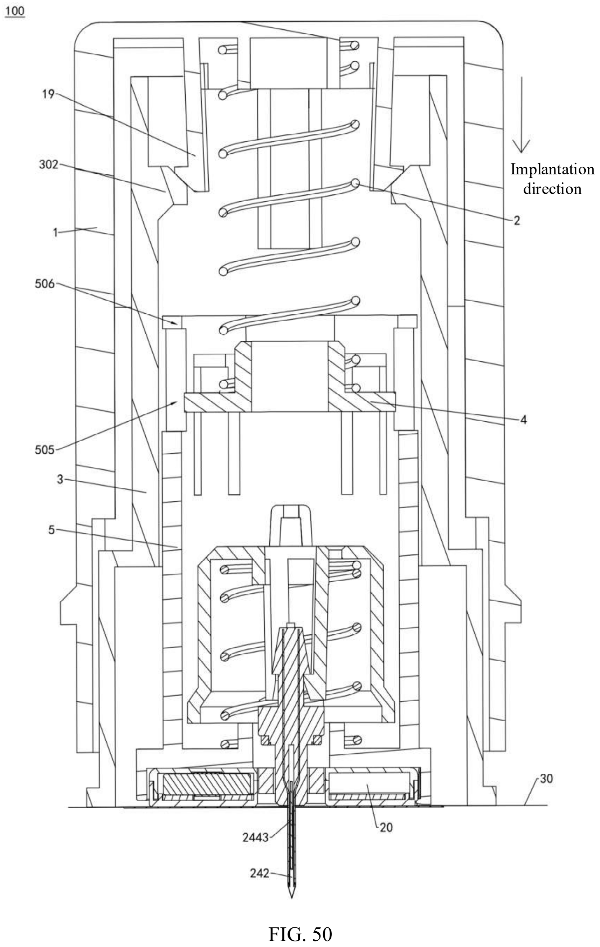

FIG. 48 is a diagram of a partial cross-sectional structure after the physiological sign detection apparatus 100 shown in FIG. 39 is cut along H-H in a pressing process. FIG. 49 is a diagram of a partial cross-sectional structure after the physiological sign detection apparatus 100 shown in FIG. 39 is cut along G-G in a pressing process. FIG. 50 is a diagram of a partial cross-sectional structure after the physiological sign detection apparatus 100 shown in FIG. 39 is cut along G-G in a puncturing process.

In some embodiments, the physiological sign detection apparatus 100 has a pressing process, a puncturing process, and a needle withdrawal process in an implantation process. In the implantation process of the physiological sign detection apparatus 100, the housing cap 8 is detached from the housing 1, and the abutting end 301 of the base 3 abuts against the organism 30, and maintains a position unchanged relative to the organism 30. The abutting end 301 may be configured to increase a contact area of abutting against skin of the organism 30 by the base 3, to reduce pressure, thereby helping reduce a pain feeling when the base 3 abuts against the skin of the organism 30, and improving user experience. When the physiological sign detection apparatus 100 is in the pressing process, and the housing 1 moves in a direction close to the organism 30 relative to the base 3, that is, when the housing 1 moves in the implantation direction, the first sliding block 151 is detached from the hook 341 of the first elastic arm 34, and moves in a direction close to the organism 30 along the sliding groove 3511 of the first guiding arm 351. The second sliding block 152 moves in the direction close to the organism 30 along the sliding groove 3521 of the second guiding arm 352. The top wall 12 of the housing 1 compresses the first elastic member 2 in the implantation direction, and the first elastic member 2 pushes the stopper member 4 to move in the implantation direction, to push the implantation base 5 toward the organism 30. The third sliding block 371 is mounted in the sliding groove 5711 of the third guiding arm 571, and the third guiding arm 571 moves in the implantation direction relative to the third sliding block 371. The first elastic member 2 may push the stopper member 4 to move by releasing elastic potential energy. The step surface 331 and the hook 341 of the first elastic arm 34 limit movement of the first sliding block 151 in the axial direction, to help control implantation strength and an implantation depth of the physiological sign detection apparatus 100.

When the housing 1 moves in the direction close to the organism 30 relative to the base 3, the second cantilever 19 moves in the implantation direction relative to the push block 302, and the second cantilever 19 is squeezed by the push block 302, so that an end that is of the second cantilever 19 and that is provided with the snap-fitting hook 191 moves toward the inner side of the side plate 51 of the implantation base 5 (as shown by an arrow in FIG. 49), and the snap-fitting hook 191 of the second cantilever 19 is detached from the snap-fitting hole 505 of the implantation base 5. The implantation base 5 is free from a limit of the second cantilever 19 of the base 3, and moves in the direction close to the organism 30 relative to the base 3 under an action of the elastic force of the first elastic member 2, and the push block 302 is detached from the snap-fitting hole 505 from the notch 506.

The bottom plate 52 of the implantation base 5 pushes the sensor assembly 20 to get close to the organism 30. The baffle plate 38 of the base 3 abuts against the fourth elastic arm 501 of the implantation base 5, and limits the fourth elastic arm 501 in the radial direction, to prevent the needle extraction base 6 from pushing away the snap-fitting hook 5011 of the fourth elastic arm 501 under an action of the second elastic member 7. When the implantation base 5 moves in the direction close to the organism 30, the fourth elastic arm 501 of the implantation base 5 pushes the needle extraction base 6 to move synchronously in the implantation direction with the implantation base 5. The first cantilever 65 of the needle extraction base 6 pushes the needle base 241 to move synchronously in the implantation direction with the implantation base 5. The implantation base 5 pushes the needle base 241 to the organism 30 by using the needle extraction base 6, so that the needle base 241 and the sensor 244 move synchronously in the direction close to the organism 30. The implantation base 5 pushes the sensor assembly 20 to move in the implantation direction until the sensor assembly 20 abuts against the organism 30, the puncture end 2421 of the puncture needle 242 and the implantation portion 2443 of the sensor 244 are punctured into the organism 30, and the second surface 272 of the bonding member 27 is stuck on the organism 30.

In the conventional technology, an implantation apparatus of a physiological sign detection apparatus starts an implantation process of a sensor by using an exposed button. In a process of product assembly, packaging, transportation, or the like, when a product drops or a user holds the physiological sign detection apparatus, the button is easily touched. Consequently, the implantation process is triggered incorrectly, and a sensor assembly cannot be ejected directly facing a detection position. Consequently, the sensor cannot be implanted into the detection position, and the physiological sign detection apparatus fails.

In this embodiment, the housing 1 is provided with no button. After the housing 1 and the housing cap 8 (as shown in FIG. 47) are assembled, the housing 1 and the housing cap 8 form a sealed cavity, to form good isolation and protection for an implantation portion 2443 and the sensor assembly 20 on an inner side. When the housing cap 8 is not detached from the housing 1, the implantation apparatus 10 does not trigger an implantation action, and the sensor assembly 20 is not ejected. In addition, the sensor assembly 20 is ejected only when the user holds the base 3 against the organism 30 and pushes the housing 1 toward the organism 30. Therefore, a risk of false triggering of the physiological sign detection apparatus 100 is low, and reliability is higher. In addition, in the implantation process, a movement direction of the housing 1 is consistent with a movement direction of the puncture needle 242, an implantation direction of the sensor assembly 20 is easy to control, an implantation force of the sensor assembly 20 is controlled by the first elastic member 2, and a magnitude of the implantation force is unchanged and is not affected by another external force. The physiological sign detection apparatus 100 has a moderate implantation force and a fast implantation speed, to help reduce a pain feeling of the user.

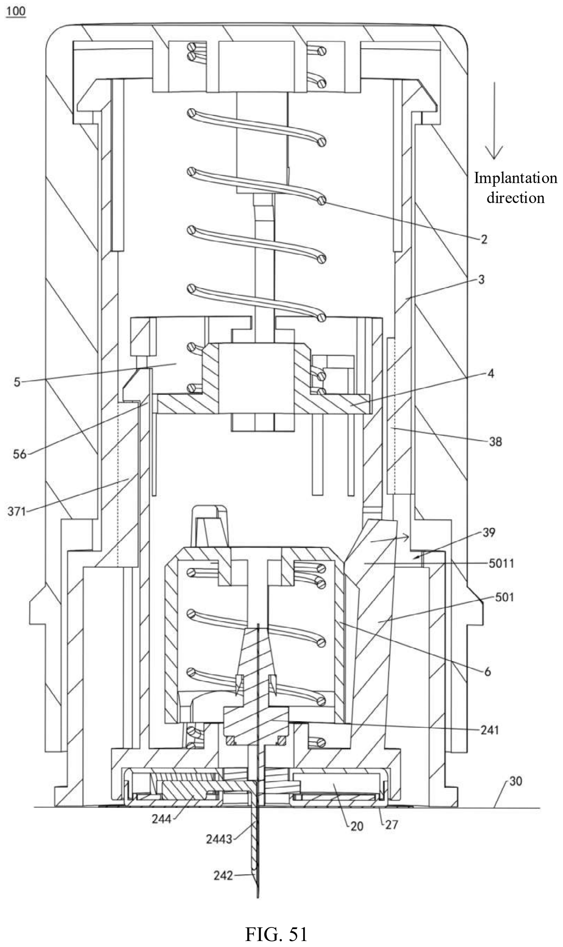

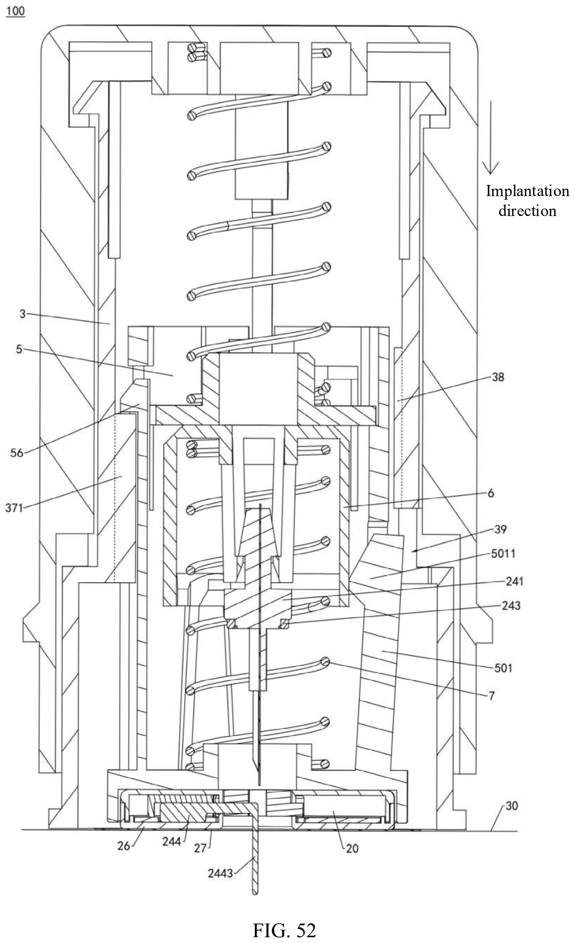

FIG. 51 is a diagram of a partial cross-sectional structure after the physiological sign detection apparatus 100 shown in FIG. 39 is cut along H-H in a puncturing process. FIG. 52 is a diagram of a partial cross-sectional structure after the physiological sign detection apparatus 100 shown in FIG. 39 is cut along H-H in a needle extraction state.

In some embodiments, when the housing 1 moves in a direction close to the organism 30 relative to the base 3, the first elastic member 2 is compressed. Under an action force of the first elastic member 2, the stopper member 4 pushes the implantation base 5 to move in the direction close to the organism 30. The first elastic member 2 may push the stopper member 4 to move by releasing elastic potential energy. The third elastic arm 56 of the implantation base 5 moves in the implantation direction relative to the third sliding block 371, the implantation base 5 pushes the sensor assembly 20 to move in the implantation direction, and the puncture needle 242 is punctured into the organism 30. When the snap-fitting hook of the third elastic arm 56 abuts against the third sliding block 371, motion of the implantation base 5 relative to the base 3 is limited. In this case, the bonding member 27 of the sensor assembly 20 reaches a surface of the organism 30, and a depth of puncturing the puncture needle 242 and the implantation portion 2443 of the sensor 244 into the organism 30 is just proper. In this embodiment, the snap-fitting hook of the third elastic arm 56 cooperates with the third sliding block 371, to limit a movement distance of the implantation base 5 in the implantation direction to a proper range, to avoid excessive impact on the organism 30 caused by free movement of the implantation base 5 under an action of the first elastic member 2, thereby causing a pain to the user.

In some embodiments, when the physiological sign detection apparatus 100 is in the puncturing process, and the sensor 244 is implanted into the organism 30 under guidance of the puncture needle 242, a speed at which the puncture needle 242 moves in the implantation direction decreases, the snap-fitting hook 5011 of the fourth elastic arm 501 reaches the avoidance space 39 of the base 3, and the snap-fitting hook 5011 of the fourth elastic arm 501 is no longer blocked by the baffle plate 38. When the physiological sign detection apparatus 100 is in the needle withdrawal process, the second elastic member 7 pushes the needle extraction base 6 to a side away from the organism 30, the snap-fitting hook 5011 of the fourth elastic arm 501 retracts to the avoidance space 39 (as shown by an arrow in FIG. 36), the needle extraction base 6 is detached from the snap-fitting hook 5011 of the fourth elastic arm 501, the needle extraction base 6 drives the needle base 241 and the puncture needle 242 to move toward the side away from the organism 30, and the puncture needle 242 is rapidly detached from the organism 30. The second elastic member 7 may push the needle extraction base 6 to move by releasing elastic potential energy. The first sealing member 243 is fastened to the needle base 241, and is detached from the sensor assembly 20 with the needle base 241. The implantation portion 2443 of the sensor 244 is reserved on an inner side of the organism 30, the lower cover 26 of the sensor assembly 20 is stuck on the organism 30 by using the bonding member 27, the implantation apparatus 10 is removed, the sensor assembly 20 is detached from the fifth elastic arm 504 of the implantation base 5 (referring to FIG. 35), and the sensor assembly 20 is reserved on the surface of the organism 30 to detect a physiological target substance such as blood glucose. In some possible embodiments, total time required in the entire implantation process of the physiological sign detection apparatus 100 is at a millisecond level, and the user almost feels no pain or has a very weak pain feeling.

In this embodiment, when the sensor 244 is implanted into the organism 30, the avoidance space 39 of the base 3 provides retraction space for the snap-fitting hook 5011 of the fourth elastic arm 501. In addition, an elastic force of the second elastic member 7 enables the needle extraction base 6 to push away and be detached from the fourth elastic arm 501, so that the needle extraction base 6 moves toward the reverse direction of the implantation direction, and drives the puncture needle 242 to be detached from the organism 30. A needle extraction force of the needle extraction base 6 is provided only by the second elastic member 7, so that the physiological sign detection apparatus 100 can automatically and quickly retract a needle, to reduce user operations, and help improve use experience of the user.

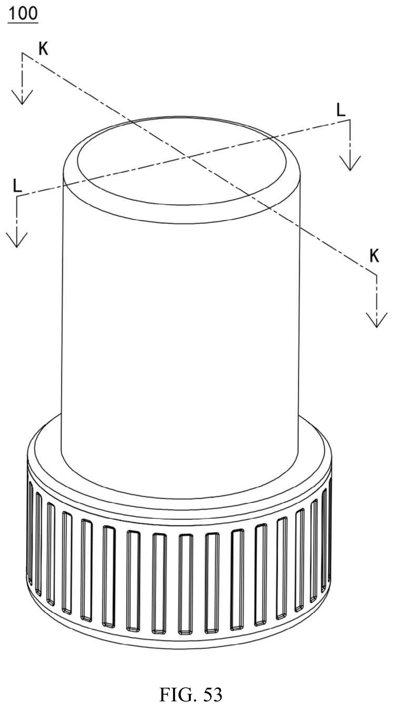

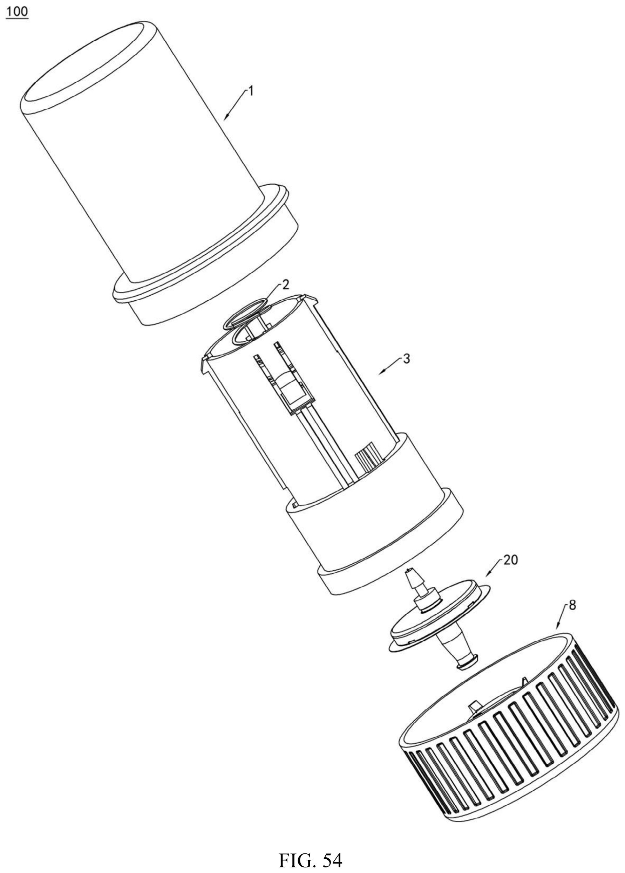

FIG. 53 is a diagram of a structure of the physiological sign detection apparatus 100 shown in FIG. 1 in some other embodiments. FIG. 54 is a diagram of a partial exploded structure of the physiological sign detection apparatus 100 shown in FIG. 53. This embodiment may include most of the technical solutions in the foregoing embodiments. The following mainly describes differences between this embodiment and the foregoing embodiments, and most same content of this embodiment and the foregoing embodiments is not described again.

In some embodiments, the physiological sign detection apparatus 100 may include a housing 1, a base 3, a first elastic member 2, a stopper member 4, an implantation base 5, a needle extraction base 6, a second elastic member 7 (not shown in FIG. 54), a sensor assembly 20, and a housing cap 8. In this embodiment, the housing 1, the first elastic member 2, the base 3, the stopper member 4, the implantation base 5, the needle extraction base 6, and the second elastic member 7 of the physiological sign detection apparatus 100 are disposed with reference to the foregoing embodiments. Details are not described again below. A main difference between this embodiment and the foregoing embodiments lies in that the housing cap 8 and the sensor assembly 20 have different partial structures.

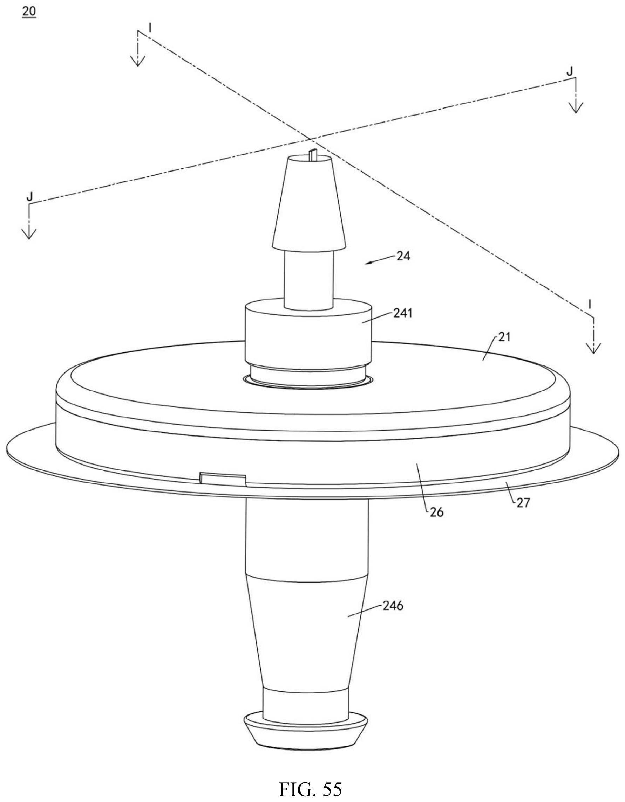

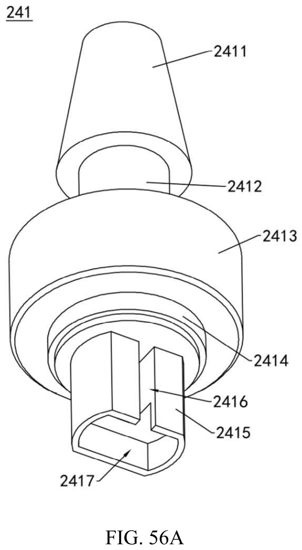

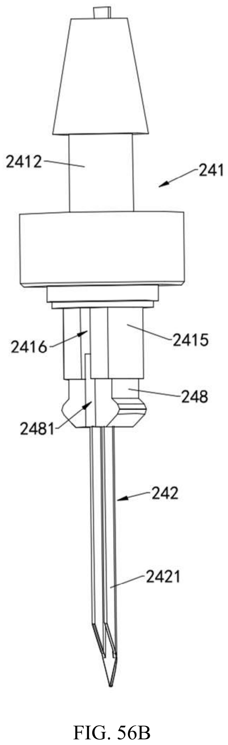

FIG. 55 is a diagram of a structure of the sensor assembly 20 shown in FIG. 54 in some embodiments. FIG. 56A is a diagram of a structure of a needle base 241 shown in FIG. 55. FIG. 56B is a diagram of an assembly structure of a snap-fit 248 and a needle base 241 in some embodiments.

In some embodiments, the sensor assembly 20 may include an upper cover 21, a sensor sealing assembly 24, a battery 22, a connector 23, a circuit board 25, a lower cover 26, and a bonding member 27. The sensor sealing assembly 24 may include a needle base 241, a puncture needle 242, a first sealing member 243, a sensor 244, a sensor base 245, a second sealing member 247, and a sealing bottle 246. The battery 22, the connector 23, the circuit board 25, the puncture needle 242, the sensor 244, the sensor base 245, and the second sealing member 247 are not shown in FIG. 55 to FIG. 56B. In this embodiment, the upper cover 21, the battery 22, the connector 23, the circuit board 25, the lower cover 26, the bonding member 27, the first sealing member 243, the sensor 244, and the sensor base 245 of the sensor assembly 20 may be disposed with reference to the foregoing embodiments. Details are not described again below. A main difference between this embodiment and the foregoing embodiments lies in that the needle base 241 and a sealing bottle 246 are different.

In some embodiments, the needle base 241 may include a limiting snap-fit 2411, a snap-fitting portion 2412, a limiting portion 2413, a sealing portion 2414, and a guiding portion 2415. The limiting snap-fit 2411, the snap-fitting portion 2412, the limiting portion 2413, the sealing portion 2414, and the guiding portion 2415 are sequentially connected. The limiting snap-fit 2411 may be generally in a shape of a circular truncated cone, the snap-fitting portion 2412 and the limiting portion 2413 may be generally in a shape of a cylinder, and a diameter of the snap-fitting portion 2412 is less than a diameter of the limiting snap-fit 2411 and a diameter of the limiting portion 2413. The guiding portion 2415 has a recessed groove 2416, and the recessed groove 2416 extends in an axial direction of the needle base 241. The guiding portion 2415 further has a positioning groove 2417, and an opening of the positioning groove 2417 faces a side that faces away from the sealing portion 2414. The sensor sealing assembly 24 may further include a snap-fit 248, an end of the snap-fit 248 is mounted in the positioning groove 2417 of the guiding portion 2415, and a flange is disposed at the other end that is of the snap-fit 248 and that faces away from the guiding portion 2415. A side surface of the snap-fit 248 has a recessed groove 2481, and the recessed groove 2481 of the snap-fit 248 is connected to the recessed groove 2416 of the guiding portion 2415.

The puncture needle 242 is fastened to the needle base 241 and the snap-fit 248, and extends in an axial direction of the needle base 241. The puncture end 2421 of the puncture needle 242 protrudes from the snap-fit 248. A part that is of the puncture needle 242 and that is fastened to the guiding portion 2415 and the snap-fit 248 wraps the recessed groove 2416 of the guiding portion 2415 and the recessed groove 2481 of the snap-fit 248, and both shapes of the recessed groove 2416 of the guiding portion 2415 and the recessed groove 2481 of the snap-fit 248 match a shape of the puncture needle 242.

The needle base 241, the snap-fit 248, and the puncture needle 242 may be integrally formed. For example, the needle base 241 and the snap-fit 248 may be integrally formed by using a vulcanization process or a dual-shot injection molding process. The needle base 241 and the puncture needle 242 may be integrally formed by using the in-mold injection molding process. The needle base 241 may be made of engineering plastic such as polycarbonate or acrylonitrile butadiene styrene. The snap-fit 248 may be made of an elastic polymer material such as rubber, polyurethane, or thermoplastic polyurethane elastomer rubber (Thermoplastic polyurethanes, TPU). The puncture needle 242 may be made of a metal material such as 316 stainless steel, 304 stainless steel, or medical-grade stainless steel (for example, SUS316L).

A cross section of the puncture needle 242 may be generally of a U-shaped structure, and the puncture needle 242 is configured to puncture and push away tissue of the organism 30. The puncture end 2421 of the puncture needle 242 wraps an implantation portion 2443 (not shown in FIG. 56B) of the sensor 244. When the puncture needle 242 is punctured into the organism 30, implantation space may be formed, so that the implantation portion 2443 of the sensor 244 is punctured into the organism 30. In addition, the puncture end 2421 of the puncture needle 242 may further partially wrap the implantation portion 2443 of the sensor 244, and the implantation portion 2443 of the sensor 244 may also be punctured into the organism 30 with the puncture needle 242. This is not strictly limited in this application.

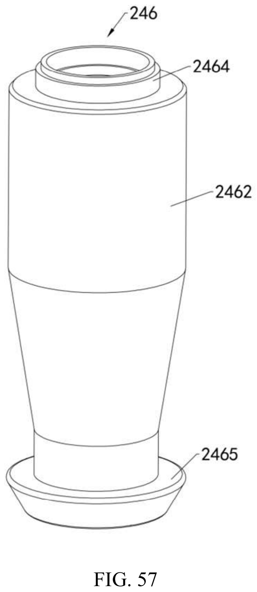

FIG. 57 is a diagram of a structure of the sealing bottle 246 shown in FIG. 55 from another perspective.

In some embodiments, the sealing bottle 246 may include a sleeving portion 2464, an accommodation portion 2462, and a boss 2465, the accommodation portion 2462 is connected to the sleeving portion 2464 and the boss 2465, and the boss 2465 is located at an end that is of the sealing bottle 246 and that faces away from the needle base 241. A diameter of the sleeving portion 2464 is less than a diameter of the accommodation portion 2462.

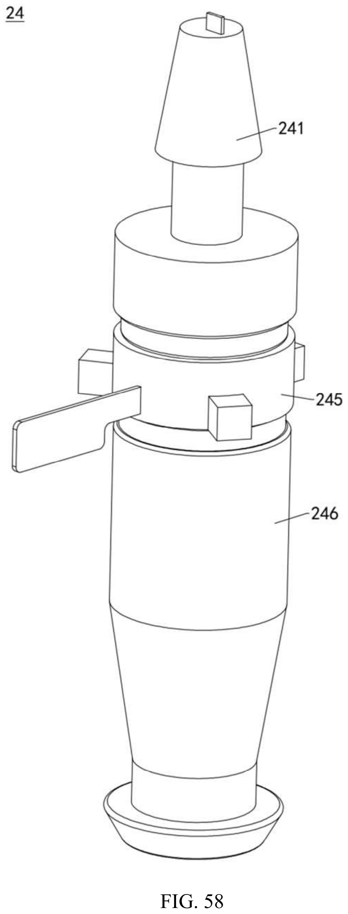

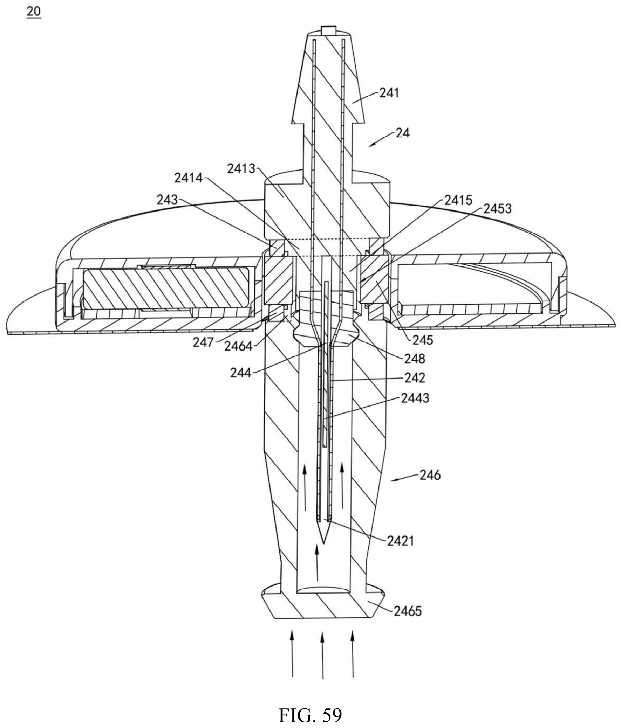

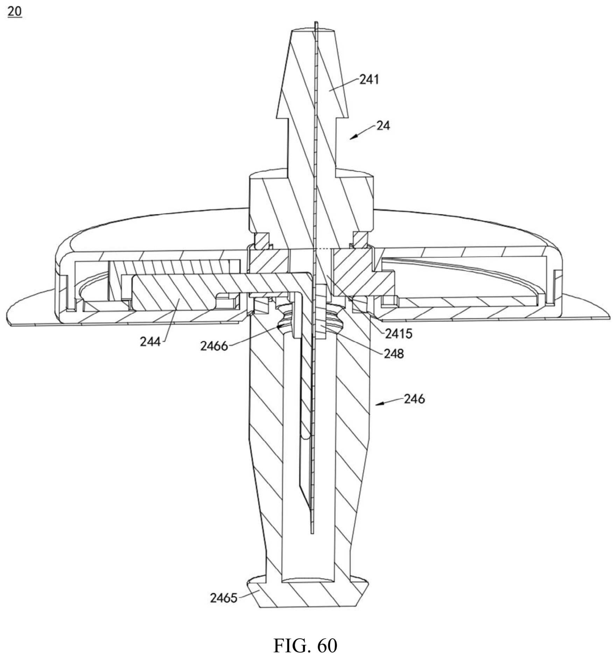

FIG. 58 is a diagram of a structure of the sensor sealing assembly 24 shown in FIG. 55. FIG. 59 is a diagram of a cross-sectional structure after the sensor assembly 20 shown in FIG. 55 is cut along I-I. FIG. 60 is a diagram of a cross-sectional structure after the sensor assembly 20 shown in FIG. 55 is cut along J-J.

In some embodiments, the sensor base 245 is located between the needle base 241 and the sealing bottle 246, an end of the needle base 241 passes through the sensor base 245, the sealing bottle 246 is snap-fitted into or interference-connected to the needle base 241 and is hermetically connected to the sensor base 245, and the needle base 241 is hermetically connected to the sensor base 245. A snap-fitting groove 2466 is disposed on an inner side of the sealing bottle 246, and the snap-fitting groove 2466 is located at an end that is of the sealing bottle 246 and that faces away from the boss 2465. For example, a flange of the snap-fit 248 may be mounted in the snap-fitting groove 2466 of the sealing bottle 246, so that the snap-fit 248 is snap-fitted into or interference-connected to the sealing bottle 246. The guiding portion 2415 of the needle base 241 passes through the sensor base 245, and the snap-fit 248 is snap-fitted into or interference-connected to the sealing bottle 246. The guiding portion 2415 is inserted into the mounting through hole 2453 of the sensor base 245, and can move relative to the sensor base 245. The first sealing member 243 is sleeved on an outer side of the sealing portion 2414, and is located between the limiting portion 2413 and the sensor base 245. The first sealing member 243 is hermetically connected to the sealing portion 2414 of the needle base 241 and the sensor base 245. The second sealing member 247 is sleeved on the sleeving portion 2464 of the sealing bottle 246, and is located between the sealing bottle 246 and the sensor base 245. The second sealing member 247 is hermetically connected to the sealing bottle 246 and the sensor base 245. In this case, the puncture needle 242 wraps the implantation portion 2443 of the sensor 244. The sealing bottle 246 has a hollow cavity, and the implantation portion 2443 of the sensor 244 and the puncture end 2421 of the puncture needle 242 are located in the hollow cavity of the sealing bottle 246.

In an existing sensor assembly, a sensor is exposed and is directly assembled with another component of the sensor assembly. In an assembly process, the sensor is easily touched, to damage a sensing material of an implantation portion of the sensor. Consequently, detection performance of the sensor is reduced, and detection accuracy is reduced. In this embodiment, the sealing bottle 246 is snap-fitted into or interference-connected to the needle base 241, to securely connect the needle base 241, the first sealing member 243, the sensor base 245, the second sealing member 247, and the sealing bottle 246, so that the first sealing member 243 is hermetically connected to the needle base 241 and the sensor base 245, the second sealing member 247 is hermetically connected to the sensor base 245 and the sealing bottle 246, and the implantation portion 2443 of the sensor 244 is located in sealed space formed by using the sensor sealing assembly 24. In addition, the implantation portion 2443 of the sensor 244 is located in the hollow cavity of the sealing bottle 246, and the sealing bottle 246 may isolate and protect the implantation portion 2443 of the sensor 244, to avoid damage to the sensing layer 2444 of the implantation portion 2443 in a subsequent assembly process of the sensor 244 and another component, thereby helping improve reliability and detection accuracy of the physiological sign detection apparatus.

The second sealing member 247 may be made of an elastic polymer material such as nitrile butadiene rubber, silicon rubber, or fluorine rubber. The second sealing member 247 and the sealing bottle 246 may be integrally formed by using a process such as a vulcanization process or a dual-shot injection molding process, so that the second sealing member 247 is fastened to a side that is of the sealing bottle 246 and that is close to the sensor base 245. The sealing bottle 246 and the second sealing member 247 are integrally formed, so that the second sealing member 247 and the sealing bottle 246 are connected more stably, to help improve sealing performance between the sealing bottle 246 and the second sealing member 247. In addition, when the sealing bottle 246 is detached from the needle base 241, the second sealing member 247 may be detached from the sensor base 245 with the sealing bottle 246.

In some embodiments, at least a part of the sealing bottle 246 allows ionizing radiation to pass through. For example, a part or all of regions of the sealing bottle 246 may allow ionizing radiation to pass through. The sealing bottle 246 may be made of engineering plastic such as polymethyl methacrylate and polycarbonate, and the engineering plastic such as methyl methacrylate and polycarbonate can both allow ionizing radiation to pass through.

Ionizing radiation may be one or a combination of electron beam radiation, gamma-ray radiation, and X-ray radiation. Ionizing radiation (as shown by an arrow in FIG. 59) passes through the sealing bottle 246, to sterilize the puncture end 2421 of the puncture needle 242 and the implantation portion 2443 of the sensor 244. When irradiation sterilization is performed on the sensor sealing assembly 24, the sensor sealing assembly 24 is placed in a positioning structure of a tray, the positioning structure of the tray may be arranged in an array, a plurality of sensor sealing assemblies 24 may be placed on one tray, and the tray is placed in a sterilization room for irradiation sterilization.

In the conventional technology, when radiation sterilization is performed on an assembled sensor assembly or physiological sign detection apparatus, a dedicated sterilization apparatus needs to be customized to implement fixed-point sterilization on a puncture end of a puncture needle and an implantation portion of a sensor. Fixed-point sterilization has a high requirement on a radiation device, and is difficult to be implemented in batched. In addition, when irradiation sterilization is performed on the entire sensor assembly or the entire physiological sign detection apparatus, the sensor assembly or the physiological sign detection apparatus needs to be assembled with the specific tooling, to perform shielding protection on an internal circuit of the sensor assembly. Consequently, sterilization operations are complex and efficiency is low. In this embodiment, at least the partial region of the sealing bottle 246 allows ionizing radiation to pass through, so that the sensor sealing assembly 24 can sterilize the puncture end 2421 of the puncture needle 242 and the implantation portion 2443 of the sensor 244 through irradiation. In addition, after sterilization, the puncture end 2421 of the puncture needle 242 and the implantation portion 2443 of the sensor 244 are still located in the sealed space formed by using the sensor sealing assembly 24. As a sterile barrier, the sealing bottle 246 may form good isolation protection for the puncture end 2421 of the puncture needle 242 and the implantation portion 2443 of the sensor 244. The puncture end 2421 of the puncture needle 242 and the implantation portion 2443 of the sensor 244 are not contaminated for a second time. In subsequent packaging, a sterilized package for ensuring that the sensor 244 is located in a sterile environment does not need to be additionally added to the physiological sign detection apparatus 100, to reduce costs. In addition, because the sensor sealing assembly 24 may be independent of an internal circuit of the sensor assembly 20, it is possible to perform only radiation sterilization on the sensor sealing assembly 24, and no additional shielding protection needs to be set. Sterilization operations are simple, and an internal circuit of the sensor 244 is not damaged. In addition, the sensor sealing assembly 24 has a small volume, and a plurality of sensor sealing assemblies 24 can be sterilized simultaneously through one radiation sterilization operation, to greatly improve sterilization efficiency and reducing sterilization costs.

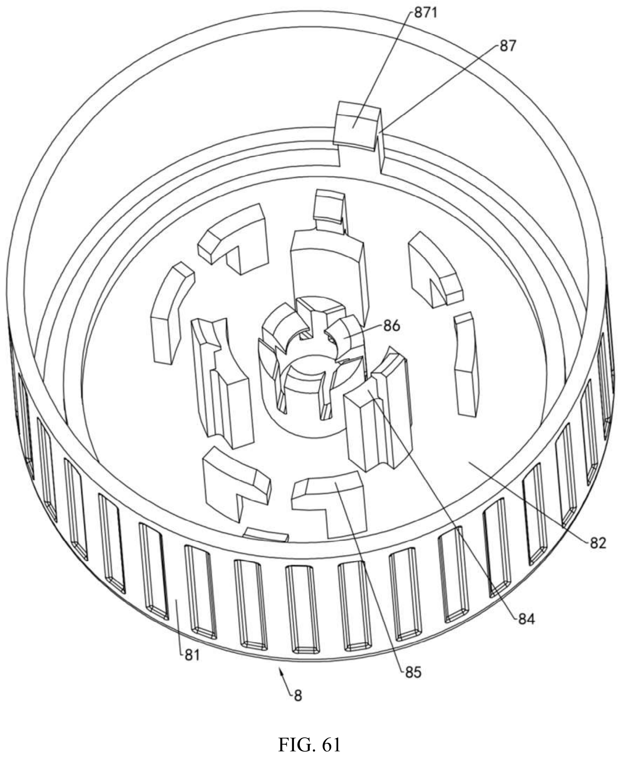

FIG. 61 is a diagram of a structure of the housing cap 8 shown in FIG. 54 in some embodiments. In some embodiments, the housing cap 8 may include a bottom wall 81, a side wall 82, a snap-fitting jaw 86, a blocking member 84, and a limiting member 85. The side wall 82 of the housing cap 8 is annularly connected to a circumferential edge of the bottom wall 81 of the housing cap 8. The snap-fitting jaw 86, the limiting member 85, the blocking member 84, and the side wall 82 of the housing cap 8 are all located on a same side of the bottom wall 81 of the housing cap 8. The snap-fitting jaw 86, the blocking member 84, and the limiting member 85 are all located on an inner side of the side wall 82 of the housing cap 8. The housing cap 8 may be generally cylindrical. The blocking member 84 and the limiting member 85 of the housing cap 8 may be disposed with reference to the foregoing embodiments. Details are not described again in this application. The blocking member 84 is located on an outer side of the snap-fitting jaw 86, and is spaced apart from the snap-fitting jaw 86. The limiting member 85 is located on an outer side of the blocking member 84 is spaced apart from the blocking member 84. The drying member 201 is snap-fitted into the limiting member 85, and the magnetic element 202 is snap-fitted into the snap-fitting member 84.

An end of the snap-fitting jaw 86 is fastened to the bottom wall 81 of the housing cap 8. There may be one or more snap-fitting jaws 86, and a plurality of snap-fitting jaws 86 are spaced apart around an axis of the housing cap 8. For example, there are three snap-fitting jaws 86.

The housing cap 8 may be an integral mechanical part. The housing cap 8 is made of engineering plastic such as polycarbonate or a polycarbonate and acrylonitrile butadiene styrene mixture. In this embodiment, the polycarbonate and acrylonitrile butadiene styrene plastic have features such as heat resistance, impact resistance, good mechanical performance, high hardness, and good processing performance. The housing cap 8 is made of the engineering plastic such as the polycarbonate or the polycarbonate and acrylonitrile butadiene styrene mixture, to help reduce manufacturing costs of the housing cap 8. In addition, the housing cap 8 has a strong scratch resistance capability. As an outer layer structure of the physiological sign detection apparatus 100, the housing cap 8 can prevent a surface of the physiological sign detection apparatus 100 from being scratched, to help improve external aesthetics of using the physiological sign detection apparatus 100.

In some other embodiments, the housing cap 8 may alternatively be made of another material, for example, engineering plastic such as polyvinyl chloride resin or polystyrene resin, or metal such as stainless steel or aluminum alloy. This is not strictly limited in this application.

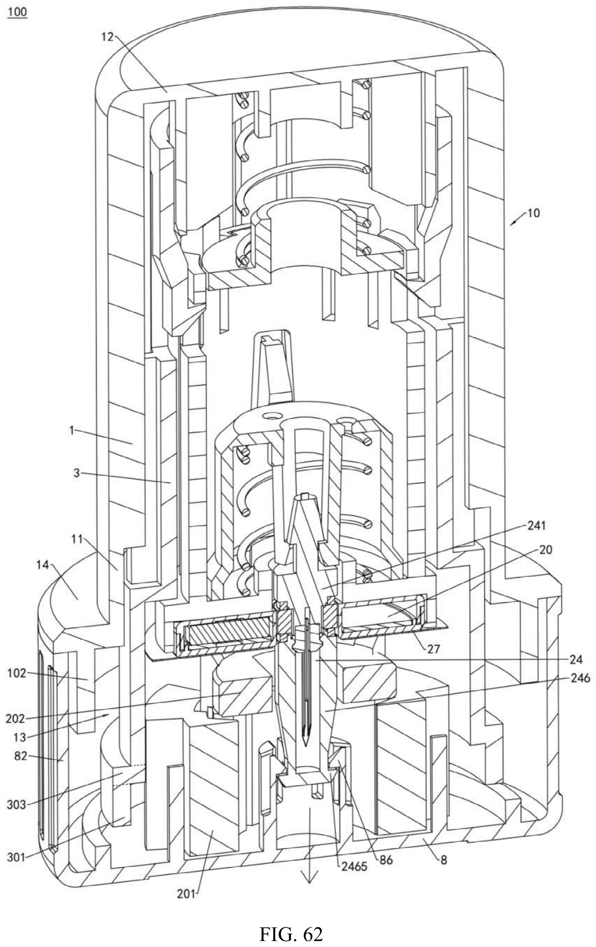

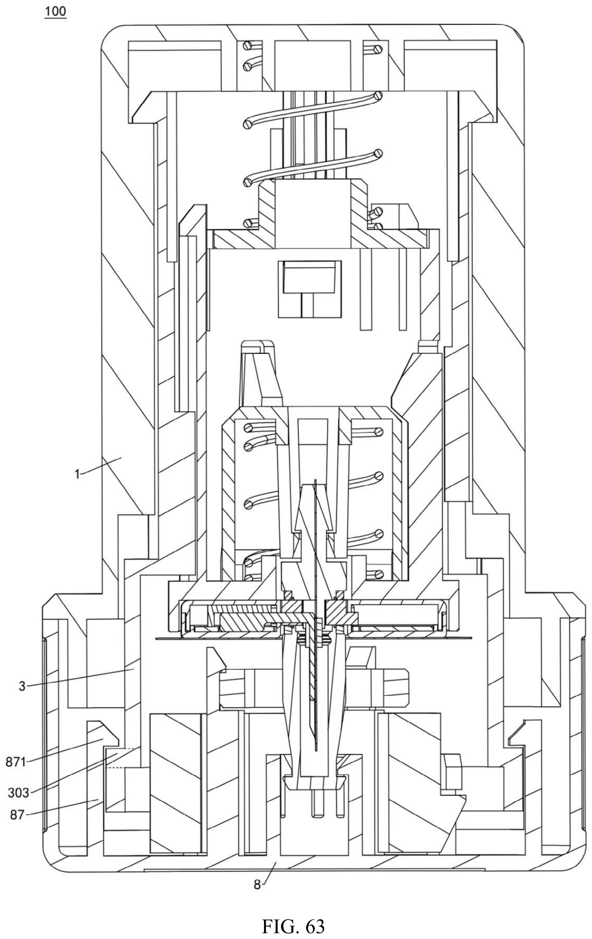

FIG. 62 is a diagram of a partial cross-sectional structure after the physiological sign detection apparatus 100 shown in FIG. 53 is cut along K-K. FIG. 63 is a diagram of a partial cross-sectional structure after the physiological sign detection apparatus 100 shown in FIG. 53 is cut along L-L.

In some embodiments, the snap-fitting jaw 86 of the housing cap 8 is snap-fitted with the boss 2465 of the sealing bottle 246. There are three snap-fitting jaws 86, so that the boss 2465 is subject to an even force, and the sealing bottle 246 is easily detached from the needle base 241. The housing cap 8 is detachably fastened to an end that is of the side wall 11 of the housing 1 and that faces away from the top wall 12 of the housing 1, and the housing cap 8 wraps the abutting end 301 of the base 3 and closes the opening 13 of the housing 1. For example, the housing cap 8 is snap-fitted into or interference-connected to the housing 1. In a process in which the housing cap 8 is detached from the housing 1 (moves in a direction shown by a solid arrow in FIG. 62), the snap-fitting jaw 86 is snap-fitted with the boss 2465, the snap-fitting jaw 86 drives the sealing bottle 246 to move toward a side away from the sensor assembly 20, and the housing cap 8 drives the sealing bottle 246 to be detached from the needle base 241.

In this embodiment, the housing cap 8 is snap-fitted into or interference-connected to the housing 1, the sealing bottle 246 is snap-fitted into or interference-connected to the needle base 241, and the snap-fitting jaw 86 of the housing cap 8 cooperates with the boss 2465 of the sealing bottle 246. When the housing cap 8 is detached from the housing 1, the sealing bottle 246 may be driven to be detached from the needle base 241, so that the puncture needle 242 is exposed, to help simplify operation steps of the physiological sign detection apparatus 100 and improve user experience.

In some embodiments, the housing 1 may include a side wall 11, a first flange 14, and a snap-fitting wall 102. The first flange 14 surrounds and is connected to an outer side of the side wall 11 of the housing 1, and a circumferential edge of the snap-fitting wall 102 is connected to a side that is of the first flange 14 and that faces away from the side wall 11 of the housing 1. In a radial direction of the physiological sign detection apparatus 100, the first flange 14 protrudes from the snap-fitting wall 102, and the snap-fitting wall 102 is located on the outer side of the side wall 11 of the housing 1. The snap-fitting wall 102 is snap-fitted into or interference-connected to the side wall 82 of the housing cap 8, so that the housing cap 8 is snap-fitted into or interference-connected to the housing 1. In addition, the snap-fitting wall 102 avoids the second flange 303 of the base 3 in the radial direction.

In some embodiments, when the housing cap 8 is fastened to the housing 1, the side wall 82 of the housing cap 8 abuts against the first flange 14, and the first flange 14 limits a mounting depth of mounting the housing cap 8 on the housing 1, to enhance sealing performance between the housing cap 8 and the housing 1, and prevent a contaminant from entering the inner side of the physiological sign detection apparatus 100 through a connection gap between the housing cap 8 and the housing 1. A knurl is disposed on the outer side surface of the side wall 82 of the housing cap 8, and the knurl may increase a friction force, so that the housing cap 8 can be screwed on the housing 1 or the housing cap 8 can be screwed off the housing 1.

In some embodiments, the physiological sign detection apparatus 100 may further include a sealing member (not shown in FIG. 62), and the housing cap 8 is hermetically connected to the housing 1 by using the sealing member. For example, the sealing member may be mounted between a first flange 14 of the housing 1 and the side wall 82 of the housing cap 8, and the side wall 82 of the housing cap 8 is hermetically connected to the first flange 14 of the housing 1 by using the sealing member. The sealing member may be made of an elastic polymer material such as nitrile butadiene rubber, silicon rubber, or fluorine rubber.

In this embodiment, the sealing member is hermetically connected to the housing 1 and the housing cap 8, to help improve sealing performance between the housing cap 8 and the housing 1, and prevent a contaminant from entering the inner side of the physiological sign detection apparatus 100. In addition, a sealed environment is formed inside the physiological sign detection apparatus 100, to prevent the bonding member 27 from being interfered by an external environment in a storage process, and help maintain bonding performance of the bonding member 27.

In some embodiments, the physiological sign detection apparatus 100 may further include a drying member 201 and a magnetic element 202. The drying member 201 and the magnetic element 202 of the physiological sign detection apparatus 100 in this embodiment may be disposed with reference to the foregoing embodiments. Details are not described again in this application.

With reference to FIG. 54, FIG. 61, and FIG. 63 together. In some embodiments, the base 3 may further include a second flange 303, and the second flange 303 is convexly disposed on an outer circumferential side of the base 3, and is located at an end that is of the base 3 and that faces away from the housing 1. The housing cap 8 may further include a sixth elastic arm 87, the sixth elastic arm 87 is located on an outer side of the limiting member 85, an end that is of the sixth elastic arm 87 and that faces the base 3 is provided with a snap-fitting hook 871, and an end that is of the sixth elastic arm 87 and that faces away from the base 3 is fastened to the bottom wall 81 of the housing cap 8. There may be one or more sixth elastic arms 87, and a plurality of sixth elastic arms 87 are spaced apart around the axis of the housing cap 8. When the side wall 82 of the housing cap 8 is snap-fitted into or is interference-connected to the housing 1, the snap-fitting hook 871 of the sixth elastic arm 87 is snap-fitted into the second flange 303 of the base 3. For example, there are two sixth elastic arms 87, and the two sixth elastic arms 87 are disposed opposite to each other, so that the second flange 303 is subject to a balanced force, and it is ensured that the housing cap 8 is stably connected to the base 3.

In this embodiment, the sixth elastic arm 87 is snap-fitted into the second flange 303, so that the housing cap 8 is snap-fitted into the base 3, to further improve stability of connecting the housing cap 8 and the base 3, thereby avoiding a case in which only the housing cap 8 is snap-fitted into or interference-connected to the housing 1, and the housing cap 8 and the housing 1 is unstably connected, and consequently, when the physiological sign detection apparatus 100 is vibrates or collides, the housing cap 8 is detached from the housing 1 and an internal component is damaged. This helps improve reliability of the physiological sign detection apparatus 100.

The snap-fitting hook of the sixth elastic arm 87 is provided with an inclined end surface, and the inclined end surface of the sixth elastic arm 87 is inclined relative to an axial direction of the housing cap 8. When the base 3 is assembled with the housing cap 8, the sixth elastic arm 87 may elastically avoid the second flange 303 in the radial direction, and the inclined end surface of the sixth elastic arm 87 avoids the second flange 303 in the axial direction, so that the housing cap 8 can be smoothly snap-fitted into the base 3, thereby reducing snap-fitting resistance, and further avoiding damage to the base 3 and the housing cap 8 during assembly.

It may be understood that the sensor assembly 20 in the foregoing other embodiments may include the sensor sealing assembly 24 in this embodiment. In addition, the sensor assembly 20 including the sensor sealing assembly 24 in this embodiment may also be of a plurality of other implementation structures. The sensor assembly 20 may also be implanted by using an implanter (including but not limited to the implanter shown in FIG. 3) of another structure, to be used in an implantation apparatus 10 of another structure.

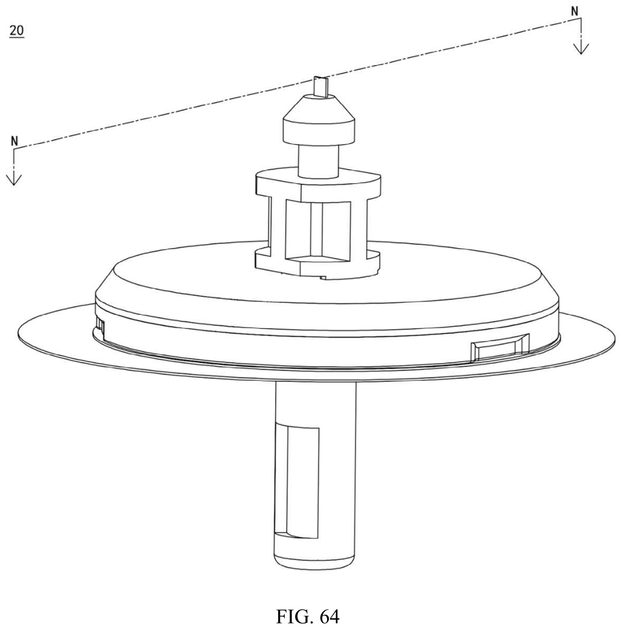

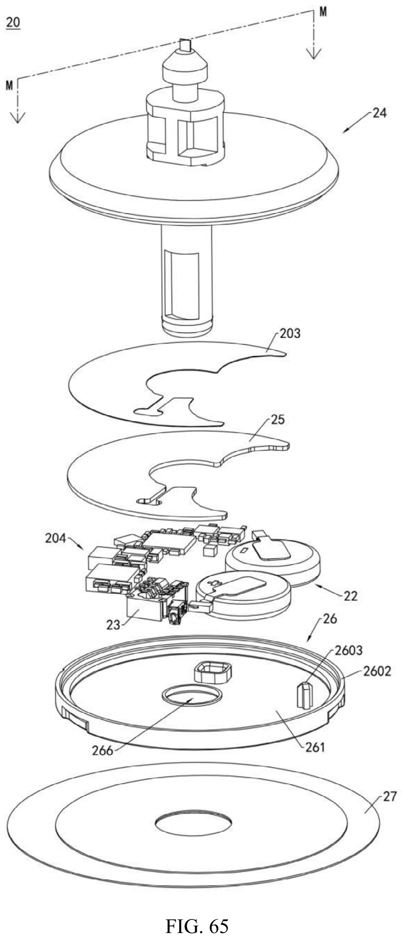

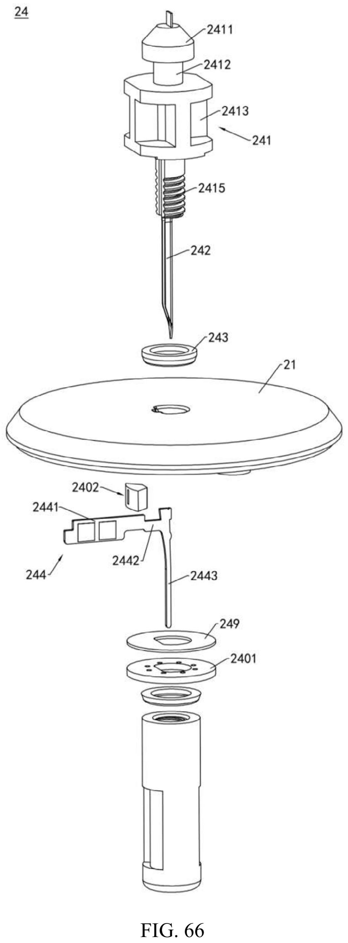

FIG. 64 is a diagram of a structure of the sensor assembly 20 shown in FIG. 2 in some other embodiments. FIG. 65 is a diagram of a partial exploded structure of the sensor assembly 20 shown in FIG. 64. FIG. 66 is a diagram of a partial exploded structure of the sensor sealing assembly 24 shown in FIG. 64. This embodiment may include most of the technical solutions in the foregoing embodiments. The following mainly describes differences between this embodiment and the foregoing embodiments, and most same content of this embodiment and the foregoing embodiments is not described again.

In some embodiments, the sensor assembly 20 may include a sensor sealing assembly 24, a first bonding member 203, a circuit board 25, a battery 22, a connector 23, an electronic element 204, a lower cover 26, and a bonding member 27. The sensor sealing assembly 24 may include a needle base 241, a puncture needle 242, a first sealing member 243, an upper cover 21, a sensor 244, a fastener 2402, a second bonding member 249, a gasket 2401, a second sealing member 247, and a sealing bottle 246. Both the upper cover 21 and the lower cover 26 may be made of engineering plastic such as polycarbonate or acrylonitrile butadiene styrene. A chip, an NFC antenna, and the like may be disposed on the circuit board 25. The electronic element 204 may include a capacitor, a resistor, Bluetooth, and the like. The second bonding member 249 and the gasket 2401 each have a through hole, and the through hole of the gasket 2401 directly faces the through hole of the second bonding member 249. Most structures of the puncture needle 242, the needle base 241, and the bonding member 27 may be set with reference to the foregoing embodiments. Details are not described again in this application.

In some embodiments, the needle base 241 may include a limiting snap-fit 2411, a snap-fitting portion 2412, a limiting portion 2413, and a guiding portion 2415. The limiting snap-fit 2411, the snap-fitting portion 2412, the limiting portion 2413, and the guiding portion 2415 are sequentially connected. The limiting snap-fit 2411 may be generally in a shape of a circular truncated cone, the snap-fitting portion 2412 may be generally in a shape of a cylinder, and a diameter of the snap-fitting portion 2412 is less than a diameter of the limiting snap-fit 2411 and a diameter of the limiting portion 2413. The sensor 244 may include a connecting portion 2441, a fastening portion 2442, and an implantation portion 2443. The fastening portion 2442 is connected to the implantation portion 2443 and the connecting portion 2441. The connecting portion 2441 of the sensor 244 is configured to be electrically connected to the connector 23, the implantation portion 2443 of the sensor 244 is configured to be punctured into the organism, and the implantation portion 2443 is provided with a sensing layer 2444 (not shown in FIG. 66). A manufacturing process of the bonding member 27, the puncture needle 242, and the sensor 244, and assembly of the puncture needle 242, the sensor 244, and the needle base 241 may be set with reference to the foregoing embodiments. Details are not described again below.

In some embodiments, the lower cover 26 may include a bottom plate 261, a side plate 2602, and a positioning protrusion 2603. The side plate 2602 of the lower cover 26 surrounds and is connected to a circumferential edge of the bottom plate 261 of the lower cover 26. The lower cover 26 is provided with a first through hole 266. An end of the positioning protrusion 2603 is fastened to the bottom plate 261 of the lower cover 26, and both the positioning protrusion 2603 and the side plate 2602 of the lower cover 26 are located on a same side of the bottom plate 261 of the lower cover 26.

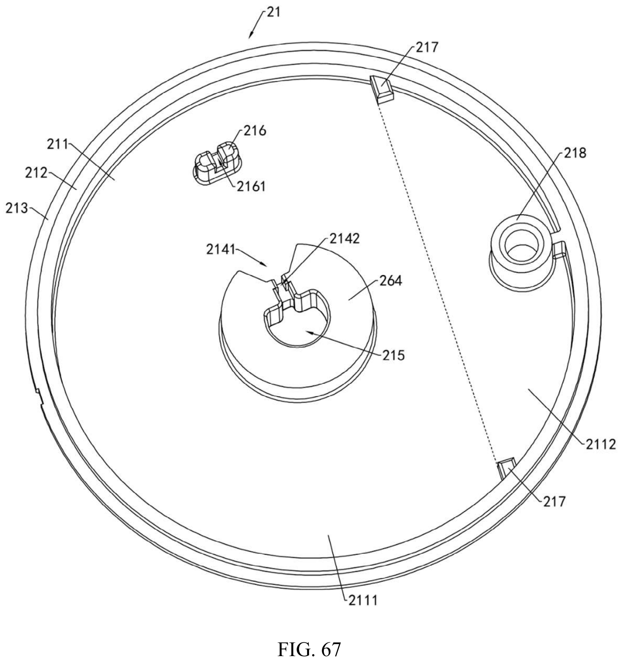

FIG. 67 is a diagram of a structure of the upper cover 21 shown in FIG. 66 from another perspective. In some embodiments, the upper cover 21 may include a top plate 211, a side plate 212, and a side plate flange 213. The top plate 211 of the upper cover 21 may be generally in a disc shape. The side plate 212 of the upper cover 21 surrounds and is connected to a circumferential edge of the top plate 211 of the upper cover 21. The side plate flange 213 surrounds and is connected to an outer circumferential side of the side plate 212 of the upper cover 21. The upper cover 21 has a second through hole 215, the second through hole 215 is located on the top plate 211 of the upper cover 21, and a center of the second through hole 215 approximately coincides with a center of the top plate 211 of the upper cover 21.

The upper cover 21 may further include a blocking flange 264, the blocking flange 264 is disposed around the second through hole 215, and the blocking flange 264 and the side plate 212 of the upper cover 21 are located on a same side of the top plate 211 of the upper cover 21. The blocking flange 264 has accommodation space 2141 and a blocking groove 2142, and the blocking groove 2142 communicates with the accommodation space 2141 and the second through hole 215. For example, the accommodation space 2141 may be a notch. The upper cover 21 may further include a first positioning member 216, the first positioning member 216 is spaced apart from the blocking flange 264, and the first positioning member 216 is disposed opposite to the accommodation space 2141 of the blocking flange 264. The first positioning member 216 has a limiting groove 2161. An extension direction of the limiting groove 2161 is the same as an extension direction of the blocking groove 2142, and both the limiting groove 2161 and the blocking groove 2142 are located or approximately located in a same straight line.

The upper cover 21 may further include a second positioning member 217 and a positioning flange 218. The second positioning member 217 and the positioning flange 218 are both spaced apart from the first positioning member 216 and the blocking flange 264. The positioning flange 218 has an accommodation groove. For example, there are two second positioning members 217, and the two second positioning members 217 are opposite and spaced apart from each other.

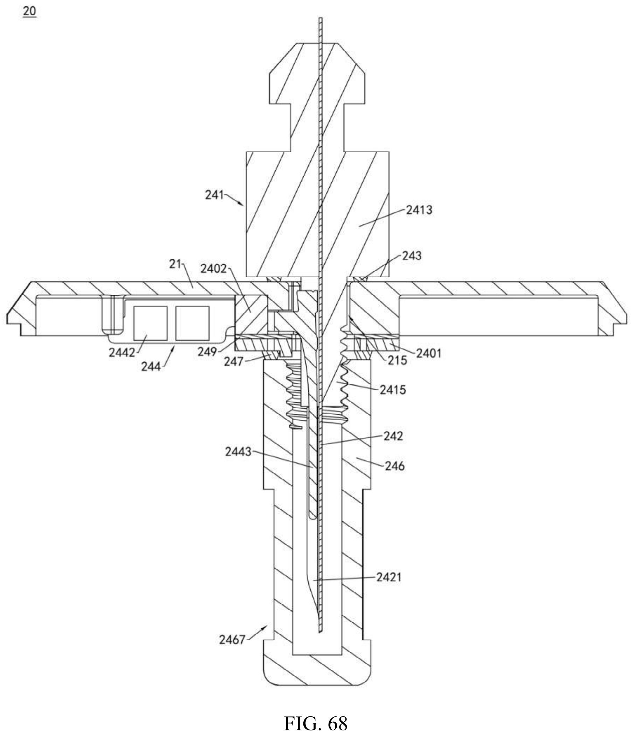

Refer to FIG. 67 and FIG. 68 together. FIG. 68 is a diagram of a cross-sectional structure after a sensor sealing assembly 24 shown in FIG. 65 is cut along M-M.

In some embodiments, the sensor 244 is snap-fitted into the upper cover 21, the upper cover 21 is located between the needle base 241 and the sealing bottle 246, an end of the needle base 241 passes through the upper cover 21, the sealing bottle 246 is detachably fastened to the needle base 241, and is hermetically connected to the upper cover 21, and the needle base 241 is hermetically connected to the upper cover 21. The guiding portion 2415 of the needle base 241 is inserted into the second through hole 215 of the upper cover 21, and the guiding portion 2415 is detachably fastened to the sealing bottle 246. For example, the needle base 241 may be threadedly connected to the sealing bottle 246, an outer side surface of the guiding portion 2415 is provided with an external thread, an inner side surface of the sealing bottle 246 is provided with an internal thread, and the guiding portion 2415 is threadedly connected to the sealing bottle 246. In some other embodiments, a connection between the needle base 241 and the sealing bottle 246 may also be a snap-fit connection, an interference-connection, or the like.

The first sealing member 243 is mounted between the limiting portion 2413 of the needle base 241 and the upper cover 21, and the first sealing member 243 is hermetically connected to the limiting portion 2413 of the needle base 241 and the upper cover 21. The fastening portion 2442 of the sensor 244 is snap-fitted into the blocking groove 2142 of the upper cover 21, and a tail end of the connecting portion 2441 is snap-fitted into the limiting groove 2161 of the upper cover 21. The blocking flange 264 and the first positioning member 216 position and limit the sensor 244, and the sensor 244 can slightly move in the blocking groove 2142 and the limiting groove 2161, to prevent the sensor 244 from being stuck or worn out. The fastener 2402 fills in the accommodation space 2141 of the blocking flange 264, and is configured to fasten the fastening portion 2442 of the sensor 244 with the upper cover 21. The puncture needle 242 wraps the implantation portion 2443 of the sensor 244. The second bonding member 249 may be a double-sided adhesive, a side of the second bonding member 249 is stuck on the blocking flange 264, and the other side of the second bonding member 249 is stuck on the gasket 2401, to fasten the gasket 2401 to the blocking flange 264. The second sealing member 247 is located between the gasket 2401 and the sealing bottle 246, and is hermetically connected to the sealing bottle 246 and the gasket 2401. In this case, the puncture needle 242 wraps the implantation portion 2443 of the sensor 244. The sealing bottle 246 has a hollow cavity, and the implantation portion 2443 of the sensor 244 and the puncture end 2421 of the puncture needle 242 are located in the hollow cavity of the sealing bottle 246.

In an existing sensor assembly, a sensor is exposed and is directly assembled with another component of the sensor assembly. In an assembly process, the sensor is easily touched, to damage a sensing material of an implantation portion of the sensor. Consequently, detection performance of the sensor is reduced, and detection accuracy is reduced. In this embodiment, the needle base 241 is connected to the sealing bottle 246 threadedly or in another manner, to securely connect the needle base 241, the first sealing member 243, the upper cover 21, the fastener 2402, the gasket 2401, the second sealing member 247, and the sealing bottle 246, so that the first sealing member 243 is hermetically connected to the needle base 241 and the sensor base 245, the gasket 2401 and the second sealing member 247 are hermetically connected to the sensor base 245 and the sealing bottle 246, and the implantation portion 2443 of the sensor 244 is located in sealed space formed by using the sensor sealing assembly 24. In addition, the implantation portion 2443 of the sensor 244 is located in the hollow cavity of the sealing bottle 246, and the sealing bottle 246 may isolate and protect the implantation portion 2443 of the sensor 244, to avoid damage to the sensing layer 2444 of the implantation portion 2443 in a subsequent assembly process of the sensor 244 and another component, thereby helping improve reliability and detection accuracy of the physiological sign detection apparatus 100, and ensuring batch consistency of the sensor 244 in a production process.

The first sealing member 243 may be made of an elastic polymer material such as nitrile butadiene rubber, silicon rubber, or fluorine rubber. In some embodiments, the needle base 241 and the first sealing member 243 may be integrally formed by using a process such as a vulcanization process or a dual-shot injection molding process, so that the first sealing member 243 is formed on the limiting portion 2413 of the needle base 241. The needle base 241 and the first sealing member 243 are integrally formed, so that the first sealing member 243 and the needle base 241 are connected more stably, to help improve sealing performance between the needle base 241 and the first sealing member 243. In addition, when the needle base 241 is detached from the upper cover 21, the needle base 241 may be detached from the upper cover 21 together with the first sealing member 243. In some other embodiments, the first sealing member 243 may alternatively be fastened to the needle base 241 through bonding, or the like.

The second sealing member 247 may be made of an elastic polymer material such as nitrile butadiene rubber, silicon rubber, or fluorine rubber. In some embodiments, the second sealing member 247 and the sealing bottle 246 may be integrally formed by using a process such as a vulcanization process or a dual-shot injection molding process, so that the second sealing member 247 is fastened to a side that is of the sealing bottle 246 and that is close to the sensor base 245. The sealing bottle 246 and the second sealing member 247 are integrally formed, so that the second sealing member 247 and the sealing bottle 246 are connected more stably, to help improve sealing performance between the sealing bottle 246 and the second sealing member 247. In addition, when the sealing bottle 246 is detached from the needle base 241, the second sealing member 247 may be detached from the gasket 2401 with the sealing bottle 246. In some other embodiments, the second sealing member 247 may alternatively be fastened to the sealing bottle 246 through bonding, or the like.

In some embodiments, an outer side surface of the sealing bottle 246 is provided with a snap-fitting groove 2467, and the snap-fitting groove 2467 is configured to cooperate with a pawl 83 (not shown in FIG. 68) of the housing cap 8, so that the housing cap 8 can drive the sealing bottle 246 to rotate. In addition, the ratchet described above may also be disposed on the outer side surface of the sealing bottle 246. In some other embodiments, the sealing bottle 246 may be snap-fitted into or interference-connected to the needle base 241, and an end that is of the sealing bottle 246 and that faces away from the needle base 241 is provided with a boss.

In some embodiments, the fastener 2402 may be formed by curing glue, so that while the sensor 244 and the upper cover 21 are fastened, shape adaptation is better implemented, and difficulty in molding and assembling is low. For example, the fastener 2402 may be ultraviolet cured glue, and the glue can be quickly cured by irradiating ultraviolet light or visible light, thereby improving assembly efficiency. The sealing bottle 246 may be made of an opaque material, for example, a black engineering plastic such as polymethyl methacrylate and polycarbonate. In an ultraviolet light curing process of the fastener 2402, ultraviolet light does not radiate to the implantation portion 2443 of the sensor 244 through the sealing bottle 246, so that an enzyme at the sensing layer 2444 can be prevented from being deactivated due to impact of the ultraviolet light, thereby helping improve detection accuracy of the physiological sign detection apparatus 100. In some other embodiments, the fastener 2402 may also be formed by curing another glue, for example, may be normal-temperature cured glue.

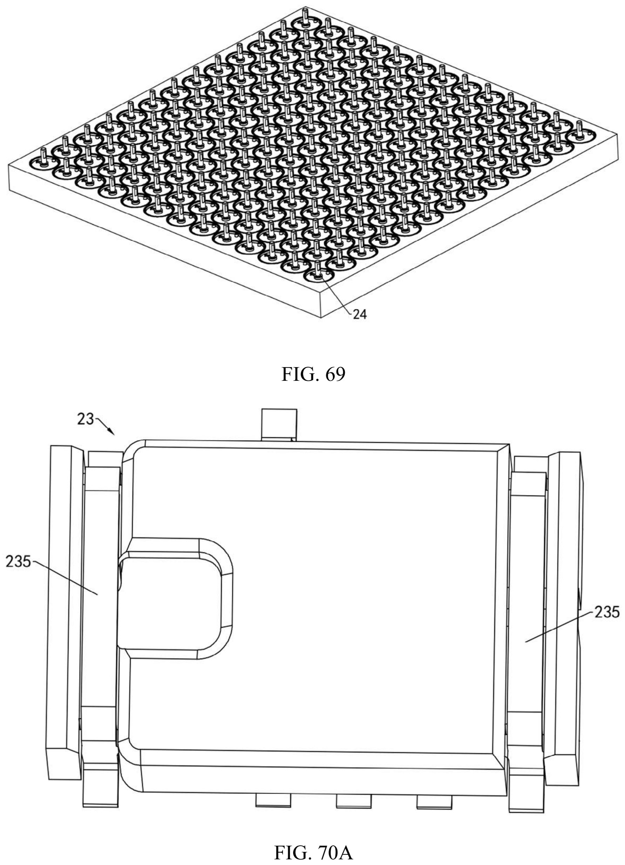

In some other embodiments, the sensor sealing assembly 24 may not be provided with the second bonding member 249 and the gasket 2401, and the sealing bottle 246 may be directly hermetically connected to the upper cover 21 and the fastener 2402 by using the second sealing member 247. Refer to FIG. 69 and FIG. 68 together. FIG. 69 is a diagram of a structure of a sterilization tooling structure of the sensor sealing assembly 24 shown in FIG. 65.

This application provides a sterilization solution. A volume of the sensor sealing assembly 24 is small, and a plurality of sensor sealing assemblies 24 may be mounted on a same tray, to perform irradiation sterilization on puncture ends 2421 of puncture needles 242 and sensors 244 in batch sensor sealing assemblies 24 through ionizing radiation. Radiation sterilization of the sensor sealing assembly 24 may be set with reference to the foregoing embodiments. Details are not described again in this application.

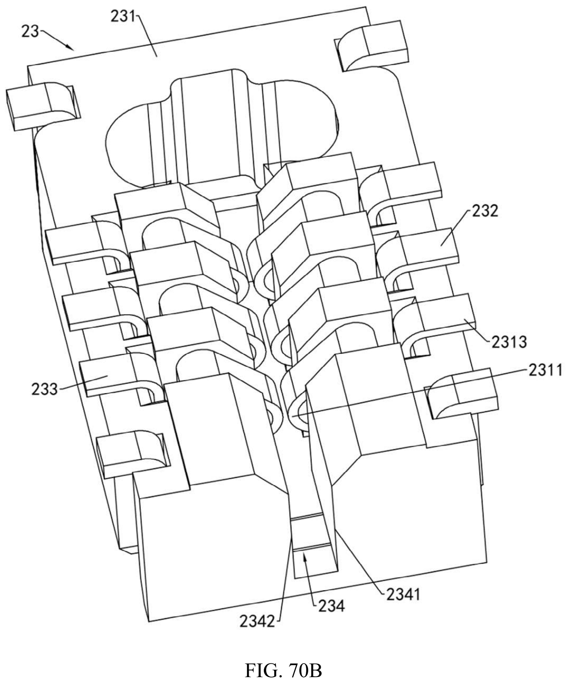

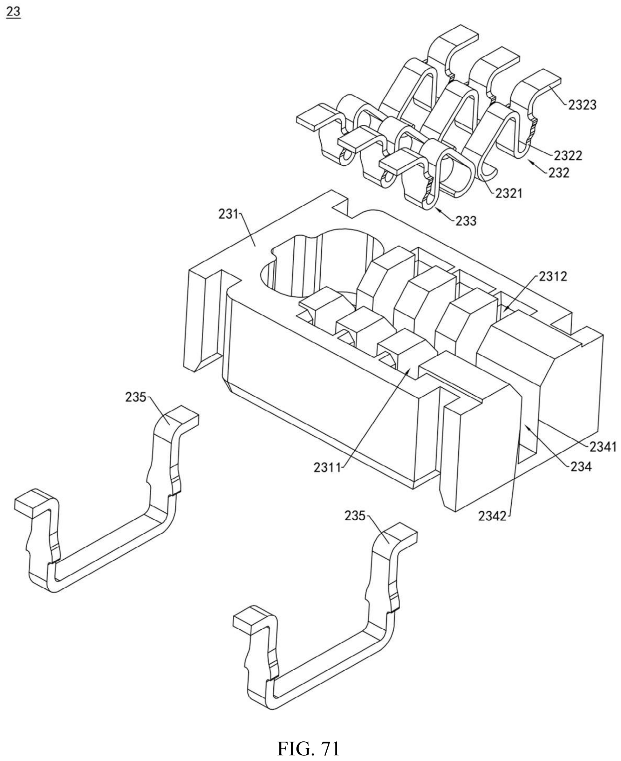

FIG. 70A is a diagram of a structure of the connector 23 shown in FIG. 65 in some embodiments. FIG. 70B is a diagram of a structure of the connector 23 shown in FIG. 70A from another perspective. FIG. 71 is a diagram of an exploded structure of the connector 23 shown in FIG. 70B. In some embodiments, the connector 23 may include a connector body 231, a plurality of first spring plates 232, and a plurality of second spring plates 233. The plurality of first spring plates 232 and the plurality of second spring plates 233 are disposed opposite to each other in a one-to-one correspondence, and the plurality of first spring plates 232 and the plurality of second spring plates 233 are all fastened to the connector body 231. The connector body 231 has a connecting groove 234, and the connecting groove 234 includes a first groove wall 2341 and a second groove wall 2342 that are disposed opposite to each other. At least a part of the plurality of first spring plates 232 is convexly disposed on the first groove wall 2341, at least a part of the plurality of second spring plates 233 is convexly disposed on the second groove wall 2342, and a gap is formed between the plurality of first spring plates 232 and the plurality of second spring plates 233. The connector body 231 further has a first mounting groove 2311 and a second mounting groove 2312, and the first mounting groove 2311 and the second mounting groove 2312 are disposed opposite to each other. The first mounting groove 2311 and the second mounting groove 2312 are respectively located on two sides of the connecting groove 234, and both are connected to the connecting groove 234. The first spring plate 232 is mounted in the first mounting groove 2311, and the second spring plate 233 is mounted in the second mounting groove 2312. For example, there may be a plurality of first mounting grooves 2311 and a plurality of second mounting grooves 2312, and a quantity of first mounting grooves 2311 and a quantity of second mounting grooves 2312 are respectively commensurate with a quantity of first spring plates 232 and a quantity of second spring plates 233.

The first spring plate 232 may include an abutting portion 2321, a fastening portion 2322, and a connecting portion 2323. The fastening portion 2322 is connected to the abutting portion 2321 and the connecting portion 2323. The abutting portion 2321 is an elastic free end, and may move in a direction close to or away from the fastening portion 2322. The fastening portion 2322 is mounted in the first mounting groove 2311 and is fastened to the connector body 231. The connecting portion 2323 protrudes from the connector body 231. The abutting portion 2321 of the first spring plate 232 protrudes from the first groove wall 2341 of the connecting groove 234. The second spring plate 233 may include an abutting portion, a fastening portion, and a connecting portion. The abutting portion, the fastening portion, and the connecting portion of the second spring plate 233 are disposed with reference to the first spring plate 232. Details are not described again below. For example, the first spring plate 232 and the second spring plate 233 each may be an integral mechanical part formed by bending a metal plate. The first spring plate 232 and the second spring plate 233 each include two plate surfaces disposed opposite to each other and a side surface connecting the two plate surfaces. A distance between two plate surfaces is a thickness of a spring plate, and the side surface is a surface on which the thickness of the spring plate is located. Surfaces that are of the first spring plate 232 and the second spring plate 233 and that are disposed opposite to each other in the connecting groove 234 are plate surfaces. In other words, a plate surface of the first spring plate 232 and a plate surface of the second spring plate 233 are disposed opposite to each other in the connecting groove 234.

In some embodiments, the connector 23 further includes a reinforcing member 235, and the reinforcing member 235 is fastened to the connector body 231. There may be two reinforcing members 235, and the two reinforcing members 235 are respectively fastened to two ends of the connector body 231.

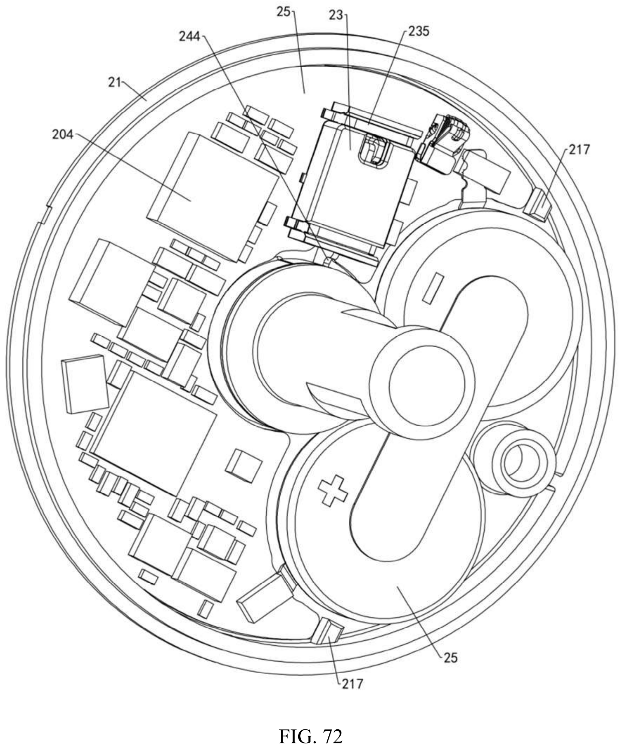

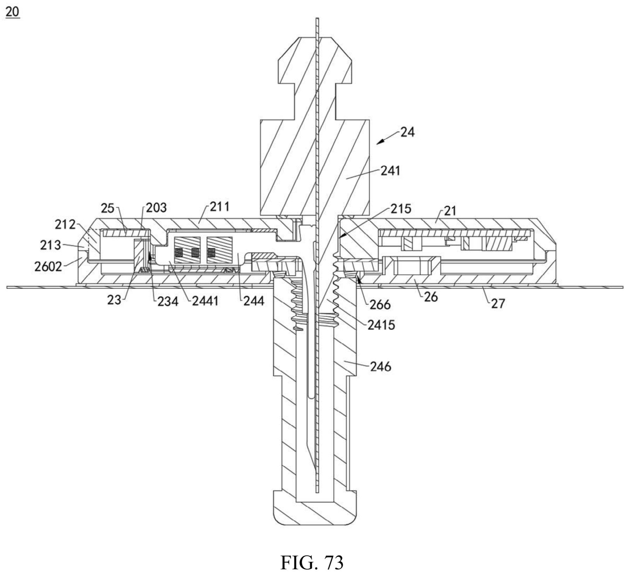

Refer to FIG. 67, FIG. 72, and FIG. 73 together. FIG. 72 is a diagram of a partial structure of the sensor assembly 20 shown in FIG. 64. FIG. 73 is a diagram of a cross-sectional structure after the sensor assembly 20 shown in FIG. 64 is cut along N-N.

In some embodiments, the top plate 211 of the upper cover 21 may include a first region 2111 and a second region 2112, the circuit board 25 is fastened in the first region 2111, and the two second positioning members 217 disposed opposite to each other position and limit the circuit board 25. The first bonding member 203 may be a double-sided adhesive, a side of the first bonding member 203 is stuck on the top plate 211 of the upper cover 21, and the other side of the first bonding member 203 is stuck on the circuit board 25. The battery 22 is fastened to the second region 2112, and is electrically connected to the circuit board 25. The electronic element 204 is fastened to the circuit board 25. The connector 23 is fastened and electrically connected to the circuit board 25. Both the connecting portion 2323 of the first spring plate 232 and the connecting portion of the second spring plate 233 may be welded to the circuit board 25, and the reinforcing member 235 may be welded to the circuit board 25. The connecting portion 2441 of the sensor 244 is snap-fitted into the connecting groove 234 of the connector 23, and is electrically connected to the plurality of first spring plates 232 and the plurality of second spring plates 233 (not shown in FIG. 73). For example, the sensor 244 is mounted between abutting portions 2321 of the plurality of first spring plates 232 and abutting portions of the plurality of second spring plates 233, and is electrically connected to the abutting portions 2321 of the plurality of first spring plates 232 and the abutting portions of the plurality of second spring plates 233.

In this embodiment, the sensor 244 is electrically connected to the abutting portions 2321 of the plurality of first spring plates 232 and the abutting portions of the plurality of second spring plates 233. A plate surface of the first spring plate 232 is in contact with the sensor 244, and a plate surface of the second spring plate 233 is in contact with the sensor 244. The sensor 244 is in face contact with the first spring plate 232 and the second spring plate 233, a contact area is large, the sensor 244 is subject to an even force, and the sensor 244 is not easily damaged. In addition, when a tail end of the connecting portion 2441 of the sensor 244 is snap-fitted into a first positioning member 216, the tail end may move slightly in a limiting groove 2161. Therefore, the connecting portion 2441 of the sensor 244 is electrically connected to an abutting portion 2321 of the first spring plate 232 and an abutting portion 23 of the second spring plate 233, and a position of the connecting portion 2441 of the sensor 244 may be slightly adjusted to implement self-adaptation, to better abut against the first spring plate 232 and the second spring plate 233, so that a connecting position is subject to an even force, thereby ensuring that the sensor 244 is stably connected to the first spring plate 232 and the second spring plate 233, to reduce fatigue caused by an uneven force applied to the first spring plate 232 and the second spring plate 233 for a long period of time.

In some embodiments, after the sensor sealing assembly 24 is completely assembled, both the circuit board 25 and the battery 22 are fastened to the top plate 211 of the upper cover 21, and then assembly of the electronic elements 204 and the connector 23 is completed. Finally, the sealing bottle 246 passes through the first through hole 266 of the lower cover 26, and the lower cover 26 is fastened to the upper cover 21, so that assembly of the sensor assembly 20 can be completed. The side plate 2602 of the lower cover 26 is snap-fitted into the side plate 212 and the side plate flange 213 of the upper cover 21, the positioning protrusion 2603 (as shown in FIG. 65) is mounted in the accommodation groove of the positioning flange 218, and the positioning protrusion 2603 cooperates with the positioning flange 218, so that the upper cover 21 and the lower cover 26 are assembled and fastened.

In this embodiment, the sensor sealing assembly 24 includes the upper cover 21, and the upper cover 21 only needs to be provided with the second through hole 215 that allows the guiding portion 2415 of the needle base 241 to pass through. A hole diameter of the second through hole 215 is smaller. In addition, the needle base 241 is fastened to the upper cover 21 by being securely connected to the sealing bottle 246. Assembly between the upper cover 21 and the needle base 241 is simpler, and the lower cover 26 does not need to be provided with a structure for being snap-fitted into the sensor sealing assembly 24, to help simplify a structure of the lower cover 26.

It may be understood that the sensor assembly 20 in the foregoing other embodiments may include the sensor sealing assembly 24 in this embodiment. In addition, other implementation structures of the sensor assembly 20 including the sensor sealing assembly 24 in this embodiment may also be of a plurality of types. The sensor assembly 20 may be applied to the implantation apparatus in the foregoing embodiments, or may be applied to another type of implantation apparatus.

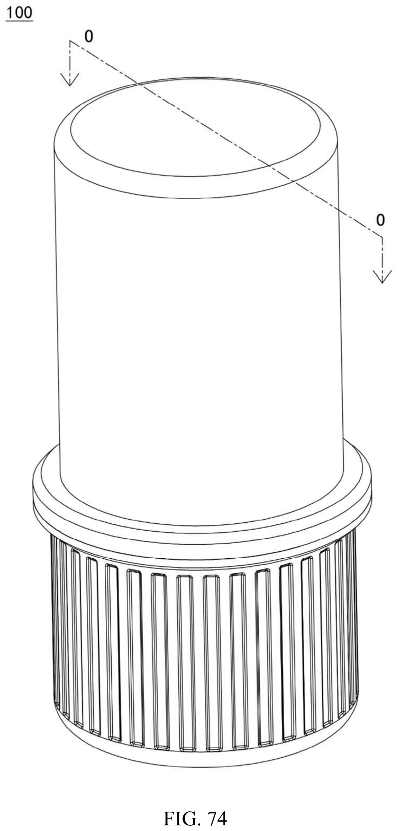

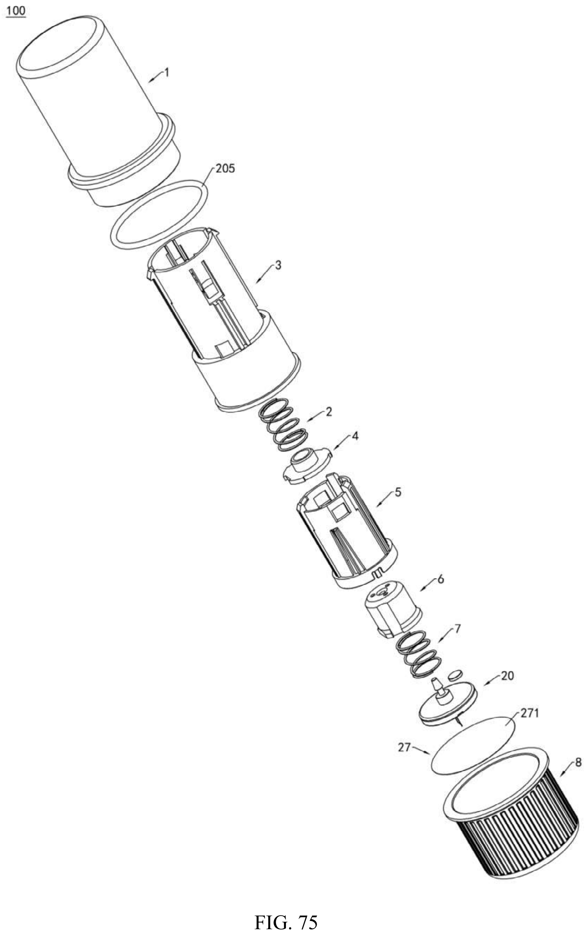

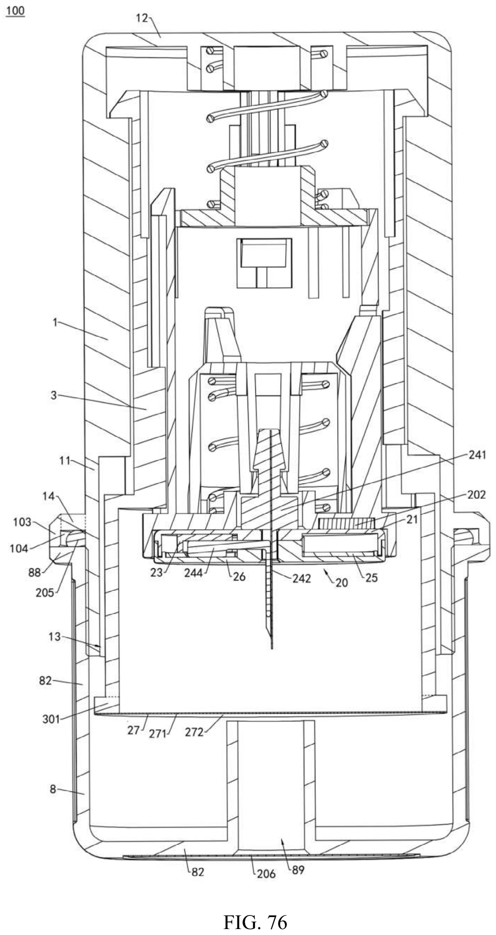

FIG. 74 is a diagram of a structure of the physiological sign detection apparatus 100 shown in FIG. 1 in some other embodiments. FIG. 75 is a diagram of a partial exploded structure of the physiological sign detection apparatus 100 shown in FIG. 74. FIG. 76 is a diagram of a partial cross-sectional structure after the physiological sign detection apparatus 100 shown in FIG. 74 is cut along O-O. This embodiment may include most of the technical solutions in the foregoing embodiments. The following mainly describes differences between this embodiment and the foregoing embodiments, and most same content of this embodiment and the foregoing embodiments is not described again.

In some embodiments, the physiological sign detection apparatus 100 may further include a housing 1, a base 3, a first elastic member 2, a stopper member 4, an implantation base 5, a needle extraction base 6, a second elastic member 7, a sensor assembly 20, and a housing cap 8. In this embodiment, the housing 1, the base 3, the first elastic member 2, the stopper member 4, the implantation base 5, the needle extraction base 6, the second elastic member 7, and the housing cap 8 of the physiological sign detection apparatus 100 may be disposed with reference to the foregoing embodiments. Details are not described again below. The sensor assembly 20 may include an upper cover 21, a needle base 241, a sensor 244, a puncture needle 242, a first sealing member 243, a sensor base 245, a battery 22 (not shown in FIG. 76), a connector 23, a circuit board 25, and a lower cover 26. The upper cover 21, the needle base 241, the first sealing member 243, the sensor 244, the sensor base 245, the puncture needle 242, the battery 22, the connector 23, the circuit board 25, and the lower cover 26 of the sensor assembly 20 may be disposed with reference to the foregoing embodiments. Details are not described again below.

In some embodiments, the physiological sign detection apparatus 100 may further include a bonding member 27, the bonding member 27 has a first surface 271 and a second surface 272 that are disposed opposite to each other, and the first surface 271 of the bonding member 27 is stuck on an abutting end 301 and covers the sensor assembly 20. The second surface 272 of the bonding member 27 is configured to be stuck on an organism 30 when the sensor assembly 20 abuts against the organism 30. In this embodiment, the first surface 271 of the bonding member 27 is stuck on the abutting end 301 of the base 3, and covers the sensor assembly 20. The bonding member 27 shields the puncture needle 242. When a user removes the housing cap 8, the puncture needle 242 and the sensor 244 cannot be seen in a field of view of the user, to help eliminate trypanophobia of the user and improve user experience.

In some embodiments, the physiological sign detection apparatus 100 may further include a sealing member 205, and the housing cap 8 is hermetically connected to the housing 1 by using the sealing member 205. For example, the housing 1 may include a side wall 11, a top wall 12, a first flange 14, and a limiting arm 103, and the side wall 11 of the housing 1 is connected to a circumferential edge of the top wall 12 of the housing 1. An opening 13 is formed on a side that is of the side wall 11 of the housing 1 and that is away from the top wall 12 of the housing 1. The first flange 14 is convexly disposed on an outer circumferential side of the side wall 11 of the housing 1. In an axial direction of the physiological sign detection apparatus 100, the first flange 14 is located at an end that is of the side wall 11 of the housing 1 and that is close to the opening 13 of the housing 1. The limiting arm 103 is disposed opposite to the side wall 11 of the housing 1, and the limiting arm 103 surrounds and is connected to the first flange 14. Mounting space 104 is formed between the limiting arm 103, the first flange 14, and the side wall 11 of the housing 1, and the sealing member 205 is mounted in the mounting space 104. The mounting space 104 limits the sealing member 205, to prevent the sealing member 205 from being detached from the housing 1, thereby helping improve sealing performance and reliability of the physiological sign detection apparatus 100. The housing cap 8 may include a side wall 82, a bottom wall 81, and a third flange 88, the side wall 82 of the housing cap 8 surrounds and is connected to a circumferential edge of the bottom wall 81 of the housing cap 8, and the third flange 88 is convexly disposed on an outer circumferential side of the side wall 82 of the housing cap 8. The third flange 88 abuts against the limiting arm 103 and the sealing member 205, so that the sealing member 205 is hermetically connected to the housing 1 and the housing cap 8.

The housing cap 8 is detachably fastened to an end that is of the side wall 11 of the housing 1 and that faces away from the top wall 12 of the housing 1, and the housing cap 8 wraps the abutting end 301 and closes the opening 13 of the housing 1. For example, a detachable connection relationship between the housing 1 and the housing cap 8 may be implemented in a connection manner such as a magnetic connection, a snap-fitting connection, an interference connection, or a threaded connection. For example, the side wall 82 of the housing cap 8 is snap-fitted into, interference-connected to, or threadedly connected to the side wall 11 of the housing 1. This is not strictly limited in this embodiment of this application.

In some embodiments, the physiological sign detection apparatus 100 may further include a thin film 206, the housing cap 8 is provided with a through hole 89, the thin film 206 covers the through hole 89 of the housing cap 8, and the thin film 206 is made of a waterproof and breathable material. For example, the through hole 89 of the housing cap 8 is located on the bottom wall 81 of the housing cap 8, and the thin film 206 is stuck on an outer side of the bottom wall 81 of the housing cap 8 and covers the through hole 89 of the housing cap 8. The thin film 206 may be made of a Tyvek material. The Tyvek material is not easy to deform, and is moisture-proofing, water-resistant, and bacterial-proofing.

In this embodiment, the physiological sign detection apparatus 100 may be sterilized by using epoxyethane. A waterproof and breathable thin film 206 allows gas to pass through and isolates water vapor. An epoxyethane gas may pass through the thin film 206, and enter an inner side of the physiological sign detection apparatus 100 through the through hole 89 of the housing cap 8, to perform overall sterilization on all components on the inner side of the physiological sign detection apparatus 100.

A procedure of sterilizing the physiological sign detection apparatus 100 by using epoxyethane is as follows: After the physiological sign detection apparatus 100 is assembled, epoxyethane is first injected into an inner side of the physiological sign detection apparatus 100 by using the thin film 206, a structure in the physiological sign detection apparatus 100 is sterilized by using epoxyethane, and epoxyethane on an inner side of the physiological sign detection apparatus 100 is extracted by using the thin film 206. In this way, sterilization is completed. After the physiological sign detection apparatus 100 is sterilized, plastic sealing packaging may be performed on the physiological sign detection apparatus 100, and a sealed plastic film is coated on the outer side of the physiological sign detection apparatus 100, to further prevent water vapor or bacteria from entering the inner side of the physiological sign detection apparatus 100.

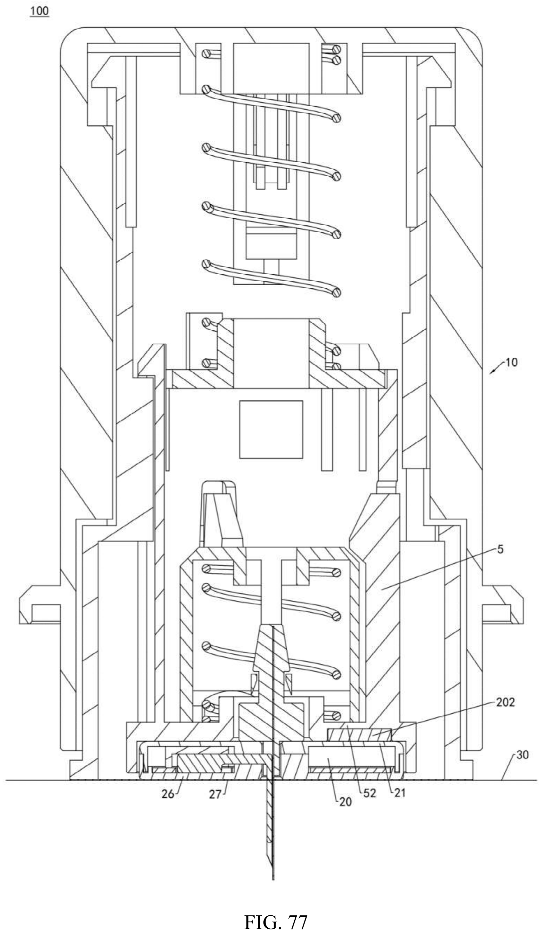

Referring to FIG. 76 and FIG. 77 together. FIG. 77 is a diagram of a partial cross-sectional structure after the physiological sign detection apparatus 100 shown in FIG. 74 is cut along O-O in a puncturing process.

In some embodiments, an implantation process of the physiological sign detection apparatus 100 may be set with reference to the foregoing embodiments. Details are not described again in this application. When the implantation base 5 pushes the sensor assembly 20 against the organism 30, the first surface 271 of the bonding member 27 is stuck on the lower cover 26 of the sensor assembly 20, and the second surface 272 of the bonding member 27 is stuck on the organism 30, so that the sensor 244 is stuck on the organism 30.

In some embodiments, the physiological sign detection apparatus 100 may further include a magnetic element 202, the bottom plate 52 of the implantation base 5 has a mounting groove, an opening of the mounting groove faces the upper cover 21 of the sensor assembly 20, and the magnetic element 202 is mounted in the mounting groove. For example, the magnetic element 202 may be a magnet. The magnetic element 202 can form a magnetic field around the magnetic element 202, and serves as a magnetic switch. A magnetic switch (not shown in the figure) is disposed on an inner side of the sensor assembly 20. When the sensor assembly 20 is snap-fitted into the implantation base 5, the magnetic element 202 is close to the sensor assembly 20, the magnetic switch is located in a magnetic field of the magnetic element 202, the magnetic switch controls the sensor assembly 20 to be in a standby state, and power consumption of the sensor assembly 20 is extremely low, to help reduce power consumption of the battery 22 in the sensor assembly 20 and prolong product use time. When the physiological sign detection apparatus 100 is implanted, and the magnetic element 202 gets away from the sensor assembly 20 with the implantation base 5, the magnetic switch cannot sense the magnetic field, and the magnetic switch controls the sensor assembly 20 to be activated, so that the sensor assembly 20 is started and is in a working state.

In this embodiment, the magnetic element 202 is mounted on the bottom plate 52 of the implantation base 5, assembly between the magnetic element 202 and the implantation base 5 is simple, and the magnetic element 202 may get close to the sensor assembly 20 or get away from the sensor assembly 20 with the implantation base 5, to control the magnetic switch in the sensor assembly 20.

It may be understood that the sensor assembly 20 and the bonding member 27 in this embodiment may also be used in the implantation apparatus 10 in the foregoing embodiments, or another similar implantation apparatus 10. This is not strictly limited in this application.

The foregoing embodiments are merely used to describe the technical solutions of this application, but are not intended to limit the technical solutions. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application.

## Claims

1. A physiological sign detection apparatus (100), comprising:
a housing (1), comprising a top wall (12) and a side wall (11), wherein the side wall (11) of the housing (1) is annularly connected to a circumferential edge of the top wall (12) of the housing (1), and an opening (13) is formed on a side that is of the side wall (11) of the housing (1) and that is away from the top wall (12) of the housing (1);
a base (3), mounted on an inner side of the housing (1) and slidably connected to the side wall (11) of the housing (1), wherein the base (3) comprises an abutting end (301), and the abutting end (301) protrudes from the housing (1) through the opening (13);
an implantation base (5), mounted on an inner side of the base (3) and slidably connected to the base (3);
a first elastic member (2), connected between the top wall (12) of the housing (1) and the implantation base (5);
a sensor assembly (20), fastened to the implantation base (5) and located on a side that is of the implantation base (5) and that faces away from the top wall (12) of the housing (1), and the sensor assembly (20) comprises a sensor (244) and a puncture needle (242); and
a housing cap (8), detachably fastened to an end that is of the side wall (11) of the housing (1) and that faces away from the top wall (12) of the housing (1), wherein the housing cap (8) wraps the abutting end (301) and closes the opening (13), wherein
when the housing cap (8) is detached from the housing (1), the abutting end (301) abuts against an organism (30), and the housing (1) moves in a direction close to the organism (30) relative to the base (3), the first elastic member (2) pushes the implantation base (5) toward the organism (30), the sensor assembly (20) abuts against the organism (30), and the sensor (244) is implanted into the organism (30) under guidance of the puncture needle (242).

2. The physiological sign detection apparatus (100) according to claim 1, wherein the base (3) further comprises a second elastic arm (36), the second elastic arm (36) is located on a side that is of the base (3) and that faces away from the abutting end (301), a tail end of the second elastic arm (36) is provided with a snap-fitting hook (361), the implantation base (5) is provided with snap-fitting space (58), and at least a part of the snap-fitting hook (361) of the second elastic arm (36) is located in the snap-fitting space (58), so that the second elastic arm (36) is snap-fitted into the implantation base (5);
the housing (1) further comprises a toggle (18), and the toggle (18) is fastened to a side that is of the top wall (12) of the housing (1) and that faces the opening (13); and
when the housing (1) moves in the direction close to the organism (30) relative to the base (3), the toggle (18) pushes the second elastic arm (36), and the snap-fitting hook (361) of the second elastic arm (36) is detached from the snap-fitting space (58).

3. The physiological sign detection apparatus (100) according to claim 2, wherein the second elastic arm (36) is provided with a guiding groove (362), at least a part of the toggle (18) is located in the guiding groove (362) of the second elastic arm (36), and the toggle (18) moves along the guiding groove (362) in a process in which the toggle (18) pushes the second elastic arm (36).

4. The physiological sign detection apparatus (100) according to claim 1, wherein the implantation base (5) has a snap-fitting hole (505) and a notch (506), and the notch (506) is located on a side that is of the snap-fitting hole (505) and that is close to the top wall (12) of the housing (1), and communicates with the snap-fitting hole (505);
the housing (1) further comprises a second cantilever (19), the second cantilever (19) is fastened to a side that is of the top wall (12) of the housing (1) and that faces the opening (13), a tail end of the second cantilever (19) is provided with a snap-fitting hook (191), and at least a part of the snap-fitting hook (191) of the second cantilever (19) is located in the snap-fitting hole (505), so that the second cantilever (19) is snap-fitted into the implantation base (5);
the base (3) further comprises a push block (302), the push block (302) is convexly disposed on an inner side surface of the base (3), and at least a part of the push block (302) is snap-fitted into the snap-fitting hole (505); and
when the housing (1) moves in the direction close to the organism (30) relative to the base (3), the second cantilever (19) is squeezed by the push block (302), the snap-fitting hook (191) of the second cantilever (19) is detached from the snap-fitting hole (505), the implantation base (5) moves in the direction close to the organism (30) relative to the base (3), and the push block (302) is detached from the snap-fitting hole (505) through the notch (506).

5. The physiological sign detection apparatus (100) according to any one of claims 1 to 4, wherein the base (3) further comprises a first elastic arm (34), an end that is of the first elastic arm (34) and that faces away from the abutting end (301) is provided with a hook (341), the hook (341) of the first elastic arm (34) protrudes from an outer side surface of the base (3), and the hook (341) of the first elastic arm (34) is snap-fitted into the side wall (11) of the housing (1), to limit movement of the housing (1) in a direction away from the abutting end (301) relative to the base (3).

6. The physiological sign detection apparatus (100) according to any one of claims 1 to 5, wherein the housing (1) further comprises a first guiding structure (15), the first guiding structure (15) is fastened to an inner side surface of the side wall (11) of the housing (1), the base (3) further comprises a second guiding structure (35), the second guiding structure (35) is fastened to the outer side surface of the base (3), and the first guiding structure (15) is slidably connected to the second guiding structure (35).

7. The physiological sign detection apparatus (100) according to any one of claims 1 to 6, wherein the base (3) further comprises a third guiding structure (37), the third guiding structure (37) is fastened to the inner side surface of the base (3), the implantation base (5) further comprises a fourth guiding structure (57), the fourth guiding structure (57) is fastened to an outer side surface of the implantation base (5), and the third guiding structure (37) is slidably connected to the fourth guiding structure (57).

8. The physiological sign detection apparatus (100) according to any one of claims 1 to 7, wherein the sensor assembly (20) further comprises a needle base (241), the puncture needle (242) is fastened to the needle base (241), a puncture end (2421) of the puncture needle (242) protrudes from the needle base (241), the sensor (244) comprises an implantation portion (2443), the puncture needle (242) wraps the implantation portion (2443) of the sensor (244), the physiological sign detection apparatus (100) further comprises a needle extraction base (241), the needle extraction base (241) is snap-fitted into the implantation base (5), and the needle extraction base (241) is snap-fitted into the needle base (241); and
when the implantation base (5) moves in the direction close to the organism (30), the implantation base (5) pushes the needle base (241) toward the organism (30) through the needle extraction base (241), and the puncture end (2421) of the puncture needle (242) and the implantation portion (2443) of the sensor (244) are punctured into the organism (30).

9. The physiological sign detection apparatus (100) according to claim 8, wherein the physiological sign detection apparatus (100) further comprises a second elastic member (7), and the second elastic member (7) is located between the implantation base (5) and the needle extraction base (241); and
when the sensor (244) is punctured into the organism (30), the second elastic member (7) pushes the needle extraction base (241) to a side away from the organism (30), the needle extraction base (241) drives the needle base (241) and the puncture needle (242) to move to the side away from the organism (30), and the puncture needle (242) is detached from the organism (30).

10. The physiological sign detection apparatus (100) according to claim 9, wherein the implantation base (5) comprises a side plate (51), a bottom plate (52), and a fourth elastic arm (501), a circumferential edge of the side plate (51) of the implantation base (5) is fastened to the bottom plate (52) of the implantation base (5), the fourth elastic arm (501) is spaced apart from an upper side plate of the implantation base (5) in a circumferential direction, an end of the fourth elastic arm (501) is fastened to the bottom plate (52) or the side plate (51) of the implantation base (5), the other end of the fourth elastic arm (501) is provided with a snap-fitting hook (5011), the needle extraction base (241) is mounted between the bottom plate (52) of the implantation base (5) and the snap-fitting hook (5011) of the fourth elastic arm (501), and the base (3) has avoidance space (39); and
when the sensor (244) is punctured into the organism (30), the needle extraction base (241) pushes away the fourth elastic arm (501), the snap-fitting hook (5011) of the fourth elastic arm (501) retreats to the avoidance space (39), and the needle extraction base (241) is detached from the snap-fitting hook (5011) of the fourth elastic arm (501).

11. The physiological sign detection apparatus (100) according to any one of claims 8 to 10, wherein the physiological sign detection apparatus (100) further comprises a stopper member (4), the stopper member (4) is fastened to an end that is of the implantation base (5) and that faces away from the needle extraction base (241), and the first elastic member (2) is mounted between the stopper member (4) and the top wall (12) of the housing (1).

12. The physiological sign detection apparatus (100) according to any one of claims 8 to 11, wherein the sensor assembly (20) further comprises a sensor base (245) and a sealing bottle (246), the sensor (244) is fastened to the sensor base (245), the sensor base (245) is located between the needle base (241) and the sealing bottle (246), an end of the needle base (241) passes through the sensor base (245), the sealing bottle (246) is detachably fastened to the needle base (241) and is hermetically connected to the sensor base (245), the needle base (241) is hermetically connected to the sensor base (245), the sealing bottle (246) has a hollow cavity, and the implantation portion (2443) of the sensor (244) and the puncture end (2421) of the puncture needle (242) are located in the hollow cavity of the sealing bottle (246).

13. The physiological sign detection apparatus (100) according to any one of claims 8 to 11, wherein the sensor assembly (20) further comprises an upper cover (21) and a sealing bottle (246), the sensor (244) is snap-fitted into the upper cover (21), the upper cover (21) is located between the needle base (241) and the sealing bottle (246), an end of the needle base (241) passes through the upper cover (21), the sealing bottle (246) is detachably fastened to the needle base (241) and is hermetically connected to the upper cover (21), the needle base (241) is hermetically connected to the upper cover (21), the sealing bottle (246) has a hollow cavity, and the implantation portion (2443) of the sensor (244) and the puncture end (2421) of the puncture needle (242) are located in the hollow cavity of the sealing bottle (246).

14. The physiological sign detection apparatus (100) according to claim 12 or 13, wherein the sealing bottle (246) is threadedly connected to the needle base (241), the sealing bottle (246) comprises a ratchet (2463), the ratchet (2463) is convexly disposed on an outer side surface of the sealing bottle (246), the housing cap (8) is threadedly connected to the housing (1), the housing cap (8) comprises a pawl (83), and the pawl (83) and the ratchet (2463) are disposed in cooperation; and
in a process in which the housing cap (8) is screwed on the housing (1), the pawl (83) is slidably connected to the ratchet (2463), and in a process in which the housing cap (8) is detached from the housing (1), the pawl (83) is snap-fitted into the ratchet (2463) and pushes the sealing bottle (246) to be detached from the needle base (241).

15. The physiological sign detection apparatus (100) according to claim 12 or 13, wherein the sealing bottle (246) is snap-fitted into or interference-connected to the needle base (241), the sealing bottle (246) comprises a boss (2465), the boss (2465) is located at an end that is of the sealing bottle (246) and that faces away from the needle base (241), the housing cap (8) is snap-fitted into or interference-connected to the housing (1), the housing cap (8) comprises a snap-fitting jaw, and the snap-fitting jaw is snap-fitted with the boss (2465); and
in a process in which the housing cap (8) is detached from the housing (1), the housing cap (8) drives the sealing bottle (246) to be detached from the needle base (241).

16. The physiological sign detection apparatus (100) according to any one of claims 12 to 15, wherein at least a partial region of the sealing bottle (246) allows ionizing radiation to pass through.

17. The physiological sign detection apparatus (100) according to claim 12, wherein the sensor (244) and the sensor base (245) are integrally formed by using an in-mold injection molding process, and a region that is of the sensor (244) and that is in contact with the sensor base (245) is covered with a heat insulation layer.

18. The physiological sign detection apparatus (100) according to any one of claims 12 to 17, wherein the sensor assembly (20) further comprises the upper cover (21), a battery (22), a connector (23), a circuit board (25), and a lower cover (26), the upper cover (21) is fastened to the lower cover (26), accommodation space (219) is formed between the upper cover (21) and the lower cover (26), the battery (22), the connector (23), the circuit board (25), and a connecting portion (2441) of the sensor (244) are located in the accommodation space (219), the circuit board (25) is fastened to the lower cover (26), the battery (22) and the connector (23) are fastened to the circuit board (25) and are electrically connected to the circuit board (25), and the sensor (244) is electrically connected to the connector (23).

19. The physiological sign detection apparatus (100) according to claim 18, wherein the connector (23) comprises a connecting groove (234), a plurality of first spring plates (232), and a plurality of second spring plates (233), the connecting groove (234) comprises a first groove wall (2341) and a second groove wall (2342) that are disposed opposite to each other, the plurality of first spring plates (232) are convexly disposed on the first groove wall (2341), the plurality of second spring plates (233) are convexly disposed on the second groove wall (2342), and the connecting portion (2441) of the sensor (244) is snap-fitted into the connecting groove (234) of the connector (23) and is electrically connected to the plurality of first spring plates (232) and/or the plurality of second spring plates (233).

20. The physiological sign detection apparatus (100) according to claim 19, wherein the first spring plate (232) and the second spring plate (233) each are formed by bending a metal plate, a plate surface of the first spring plate (232) is in contact with the sensor (244), and a plate surface of the second spring plate (233) is in contact with the sensor (244).

21. The physiological sign detection apparatus (100) according to any one of claims 18 to 20, wherein the lower cover (26) has a first through hole (266), the upper cover (21) has a second through hole (215), the second through hole (215) directly faces and communicates with the first through hole (266), and the sensor base (245) is snap-fitted into the lower cover (26) and/or the upper cover (21), the sensor base (245) is exposed from the first through hole (266) and is exposed from the second through hole (215).

22. The physiological sign detection apparatus (100) according to any one of claims 18 to 21, wherein the sensor assembly (20) further comprises a bonding member (27), the bonding member (27) has a first surface (271) and a second surface (272) that are disposed facing away from each other, and the first surface (271) of the bonding member (27) is stuck on a side that is of the lower cover (26) and that faces away from the upper cover (21); and
when the sensor assembly (20) abuts against the organism (30), the second surface (272) of the bonding member (27) is stuck on the organism (30).

23. The physiological sign detection apparatus (100) according to any one of claims 1 to 12, wherein the physiological sign detection apparatus (100) further comprises a thin film (206), the housing cap (8) is provided with a through hole (89), the thin film (206) covers the through hole (89), and the thin film (206) is made of a waterproof and breathable material.

24. The physiological sign detection apparatus (100) according to claim 23, wherein the physiological sign detection apparatus (100) further comprises a sealing member (205), and the housing cap (8) is hermetically connected to the housing (1) by using the sealing member (205).

25. The physiological sign detection apparatus (100) according to any one of claims 1 to 12, 23, and 24, wherein the physiological sign detection apparatus (100) further comprises a bonding member (27), the bonding member (27) has a first surface (271) and a second surface (272) that are disposed facing away from each other, and the first surface (271) of the bonding member (27) is stuck on the abutting end (301) and covers the sensor assembly (20); and
when the sensor assembly (20) abuts against the organism (30), the first surface (271) of the bonding member (27) is stuck on the sensor assembly (20), and the second surface (272) of the bonding member (27) is stuck on the organism (30).
